# EUROPÄISCHE PATENTSCHRIFT

(11) **EP 3 400 951 B1**
(45) Veröffentlichungstag und Bekanntmachung des Hinweises auf die Patenterteilung: **11.12.2019**
(21) Anmeldenummer: 17173823.0
(22) Anmeldetag: 31.05.2017
(51) Int. Cl.: A61K 31/737, A61K 31/728, A61K 9/08, A61P 13/10, A61P 29/00

(54) **BLASENINSTILLATIONSZUSAMMENSETZUNG ENTHALTEND CHONDOITINSULFAT (4,5 MG/ML), HYALURONSÄURE (16 MG/ML) UND PHOSPHATPUFFER (PH 6,1 BIS 7,9) MIT ERHÖHTER LAGERSTABILIÄT ZUR BEHANDLUNG VON CYSTITIS**
BLADDER INSTILLATION COMPOSITION CONTAINING CHONDOITIN SULFATE (4,5 MG/ML), HYALURONIC ACID (16 MG/ML) AND PHOSPHATE BUFFER (PH 6,1 TO 7,9) WITH AN IMPROVED STORAGE STABILITY FOR THE TREATMENT OF CYSTITIS
COMPOSITION D'INSTILLATION DE LA VESSIE CONTENANT SULFATE CHONDOITINIQUE (4,5 MG/ML), ACIDE HYALURONIQUE (16 MG/ML) ET TAMPON PHOSPHATE (PH 6,1 À 7.9) À STABILITÉ AU STOCKAGE AMÉLIORÉE POUR LE TRAITEMENT DE CYSTITIS

(30) Priorität: 12.05.2017 EP 17000821
(43) Veröffentlichungstag der Anmeldung: 14.11.2018
(73) Patentinhaber: Farco-Pharma GmbH, 50670 Köln (DE)
(72) Erfinder: MEIER, Andreas, D - 50670 Köln (DE)
(74) Vertreter: Von Rohr Patentanwälte Partnerschaft mbB

(56) Entgegenhaltungen:
- WO-A1-2013/144867
- JUNG-SOO PYO ET AL: "Systematic Review and Meta-Analysis of Intravesical Hyaluronic Acid and Hyaluronic Acid/Chondroitin Sulfate Instillation for Interstitial Cystitis/Painful Bladder Syndrome", CELLULAR PHYSIOLOGY AND BIOCHEMISTRY, Bd. 39, Nr. 4, 15. September 2016 (2016-09-15), Seiten 1618-1625, XP002775407,
- Anonymous: "iAluRil®: Product Information", Juno Pharmaceuticals , 10. Oktober 2015 (2015-10-10), XP002775632, Gefunden im Internet: URL:http://www.ialuril.com.au/downloads/iA luRil-Product-Information-JUNO-Aus-Oct-10- 2015.pdf [gefunden am 2017-11-16]

## Beschreibung

Die vorliegende Erfindung betrifft das medizinisch-therapeutische Gebiet der Behandlung von vorzugsweise entzündlichen Erkrankungen des Urogenitaltraktes, insbesondere Cystitis, wie interstitieller Cystitis.

Insbesondere betrifft die vorliegende Erfindung eine Zusammensetzung, insbesondere pharmazeutische Zusammensetzung, welche sich insbesondere zur prophylaktischen bzw. therapeutischen Behandlung von vorzugsweise entzündlichen Erkrankungen des Urogenitaltraktes, insbesondere von entzündlichen Erkrankungen der Harnblase, bevorzugt von Cystitis, eignet. Gleichermaßen betrifft die vorliegende Erfindung auch eine die erfindungsgemäße Zusammensetzung enthaltende Aufbewahrungs- bzw. Applikationsvorrichtung. Die vorliegende Erfindung betrifft auch eine die erfindungsgemäße Aufbewahrungs- bzw. Applikationsvorrichtung enthaltende Verpackungseinheit sowie ein Kit, welches die Aufbewahrungs- bzw. Applikationsvorrichtung nach der Erfindung, die Zusammensetzung nach der Erfindung sowie eine an die Aufbewahrungs- bzw. Applikationsvorrichtung anschließbare Instillationsvorrichtung enthält.

Das Krankheitsbild der Cystitis kann im Allgemeinen in zwei Gruppen eingeteilt werden. Neben einer solchen Cystitis, die durch insbesondere bakterielle Infektionen verursacht wird und die in der Regel mit Antibiotika oder aber durch chirurgische Intervention zur Beseitigung von Ursachen der Infektion, z. B. bei Obstruktionen oder Reflux, zu therapieren ist, gibt es eine Reihe von Entzündungen der Blase, die nicht durch Infektionen hervorgerufen werden. Dazu zählen die radiogene Cystitis (= Strahlencystitis) und die interstitielle Cystitis.

Die radiogene Cystitis tritt bei ca. 5 % der wegen Malignomen im kleinen Becken bestrahlten Patienten auf. Diese hämorrhagische Cystitis tritt in der Regel sechs Monate bis zehn Jahre nach der Bestrahlung auf und kann auf wahrscheinlich irreversible Gewebeveränderungen zurückgeführt werden.

Die Behandlungsmöglichkeiten bei der radiogenen Cystitis umfassen eine Therapie mit Antispasmodika, z. B. Trospiumchlorid, Darifenacin etc. oder eine sogenannte hyperbare Oxygenierung.

Ein weitaus größeres Feld ist die interstitielle Cystitis, insbesondere da hiervon ein großer Personenkreis betroffen ist. Die interstitielle Cystitis wird mitunter auch als "chronisch idiopathische Blasenentzündung unklarer Genese" definiert. Die interstitielle Cystitis ist schwer zu diagnostizieren, und ihre Behandlungsmöglichkeiten werden als schwierig angesehen. Die interstitielle Cystitis wird auch unter den Ausdruck *"painful bladder syndrome"* (schmerzhaftes Blasensyndrom) subsumiert.

Hinsichtlich der Ätiologie gibt es mitunter noch keine vollständig gesicherten Erkenntnisse. Diesbezüglich werden diverse Hypothesen diskutiert, wie eine Freisetzung von inflammatorischen Substanzen durch Mastzellaktivierung aufgrund von unterschiedlichen Stimuli; okkulten Infektionen; Steigerung der Durchlässigkeit der Blasenwand für toxische Substanzen; immunologischen Prozessen sowie einer Hypersensitivität von Nervenfasern mit Erhöhung der Nervenfaserdichte.

Das diagnostische Spektrum der interstitielle Cystitis umfasst neben dem Miktionsprotokoll und dem Schmerztagebuch mit VAS (Visuelle Analogskala) sowie der bakteriologischen Untersuchung zum Ausschluss eines Harnwegsinfektes und der Urinzystologie zum Ausschluss eines Karzinoms in situ und die unter Narkose durchzuführende Cystoskopie mit gegebenenfalls einhergehender Biopsie.

Die interstitielle Cystitis ist eine chronisch-entzündliche Erkrankung der Blase ohne nachweisbare Bakterien im Urin. Folglich handelt es sich hierbei um eine Cystitis nichtbakteriellen Ursprungs. Es handelt sich um eine bis heute nicht völlig geklärte Erkrankung, unter der die Patienten oft schlimmer leiden als unter einer Tumorerkrankung. Auch das US National Health Institute hat die interstitielle Cystitis als Erkrankung mit höherer Priorität eingestuft. Die Lebensqualität kann durch starken Harndrang, häufiges Wasserlassen tagsüber wie auch in der Nacht und zunehmenden Schmerzen extrem beeinträchtigt werden.

Nach jüngsten Erhebungen scheint die Häufigkeit der Erkrankung zuzunehmen. Patienten, bei denen die Erkrankung auftritt, sind meist im mittleren Lebensalter. Die Erkrankung kommt im Verhältnis bei Frauen häufiger vor als bei Männern. Ein Grund dafür kann in der Anatomie der Frau gesehen werden, deren Harnröhre kürzer ist als die des Mannes. Dies führt zu einer höheren Anfälligkeit gegenüber aufsteigenden Harnwegsinfektionen. Bei Frauen mit wiederholten (rezidivierenden) Harnwegsinfektionen ist die Schleimhaut der Blase in stärkerem Maße geschädigt. Diese ständige Reizung kann zu der nichtbakteriellen, insbesondere chronischen interstitiellen Cystitis führen. Die interstitielle Cystitis wird somit überwiegend bei Frauen diagnostiziert, wobei jedoch auch Männer von der Erkrankung betroffen sein können.

Die häufigsten Symptome der interstitiellen Cystitis sind (Reihenfolge in absteigender Häufigkeit):
- erhöhter Harndrang;
- häufiges Wasserlassen;
- Schmerzen des Beckens, des unteren Bauchraumes und des Darmes;
- Beckendruck;
- Schmerzen beim Wasserlassen, verbunden mit Abgabe geringster Mengen von Urin;
- starker Schmerz während und nach dem Geschlechtsverkehr;
- brennendes Schmerzgefühl;
- massive Durchschlafprobleme durch Schmerzen;
- Blut im Urin.

Die Hauptsymptome der interstitiellen Cystitis sind somit ein Kapazitätsverlust der Blase mit füllungsabhängigen Schmerzen und häufigem und massivem Harndrang. Man spricht auch von der Trias: *"frequency, urgency, pain"* (Häufigkeit, Dringlichkeit, Schmerzen).

Die vorgenannten Symptome der interstitiellen Cystitis können z. B. durch eine verstärkte Schmerzempfindlichkeit oder durch psychische Faktoren noch zunehmen. Gezeigt wurde eine Störung der Zusammensetzung der Glykosaminoglykanschicht, und immunhistochemische Untersuchungen zeigen u. a. eine verminderte Chondroitisulfatfärbung.

Ohne sich auf eine Theorie beschränken oder festlegen zu wollen, kann die Entstehung und Entwicklung einer interstitiellen Cystitis wie folgt erklärt werden: Durch Lücken der Schutzschicht der Blasenschleimhaut, der sogenannten Glykosaminoglykanschicht, gelangen Bakterien, Mikrokristalle, Proteine und/oder schädliche, gelöste Bestandteile des Harns, wie Harnstoff, direkt in tiefere Schichten der Blasenschleimhaut und bewirken dort eine weitere Schädigung.

Durch die Schädigung der Blasenschleimhaut und durch die resultierende chronische Entzündung kommt es zu Reparaturvorgängen, die oftmals mit einer Vernarbung einhergehen. Dies kann zu einer eingeschränkten Elastizität der Harnblasenwand und zu einem zunehmenden Verlust des Fassungsvermögens der Harnblase führen. Im Spätstadium der interstitiellen Cystitis kann eine Schrumpfblase entstehen, und eine operative Entfernung der Harnblase kann unter bestimmten Umständen notwendig werden. Daher ist eine frühzeitige Erkennung und Therapie zur Vermeidung des Spätstadiums der interstitiellen Cystitis von großer Bedeutung.

Die Hauptursache der interstitiellen Cystitis ist die Schädigung der Blasenschleimhaut. Die Blasenschleimhaut ist durch eine Schutzschicht, die unter anderem Hyaluronsäure enthält, gegen Mikroorganismen, krebsverursachende Substanzen, und andere schädliche Stoffe, die im Urin vorkommen, abgeschirmt. Diese Schutzschicht, welche auch als Glykosaminoglykanschicht (GAG-Schicht) bezeichnet wird und welche neben Hyaluronsäure auch Chondroitinsulfat, Heparin und Pentosanpolyphosphat als wichtige Bestandteile enthält, ist extrem wasserbindend und bildet sozusagen einen "Wasserfilm" und somit eine weitere physikalische Barriere auch gegen schädigende Substanzen im Urin.

Bei Patienten mit Cystitis und insbesondere mit interstitieller Cystitis bestehen Defekte in dieser Schutzschicht der Blasenschleimhaut. Insbesondere hat man einen Verlust von Hyaluronsäure festgestellt.

Weitere Ursachen der interstitiellen Cystitis können z. B. Autoimmunreaktionen, die sich gegen körpereigene Zellen in der Blase richten, oder frühere chronische bakterielle Infektionen sein.

Die typische Symptomatik der interstitiellen Cystitis ist häufiges Wasserlassen, verstärkter Harndrang sowie in manchen Fällen auch ein unkontrolliertes Wasserlassen (Harninkontinenz) und Blut im Urin. Starker Schmerz entwickelt sich vor allem bei voller Blase, typisch ist ein Nachlassen des Schmerzgefühls nach dem Wasserlassen. Weitere Kennzeichen sind Schmerzen des Beckens, des unteren Bauchraumes und des Dammes, Beckendruck sowie Schmerzen beim Wasserlassen, verbunden damit, dass Urin nur tröpfchenweise abgegeben werden kann. Auch während und nach dem Geschlechtsverkehr treten häufig starke Schmerzen auf. Die Beschwerden der Patienten bedingen in nicht wenigen Fällen einen so enormen Leidensdruck, dass auch operative Verfahren bis hin zur Cystektomie erforderlich werden.

Zur Diagnose der interstitiellen Cystitis ist es wichtig, dass andere Blasenerkrankungen mit ähnlichen Symptomen auszuschließen sind. In einem ersten Schritt ist zu klären, ob der Patient durch eine frühere Operation (beispielsweise im Unterbauch) schmerzbelastet ist, ob die Blasenentzündung durch eine Strahlen- oder Chemotherapie hervorgerufen wurde oder ob wiederholte, rezidivierende Infekte vorlagen oder vorliegen. In der Folge ist zu untersuchen, ob gynäkologische, neurologische, psychiatrische und/oder rheumatische Erkrankungen auszuschließen sind. Des Weiteren sollten Beschwerden der Wirbelsäure und Allergien ausgeschlossen werden.

Im Rahmen der Untersuchung bzw. Diagnose der interstitiellen Cystitis kann beispielsweise in einem Labor eine Harnkultur und eine Untersuchung der zellulären Bestandteile im Urin (Harnzytologie) durchgeführt werden. Bei weiblichen Patienten sollte zum Ausschluss von sexuell übertragbaren Erkrankungen ein Vaginalabstrich erstellt werden.

Die Schmerzempfindlichkeit wird über eine Tastuntersuchung der Scheide erhoben. Bei männlichen Patienten wird zum Ausschluss einer bakteriell verursachten Entzündung der Prostata eine Bakterienkultur aus dem Ejakulat angelegt. Um ein Prostatakarzinom auszuschließen, wird der Wert des prostataspezifischen Tumormarkers (PSA = prostataspezifisches Antigen) bestimmt. Über eine Ultraschalluntersuchung wird der Restharn bestimmt und ein Einwachsen der Prostata in die Blase ausgeschlossen.

Eine weitergehende Untersuchung kann auch über eine Blasenspiegelung (Cystoskopie) durchgeführt werden. Die Blasenspiegelung kann unter Narkose durchgeführt werden. Typische Zeichen für eine interstitielle Cystitis, welche mittels der Cytoskopie manifestiert werden können, sind vermehrtes Einwachsen von Blutgefäßen in die Blasenschleimhaut, Flüssigkeitsansammlungen in der Schleimhaut, Aufbersten der Schleimhaut (Glomeruli), punktförmige Blutungen nach Aufdehnung der Blase unter Druck durch Wassereinspülung (Hydrodistension) sowie bei etwa 10 bis 20 % der Patienten Zeichen von Blasengeschwüren (Hunner's Ulcera).

Bei der interstitiellen Cystitis verspüren die Patienten schon bei geringen Urinmengen einen starken Harndrang; ihre Blasenkapazität ist vermindert. Zur Diagnose der interstitiellen Cystitis kann deshalb die Bestimmung des maximalen Füllvolumens und anschließend eine vergleichende Blasenkapazitätsmessung (Cystometrie) durchgeführt werden.

Untersuchungen zur interstitiellen Cystitis zeigen, dass das Blasenepithel bzw. das Urothel der Harnblase bei Vorliegen einer Cystitis defizient ist. Diese Schwächung trägt zu den klinischen Symptomen der interstitielle Cystitis wesentlich bei.

Was die Therapie der interstitiellen Cystitis anbelangt, so gibt es bis zum gegenwärtigen Zeitpunkt weder ein Heilmittel, noch eine Behandlungsmethode, die für alle Patienten wirksam ist.

So ist im Stand der Technik eine Zusammensetzung auf Basis von Pentosanpolysulfat-Natrium bekannt. Man geht davon aus, dass die Wirkungsweise in der Reparatur einer dünnen oder beschädigten Blasenwand besteht. Die Erfolge sind aber nicht immer befriedigend.

Auch Antidepressiva, wie trizyklische Antidepressiva, haben sich in gewisser Weise als wirksam zur Linderung der Schmerzen und Miktionshäufigkeit bei interstitieller Cystitis erwiesen. Bei der interstitiellen Cystitis werden diese Medikamente jedoch nur wegen ihrer schmerzlindernden Eigenschaften verwendet.

Weitere orale Arzneimittel umfassen entzündungshemmende Mittel, Antispasmodika, Antihistaminika und muskelentspannende Mittel. Derartige Medikamente können die Erkrankung jedoch nur im bestimmten Maße lindern. Ein durchgreifender Therapieerfolg ist mit diesen Medikamenten in der Regel nicht möglich.

Weiterhin können Blaseninstillationen mit bestimmten Substanzen durchgeführt werden. So kann eine Blasendehnung realisiert werden, wobei die Blase unter Vollnarkose zur Dehnung mit Wasser gefüllt wird. Dies gehört zwar vorrangig zum Diagnoseverfahren für die interstitiellen Cystitis, es kann aber auch therapeutisch eingesetzt werden.

Darüber hinaus kann DMSO (Dimethylsulfoxid) als Arzneimittel direkt in die Blase gefüllt werden. Es soll entzündungshemmend wirken und somit die Schmerzen verringern. DMSO kann mit Steroiden, Heparin und anderen Inhaltsstoffen zu einem "Blasencocktail" gemischt werden. Die Nebenwirkungen sind jedoch oftmals hoch.

Andere Blaseninstillationen, beispielsweise unter Verwendung von Oxychlorosen-Natrium sind größtenteils sehr schmerzhaft und erfordern eine Vollnarkose. Silbernitrat wird selten verwendet und gilt als veraltete Therapie.

Andere Behandlungsmethoden, wie eine gezielte Ernährung, wobei bestimmte Nahrungsmittel, insbesondere säurehaltige, scharfe Nahrungsmittel, vermieden werden, können die Schwere der Symptome nur geringfügig lindern. Die interstitielle Cystitis kann auch durch Rauchen, Kaffee oder Tee und alkoholische Getränke verschlimmert werden.

Selbsthilfetechniken können die Lebensqualität im geringen Umfang verbessern und die Inzidenz und Schwere von Anfällen verringern. Dazu gehören beispielsweise eine Änderung im Lebensstil, Stressreduzierung, Visualisierung, Biofeedback, Blasentraining und sportliche Betätigung. Ein dauerhafter Therapieerfolg ist mit diesen Methoden jedoch oftmals nicht möglich.

Für eine geringe Anzahl an Patienten mit schweren Symptomen, die nicht auf andere Behandlungsmethoden ansprechen, kann eine Blasenoperation in Erwägung gezogen werden. In manchen Fällen werden die Symptome jedoch auch dadurch nicht besser. Zur Behandlung der interstitiellen Cystitis wurden mehrere Arten von Operationen eingesetzt, einschließlich Cystektomie und Harnumleitung. Aufgrund der schwere des operativen Eingriffs sollten Operationen jedoch stets das letzte Mittel darstellen.

Die Therapiemöglichkeiten sind somit ebenso vielfältig wie insgesamt unbefriedigend. Zusammenfassend und in Ergänzung zu den obigen Ausführungen kommen bislang die folgenden Behandlungsmethoden für die interstitielle Cystitis in Betracht. Die Innervation beeinflussende Medikamente (Antispasmodika, Antihistaminika); eine cytodestruktive Therapie mit anschließender Regeneration (z. B. DMSO, Hydrodistension); eine cytoprotektive Therapie zur Wiederherstellung der Glykosaminoglykanschicht (Heparin, Pentosulfanpolysulfat). Im Rahmen der konservativen Therapie geht es um die Reduktion der Symptome mittels oral verabreichter Substanzen, wie Antispasmodika (Erfolg gering); Antihistaminika; Antidepressiva, vor allem Trizyklika (Amitriptylin); Zytoprotektiva, wie Pentosulfanpolysulfat (sehr lange Latenzzeit bis zwei Jahre, bis ein Erfolg messbar wird); Immunsuppresiva, wie Azathioprin, Cyclosporin, Chloroquin; Calciumantagonisten, z. B. Nifedipin. Die Hydrodistension ist die Überdehnung der Blase mittels eines intravesikal eingeführten Ballons. In der Regel wird über einen Zeitraum von drei Stunden gedehnt, die therapeutische Wirkung ist jedoch gering und nur kurz anhaltend. Weiter gibt es verschiedene intravesikale pharmakotherapeutische Maßnahmen, insbesondere zur Wiederherstellung der Glykosaminoglykanschicht. Hierzu werden, wie zuvor erörtert, Pentosanpolysulfat, Heparin oder DMSO eingesetzt. Auch alternative Behandlungsverfahren werden durchgeführt. Dazu zählen Entspannungsübungen, Verhaltenstraining, Akupunktur, Neuromodulation und diätetische Maßnahmen.

Im Stand der Technik werden zur Behandlung von Cystitis, wie der interstitiellen Cystitis, weitere Zusammensetzungen bzw. Verfahren vorgeschlagen; diese weisen jedoch nicht immer die gewünschte bzw. erforderliche Wirkeffizienz auf. Zudem sind derartige Zusammensetzungen des Standes der Technik mitunter nicht ausreichend lagerungsstabil.

Die WO 2004/073584 A2 betrifft eine pharmazeutische Zusammensetzung für den Einsatz in die Blase eines Patienten, wobei die Zusammensetzung Chondroitinsulfat umfasst. Dieses Dokument fokussiert auf die Verwendung eines einzelnen Wirkstoffs.

Weiterhin betrifft die US 6 083 933 A Chondroitinsulfat enthaltende Zusammensetzungen, welche im Rahmen der Behandlung der interstitiellen Cystitis eingesetzt werden können. Durch die Verwendung einer Monozusammensetzung, welche außer Chondroitinsulfat keine weiteren Wirkstoffe enthält, ist die Wirkung hinsichtlich der zu behandelnden Cystitiden jedoch nicht immer ausreichend.

Die US 5 880 108 A1 betrifft ein Verfahren zur Behandlung von Cystitis, wobei die Harnblase und die damit in Verbindung stehenden Strukturen mit einer Lösung in Kontakt gebracht werden sollen, wobei die Lösung Hyaluronsäure aufweist. Die Zusammensetzung kann weiterhin bestimmte Substanzen enthalten, welche sich für die Behandlung von mit Cystitis im Zusammenhang stehenden Grunderkrankungen eignen sollen.

Die CN 105982912 A betrifft eine pharmazeutische Zusammensetzung, welche Natriumhyaluronat mit einem mittleren Molekulargewicht und Chondroitinsulfat mit einem relativ geringen Molekulargewicht enthält. Das Molekulargewicht des Natriumhyaluronats liegt im Bereich von 200 bis 1.000 kDa und die Konzentration im Bereich von 1 bis 2 %. Das Molekulargewicht des Chondroitinsulfats liegt im Bereich von 5 bis 10 kDa und die Konzentration im Bereich von 1,5 bis 3 %. Die Zusammensetzung kann zur Behandlung einer nicht-bakteriellen Cystitis, chronischer, rekurrenter bakterieller Cystitis, Cystitis in Folge von Strahlung bzw. Bestrahlung, interstitieller Cystitis und neurogenen Infektionen des Harntrakts eingesetzt werden.

Weiterhin beschreibt die wissenschaftliche Publikation gemäß Pyo und Cho: "Systematic Review and Meta-Analysis of Intravesical Hyaluronic Acid and Hyaluronic Acid/Chondroitin Sulfate Instillation for Interstitial Cystitis/Painful Bladder Syndrome", erschienen in "Cellular Physiology and Biochemistry", September 2016, Seiten 1618-1625, Zusammensetzungen für die Behandlung von interstitieller Cystitis, welche Hyaluronsäure und Chondroitinsulfat enthalten. Die Behandlung erfolgt dabei mittels Blaseninstillation.

Weiterhin wird auf Basis des Marktprodukts "iAluRil®" eine Injektionsspritze für die Blaseninstillation vertrieben, welche eine Zusammensetzung auf Basis von Natriumhyaluronat mit einer Konzentration von 800 mg/50 ml und Natriumchondroitinsulfat mit einer Konzentration von 1 g/50 ml enthält. Die Zusammensetzung wird zur Behandlung von interstitieller Cystitis eingesetzt.

Die WO 2007/138014 A1 beschreibt im Übrigen ebenfalls Zusammensetzungen auf Basis von Glycosaminoglycanen, insbesondere Hyaluronsäure, in Kombination mit Chondroitinsulfat zur Behandlung von interstitieller Cystitis.

Schließlich beschreibt die WO 2013/144867 A1 pharmazeutische Zusammensetzungen mit Chondroitinsulfat und Hyaluronsäure-Derivaten, welche zur Behandlung von interstitieller Cystitis eingesetzt werden und zur Stabilisierung einen Phosphatpuffer enthalten, so dass die Zusammensetzung einen stabilen pH-Wert von ca. 7 im physiologischen Bereich aufweisen.

Im Hinblick auf die medizinisch-therapeutischen Anforderungen an entsprechende Instillationszusammensetzungen sowie deren Handhabbarkeit besteht im Stand der Technik neben der Bereitstellung einer hohen Wirksamkeit in Bezug auf die zugrundeliegende Erkrankung zudem ein großer Bedarf an stabilen Zusammensetzungen bzw. Zusammensetzungen mit hoher Haltbarkeit, d. h. an solchen Zusammensetzungen zur Instillation, welche über einen entsprechend großen Zeitraum über definierte bzw. konstante (Produkt-)Eigenschaften verfügen, beispielsweise im Hinblick auf die Stabilität der Wirksubstanzen mit diesbezüglich einhergehenden geringen Mengen an mitunter toxischen Abbauprodukten, pH-Wert-Stabilität, konstante Viskosität oder dergleichen. Insbesondere besteht ein entsprechender Bedarf an Zusammensetzungen mit hoher Lagerungsstabilität, d. h. solchen Zusammensetzungen zur Instillation, welche auch über einen langen Zeitraum hinsichtlich ihrer Wirk- bzw. Inhaltsstoffe bzw. ihrer physikochemischen Eigenschaften stabil bzw. konstant sind.

Denn aus dem Stand der Technik bekannte Zusammensetzungen, welche im Rahmen der Behandlung von insbesondere entzündlichen Erkrankungen des Urogenitaltraktes, wie Cystitis, verabreicht werden, weisen mitunter nicht optimale Stabilitätsverhalten auf, wobei zudem eine nicht immer optimale Wirksamkeit vorliegt. In diesem Zusammenhang besteht insbesondere auch eine Gefahr, dass im Rahmen der zugrundeliegenden Behandlung mitunter vorzeitig degradierte Zusammensetzungen mit gegebenenfalls verringerter Wirkstoffmenge bzw. entsprechend erhöhtem Anteil an gegebenenfalls schädlichen Abbauprodukten zur Anwendung kommen, was aber einem durchgreifenden Behandlungserfolg abträglich sein kann.

Vor diesem Hintergrund besteht daher eine Aufgabe der vorliegenden Erfindung darin, eine entsprechende Zusammensetzung, insbesondere pharmazeutische Zusammensetzung, vorzugsweise zur prophylaktischen bzw. therapeutischen Behandlung von vorzugsweise entzündlichen Behandlungen des Urogenitaltraktes, beispielsweise Cystitits, insbesondere interstitieller Cystitis, bereitzustellen, welche die zuvor geschilderten Nachteile des Standes der Technik zumindest weitgehend vermeidet oder aber wenigstens abschwächt.

Insbesondere soll eine solche Zusammensetzung eine gegenüber herkömmlichen, für die Behandlung von insbesondere entzündlichen Erkrankungen des Urogenitaltraktes, insbesondere Cystitis, wie interstitieller Cystitis, bestimmten pharmazeutischen Zusammensetzungen bzw. Präparten eine verbesserte Stabilität, insbesondere Lagerungsstabilität, aufweisen, insbesondere im Hinblick auf auch über längere Zeiträume konstante Wirkstoffmengen und physikochemischen Eigenschaften der zugrundeliegenden Zusammensetzungen.

Darüber hinaus besteht eine wiederum weitere Aufgabe der vorliegenden Erfindung auch darin, eine entsprechende Zusammensetzung bereitzustellen, welche zudem eine hohe Wirkeffizienz bei der Behandlung von vorzugsweise entzündlichen Erkrankungen des Urogenitaltraktes, insbesondere Cystitis, wie interstitieller Cystitis, aufweist, wobei gleichermaßen deren Handhabung und Verträglichkeit weiterführend verbessert sein soll.

Zur Lösung der zuvor geschilderten Aufgabe schlägt die vorliegende Erfindung - gemäß einem **ersten** Aspekt der vorliegenden Erfindung - eine Zusammensetzung, insbesondere pharmazeutische Zusammensetzung, nach Anspruch 1 vor; weitere, insbesondere vorteilhafte Ausgestaltungen der erfindungsgemäßen Zusammensetzung sind Gegenstand der diesbezüglichen Unteransprüche.

Zudem betrifft die vorliegende Erfindung - gemäß einem **zweiten** Aspekt der vorliegenden Erfindung - eine die erfindungsgemäße Zusammensetzung aufweisende bzw. enthaltende Aufbewahrungs- bzw. Applikationsvorrichtung, wie sie in dem diesbezüglichen, die Aufbewahrungs- bzw. Applikationsvorrichtung betreffenden unabhängigen Anspruch definiert ist; weitere, insbesondere vorteilhafte Ausgestaltungen der erfindungsgemäßen Aufbewahrungs- bzw. Applikationsvorrichtung sind Gegenstand des diesbezüglichen Unteranspruchs.

Darüber hinaus betrifft die vorliegende Erfindung - gemäß einem **dritten** Aspekte der vorliegenden Erfindung - auch eine Verpackungseinheit, welche die Aufbewahrungs- bzw. Applikationsvorrichtung nach der Erfindung enthält, gemäß dem diesbezüglichen, die Verpackungseinheit betreffenden unabhängigen Anspruch.

Schließlich betrifft die vorliegende Erfindung - gemäß einem **vierten** Aspekt der vorliegenden Erfindung - auch ein Kit, insbesondere Installationssystem, wie es in dem das Kit betreffenden unabhängigen Anspruch definiert ist.

Es versteht sich im Zusammenhang mit den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbezug sämtlicher Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Weiterhin gilt, dass der Fachmann - anwendungsbezogen oder einzelfallbedingt - von den nachfolgend angeführten Konzentrations-, Gewicht-, Mengen- und Bereichsangaben abweichen kann, ohne dass er den Rahmen der vorliegenden Erfindung verlässt.

Zudem gilt, dass sämtliche im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Der Begriff des Arzneimittels bzw. Medikaments (synonym auch "Pharmazeutikum"), wie er im Rahmen der vorliegenden Erfindung verwendet wird, ist sehr umfänglich zu verstehen und umfasst nicht nur Arzneimittel bzw. Pharmazeutika als solche (d. h. in arzneimittelrechtlicher Hinsicht), sondern vor allem auch sogenannte Medizinprodukte und darüber hinaus aber auch Homöopathika und Nahrungsergänzungsmittel sowie Kosmetika und Gebrauchsgegenstände. Mit anderen Worten kann also die erfindungsgemäße Zusammensetzung in Form eines Arzneimittels (Pharmazeutikums), Medizinprodukts, Homöopathikums, Nahrungsergänzungsmittels, Kosmetikums oder in Form eines Gebrauchsgegenstands vorliegen.

Dies vorausgeschickt, wird die vorliegende Erfindung nunmehr nachfolgend im Detail erläutert.

Gegenstand der vorliegenden Erfindung - gemäßen einem **ersten** Aspekt der vorliegenden Erfindung - ist somit eine Zusammensetzung, wobei die Zusammensetzung in Kombination und jeweils in wirksamen Mengen
(a) Chondroitinsulfat und/oder ein physiologisch verträgliches Chondroitinsulfat-Salz in einer Konzentration von (4,5 ± 0,5) mg/ml (Komponente (a)); und
(b) Hyaluronsäure und/oder ein physiologisch verträgliches Hyaluronsäure-Salz (Hyaluronat) in einer Konzentration von (16 ± 1,6) mg/ml (Komponente (b));
enthält,
wobei die Zusammensetzung einen pH-Wert im Bereich von 6,1 bis 7,9 aufweist und/oder wobei die Zusammensetzung auf einen pH-Wert im Bereich von 6,1 bis 7,9 eingestellt ist,
dadurch gekennzeichnet,
dass die Zusammensetzung (c) ein Dihydrogenphosphat/Monohydrogenphosphat-Puffersystem (Komponente (c)) enthält.

Überraschend wurde im Rahmen der vorliegenden Erfindung gefunden, dass auf Basis der erfindungsgemäßen Konzeption eine spezielle Zusammensetzung - welche nämlich als Komponente (a) Chondroitinsulfat bzw. ein physiologisch verträgliches Chondroitinsulfat-Salz mit definierter Konzentration, als Komponente (b) Hyaluronsäure bzw. ein physiologisch verträgliches Hyaluronsäure-Salz (synonym auch als "Hyaluronat" bezeichnet) mit definierter Konzentration, als Komponente (c) ein Dihydrogenphosphat/Monohydrogenphosphat-Puffersystem (synonym auch als "H₂PO₄⁻/HPO₄²⁻-Puffer(system)" bzw. "Phosphatpuffer(system)" bzw. chemisches Puffersystem bezeichnet) sowie gegebenenfalls als Komponente (d) mindestens einen physiologisch verträglichen Elektrolyten enthält, wobei die Zusammensetzung zudem einen definierten und mittels des chemischen Puffersystems eingestellten bzw. konstantgehaltenen pH-Wert aufweist - zur zweckgerichteten Applikation, insbesondere Instillation, im Rahmen der Behandlung von vorzugsweise entzündlichen Erkrankungen des Urogenitaltraktes, wie Cystitis, insbesondere interstitieller Cystitis, bereitgestellt werden kann, welche gegenüber dem Stand der Technik bei gleichzeitig hoher Wirksamkeit bzw. Wirkeffizienz und sehr guter Verträglichkeit darüber hinaus eine verbesserte Stabilität, insbesondere Lagerungsstabilität, aufweist.

Auf Basis der erfindungsgemäßen Zusammensetzung wird folglich ein hochwirksames Arzneimittel bzw. Medikament bzw. (Medizin-)Produkt bereitgestellt, welches aufgrund der gezielten Kombination und Abstimmung der zugrundeliegenden Komponenten und der Einstellung eines definierten pH-Wertes eine hervorragende Stabilität, insbesondere Lagerungsstabilität, aufweist, wobei auch nach entsprechend langen Lagerungszeiträumen kein wesentlicher und sowohl die Wirksamkeit als auch die Verträglichkeit negativ beeinflussender Abbau der Wirk- bzw. Inhaltsstoffe vorliegt, wobei darüber hinaus auch die physikochemischen Eigenschaften und die Wirksamkeit der Zusammensetzung zumindest im Wesentlichen unverändert bleiben.

Was die im Rahmen der vorliegenden Erfindung in völlig überraschender Weise gefundene Verbesserung der Stabilität, insbesondere Lagerungsstabilität, bei gleichzeitig hoher Wirksamkeit der erfindungsgemäßen Zusammensetzung anbelangt, so kommt diesbezüglich auch den in der erfindungsgemäßen Zusammensetzung vorliegenden Konzentrationen der Komponenten (a) und (b) eine hohe Bedeutung zu. Denn die Anmelderin hat in diesem Zusammenhang gefunden, dass im Hinblick auf die erfindungsgemäß vorgesehenen Konzentrationen und den damit einhergehenden Stoffmengen insbesondere auf Basis der Komponenten (a) und (b) ein diesbezügliches Stabilitäts- und Wirkoptimum vorliegt.

Dabei greifen die erfindungsgemäßen Maßnahmen auch hinsichtlich der Verwendung eines speziellen Puffersystems gemäß Komponente (c) und der Einstellung eines speziellen pH-Wertes sozusagen ineinander und verstärken sich gegenseitig und über die Wirkung der Einzelmaßnahmen im Hinblick auf die Gewährleistung einer hohen Stabilität, insbesondere Lagerungsstabilität, und Wirksamkeit hinaus, so dass diesbezüglich ein synergistischer Effekt vorliegt, wie auch anhand der nachfolgend angeführten Ausführungsbeispiele aufgezeigt. Ohne sich auf die nachfolgende Theorie beschränken oder festlegen zu wollen, liegt im Hinblick auf die erfindungsgemäße Zusammensetzung infolge der diesbezüglichen Maßnahmen, wie zuvor angeführt, sozusagen eine optimale Ausbildung einer Matrix bzw. eines Hydrogels auf Basis der zugrundeliegenden Komponenten, insbesondere auf Basis der Komponenten (a) und (b), vor, was den Abbau der zugrundeliegenden Wirk- bzw. Inhaltsstoffe bzw. eine lagerungs- bzw. zeitbedingte Veränderung der physikochemischen Eigenschaften, wie Viskosität oder dergleichen, entgegenwirkt. Folglich liegt eine entsprechende Vergrößerung der Stabilität, einhergehend mit größeren Lagerungszeiträumen, vor.

Das chemische Puffersystem gemäß Komponente (c) dient im Rahmen der vorliegenden Erfindung dabei insbesondere zur Stabilisierung der erfindungsgemäßen Zusammensetzung. Insbesondere führt die Einstellung eines konstanten pH-Wertes mittels des Puffersystems überraschenderweise dazu, dass einem unerwünschten Abbau der Wirkstoffe, insbesondere der Komponenten (a) und (b), bei Lagerung auch über einen längeren Zeitraum effizient entgegengewirkt wird. Auf diese Weise wird die Stabilität bzw. Lagerfähigkeit der erfindungsgemäßen Zusammensetzung weiterführend verbessert.

Hinsichtlich des Stabilitätsverhaltens hat es sich im Rahmen der vorliegenden Erfindung zudem in völlig überraschender Weise gezeigt, dass die Verwendung eines speziellen Phosphatpuffer(systems) gemäß Komponente (c) gegenüber anderen Puffern bzw. Puffersystemen überlegen ist, so dass infolge der Verwendung des speziellen Phosphatpuffer(systems) eine weiterführende Verbesserung der zugrundeliegenden Stabilitätseigenschaften vorliegt. Gleichermaßen ohne sich auf diese Theorie beschränken bzw. festlegen zu wollen, führt der Einsatz eines speziellen Puffers auch zu einer weiterführenden Stabilisierung der aus den Komponenten (a) und (b) resultierenden Matrix bzw. des diesbezüglichen Hydrogels, und zwar auch dadurch bedingt, dass auf Basis des speziellen Puffersystems eine diesbezüglich optimale Stabilisierung des pH-Wertes vorliegt, was der Stabilität der Zusammensetzung insgesamt zuträglich ist.

Darüber hinaus zeichnet sich die erfindungsgemäße Zusammensetzung auch über eine hervorragende Wirksamkeit bzw. Wirkeffizienz bei gleichzeitig guter Verträglichkeit im Hinblick auf die Behandlung von vorzugsweise entzündlichen Erkrankungen des Urogenitaltraktes, insbesondere Cystitis, wie interstitieller Cystitis, aus. Aufgrund der definierten und auch nach großen Lagerungszeiträumen vorliegenden konstanten physikochemischen Eigenschaften der erfindungsgemäßen Zusammensetzung ist zudem der Handhabung bzw. Applikation insbesondere im Hinblick auf die Instillation in die Harnblase, verbessert, was auch zu einer erhöhten Akzeptanz seitens des Patienten und zu einer Verringerung von Fehlanwendungen führt. Auf Basis der erfindungsgemäßen Konzeption wird dabei insgesamt einer zeitlichen Degradation bzw. einem zeitlich bedingten Abbau der entsprechenden Wirk- bzw. Inhaltsstoffe, insbesondere auch im Hinblick auf die Komponenten (a) und (b), entgegengewirkt, so dass auch nach entsprechend langen Zeiträumen hohe bzw. konstante Wirkstoffkonzentrationen bei zeitlich konstantem pH-Wert vorliegen. Hierdurch ist zudem gewährleistet, dass mitunter toxische bzw. schädliche Abbauprodukte nicht bzw. nur in geringen Mengen vorliegen, was gleichermaßen positiv für die Wirkeffizienz und Verträglichkeit der erfindungsgemäßen Zusammensetzung zu werten ist.

Insgesamt wird somit auf Basis der erfindungsgemäßen Konzeption eine Zusammensetzung bereitgestellt, welche sich in hervorragender Weise zur Behandlung von vorzugsweise entzündlichen Erkrankungen des Urogenitaltraktes, wie Cystitis, eignet und gegenüber dem Stand der Technik über signifikant verbesserte Eigenschaften verfügt.

Was die erfindungsgemäß eingesetzte Komponente (a) in Form von Chondroitinsulfat bzw. des physiologisch verträglichen Chondroitinsulfat-Salzes anbelangt (synonym auch als Chondroitinpolysulfat bzw. Chondroitinpolysulfat-Salz bezeichnet), so hat es sich erfindungsgemäß als vorteilhaft erwiesen, wenn die Zusammensetzung das Chondroitinsulfat bzw. das physiologisch verträgliche Chondroitinsulfat-Salz (Komponente (a)) in einer Konzentration von (4,5 ± 0,4) mg/ml, insbesondere in einer Konzentration von (4,5 ± 0,3) mg/ml, vorzugsweise in einer Konzentration von (4,5 ± 0,15) mg/ml, besonders bevorzugt in einer Konzentration von etwa 4,5 mg/ml, aufweist bzw. enthält. Insbesondere hat die Anmelderin gefunden, dass, wie zuvor angeführt, in Bezug auf die in Rede stehenden Konzentrationsbereiche - insbesondere auch im Zusammenspiel mit der Komponente (b) - ein entsprechendes Stabilitäts- und Wirksamkeitsmaximum im Hinblick auf die erfindungsgemäße Zusammensetzung vorliegt.

Darüber hinaus kommt auch dem Molekulargewicht (synonym auch als "Molmasse" bezeichnet) der Komponente (a) eine hohe Bedeutung zu:
So kann es erfindungsgemäß vorgesehen sein, dass das Chondroitinsulfat bzw. das physiologisch verträgliche Chondroitinsulfat-Salz (Komponente (a)) ein zahlenmittleres Molekulargewicht (Molmasse) Mₙ im Bereich von 2 kDa bis 200 kDa, insbesondere im Bereich von 5 kDa bis 150 kDa, vorzugsweise im Bereich von 10 kDa bis 100 kDa, bevorzugt im Bereich von 12 kDa bis 75 kDa, aufweist.

Insbesondere kann das Chondroitinsulfat bzw. das physiologisch verträgliche Chondroitinsulfat-Salz (Komponente (a)) ein gewichtsmittleres Molekulargewicht (Molmasse) M_{w} im Bereich von 10 kDa bis 200 kDa, insbesondere im Bereich von 15 kDa bis 175 kDa, vorzugsweise im Bereich von 20 kDa bis 150 kDa, bevorzugt im Bereich von 30 kDa bis 120 kDa, aufweisen.

Zudem kann das Chondroitinsulfat bzw. das physiologisch verträgliche Chondroitinsulfat-Salz (Komponente (a)) ein zentrifugenmittleres Molekulargewicht (Molmasse) M_{z} im Bereich von 30 kDa bis 1.000 kDa, insbesondere im Bereich von 40 kDa bis 800 kDa, vorzugsweise im Bereich von 50 kDa bis 600 kDa, bevorzugt im Bereich von 100 kDa bis 450 kDa, aufweisen.

In diesem Zusammenhang kommt auch dem Polydispersitätsindex (PDI) eine entsprechende Bedeutung zu: Diesbezüglich kann es erfindungsgemäß vorgesehen sein, dass das Chondroitinsulfat bzw. das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ (M_{w} : Mₙ), von mindestens 1, insbesondere mindestens 1,2, vorzugsweise mindestens 1,5, bevorzugt mindestens 2, aufweist.

Zudem kann das Chondroitinsulfat bzw. das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ, von höchstens 30, insbesondere höchstens 20, vorzugsweise höchstens 10, bevorzugt höchstens 8, aufweisen.

Weiterhin kann das Chondroitinsulfat bzw. das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ, im Bereich von 1 bis 30, insbesondere im Bereich von 1,2 bis 20, vorzugsweise im Bereich von 1,5 bis 10, bevorzugt im Bereich von 2 bis 8, aufweisen.

Die vorgenannten Eigenschaften der Komponente (a) hinsichtlich des Molekulargewichts bzw. der Molmasse bzw. des diesbezüglichen Polydispersitätsindexes führen - ohne sich auf diese Theorie beschränken oder festlegen zu wollen - zu einer weiterführend verbesserten Ausbildung der der erfindungsgemäßen Zusammensetzung zugrundeliegenden Matrix, insbesondere auch im Hinblick auf die Ausbildung eines Hydrogels, mit entsprechender Stabilisierung der jeweiligen Wirk- bzw. Inhaltsstoffe. Gleichermaßen führen die in Rede stehenden Molekulargewichte bzw. Molmassen in Bezug auf Komponente (a) auch zu einer guten Wirkeffizienz der erfindungsgemäßen Zusammensetzung im Hinblick auf die zugrundeliegende Erkrankung, insbesondere - gleichermaßen ohne sich auf diese Theorie beschränken oder festlegen zu wollen - infolge einer guten bzw. optimalen Wechselwirkung mit bzw. Anhaftung an dem Urothel der Harnblase.

Ohne sich diesbezüglich auf eine Theorie beschränken bzw. festlegen zu wollen, eignet sich dieses spezielle Chondroitinsulfat aufgrund seiner molekularen Struktur in besonderer Weise, um - insbesondere in Verbindung mit der Komponente (b) - die Glykosaminoglykanschicht des Urothels zu regenerieren bzw. gewissermaßen aufzufüllen, wodurch die Permeabilität dieser Schicht erniedrigt wird. Hierdurch wird die Schutzfunktion der Glykosaminoklykanschicht bzw. der Schleimschicht des Urothels (Mucin-Layer) erhöht, so dass es zu einer deutlichen Linderung bzw. sogar Ausheilung der Krankheitssymptome kommen kann.

Was die Bestimmung des Molekulargewichts der erfindungsgemäß eingesetzten Komponente (a) anbelangt, so wird das zahlenmittlere Molekulargewicht Mₙ bzw. das gewichtsmittlere Molekulargewicht M_{w} bzw. das zentrifugenmittlere Molekulargewicht M_{z} und/oder der Polydispersitätsindex (PDI) des Chondroitinsulfats bzw. des physiologisch verträglichen Chondroitinsulfat-Salzes (Komponente (a)) insbesondere mittels Gelpermeationschromatographie (GPC) bzw. gemäß der DIN 55672-3:2016-03 bestimmt.

Insbesondere können das zahlenmittlere Molekulargewicht Mₙ bzw. das gewichtsmittlere Molekulargewicht M_{w} bzw. das zentrifugenmittlere Molekulargewicht M_{z} bzw. der Polydispersitätsindex (PDI) des Chondroitinsulfats bzw. des physiologisch verträglichen Chondroitinsulfat-Salzes (Komponente (a)) mittels Gelpermeationschromatographie (GPC), insbesondere bei einer Temperatur im Bereich von 20 °C bis 40 °C bzw. mit 0,1 mol/l NaCI-Lösung in deionisiertem Wasser als Elutionsmittel und/oder an einer Lösung von Chondroitinsulfat und/oder des physiologisch verträglichen Chondroitinsulfat-Salzes mit einer Konzentration von 3 g/l und/oder unter Verwendung eines Dextran/Pullulan-Standards als Kalibrierreagenz und/oder gemäß DIN 55672-3:2016-03, bestimmt sein.

In erfindungsgemäß bevorzugter Weise kann das Chondroitinsulfat bzw. das physiologisch verträgliche Chondroitinsulfat-Salz (Komponente (a)) marinen Ursprungs sein. Diesbezüglich kann das Chondroitinsulfat bzw. das physiologisch verträgliche Chondroitinsulfat-Salz (Komponente (a)) aus Knorpelfischen, insbesondere Haien, vorzugsweise Haiknorpel, gewonnen sein. Die Anmelderin hat diesbezüglich völlig überraschend gefunden, dass ein derartiges Chondroitinsulfat marinen Ursprungs insbesondere bei der Behandlung von vorzugsweise entzündlichen Erkrankungen des Urogentinaltraktes, wie Cystitis, zu besonders guten Ergebnissen führt, insbesondere wenn dies in zweckgerichteter Kombination mit der Hyaluronsäure gemäß Komponente (b) eingesetzt wird. Die diesbezügliche Isolierung der Wirksubstanzen ist dem Fachmann als solche geläufig, so dass es diesbezüglich keiner weiterführender Ausführungen bedarf.

Erfindungsgemäß ist es insbesondere vorgesehen, dass das Chondroitinsulfat bzw. das physiologisch verträgliche Chondroitinsulfat-Salz (Komponente (a)) in Form eines Alkalisalzes, vorzugsweise in Form eines Natrium-Salzes, vorliegt, was im Rahmen der vorliegenden Erfindung zu besonders guten Ergebnissen führt.

Erfindungsgemäß kann das Chondroitinsulfat bzw. das physiologisch verträgliche Chondroitinsulfat-Salz (Komponente (a)) vorzugsweise in Form von Chondroitinsulfat-Natrium vorliegen.

Erfindungsgemäß kann das Chondroitinsulfat bzw. das physiologisch verträgliche Chondroitinsulfat-Salz (Komponente (a)) zudem ausgewählt sein aus der Gruppe von Chondroitin-4-sulfat, Chondroitin-6-sulfat, Chondroitin-2,6-sulfat, Chondroitin-4,6-sulfat und deren Kombinationen oder Mischungen, vorzugsweise Chondroitin-2,6-sulfat (Chondroitinsulfat D). Auch die vorgenannten speziellen Chondroitinsulfate werden erfindungsgemäß in bevorzugter Art und Weise in Form ihrer Alkalisalze, vorzugsweise Natriumsalze, eingesetzt, was mit besonders guten Ergebnissen hinsichtlich der Behandlung von vorzugsweise entzündlichen Erkrankungen des Urogenitaltraktes, wie Cystitis, einhergeht.

Im Rahmen der vorliegenden Erfindung kann die Komponente (a) der erfindungsgemäßen Zusammensetzung vorzugsweise auf einer sterilen und/oder hochgereinigten Lösung oder Suspension insbesondere des Natriumsalzes von Chondroitinsulfat basieren.

Im Rahmen der vorliegenden Erfindung einsetzbares Chondroitinsulfat bzw. Chondroitinsulfat-Salz ist im Allgemeinen kommerziell erhältlich, beispielsweise von Nexira, Rouen (FR), Artesan Pharma GmbH & Co. KG oder von Pharma Greven GmbH, Greven (DE).

Für weitergehende Einzelheiten zum Begriff der Chondroitinsulfate kann auf RÖMPP Chemielexikon, 10. Auflage, Band1, 1996, Georg Thieme Verlag Stuttgart/New York, Seite 736, Stichwort: "Chondroitinsulfate", sowie auf die dort referierte Literatur verwiesen werden, wobei der gesamte Offenbarungsgehalt der vorgenannten Literatur hiermit durch Bezugnahme vollumfänglich eingeschlossen ist.

Was die weitere Komponente in Form der Hyaluronsäure bzw. des physiologisch verträglichen Hyaluronsäure-Salzes gemäß Komponente (b) anbelangt, welche für die erfindungsgemäße Zusammensetzung eingesetzt wird, so ist diesbezüglich insbesondere Folgendes anzuführen:

Erfindungsgemäß kann es im Allgemeinen vorgesehen sein, dass die Zusammensetzung die Hyaluronsäure bzw. das physiologisch verträgliche Hyaluronsäure-Salz (Komponente (b)) in einer Konzentration von (16 ± 1,2) mg/ml, insbesondere in einer Konzentration von (16 ± 0,8) mg/ml, vorzugsweise in einer Konzentration von (16 ± 0,4) mg/ml, besonders bevorzugt in einer Konzentration von etwa 16 mg/ml, enthält. In den vorgenannten Konzentrationsbereichen werden besonders gute Eigenschaften im Hinblick auf Stabilität und Wirksamkeit erhalten, insbesondere auf Basis eines Zusammenwirkens insbesondere mit den Komponenten (a) und/oder (c) sowie dem speziellen pH-Wert der Zusammensetzung nach der Erfindung.

Insbesondere im Hinblick auf die Stabilität, vorzugsweise Lagerungsstabilität, der erfindungsgemäßen Zusammensetzung sowie deren Wirksamkeit bei der Behandlung von vorzugsweise entzündlichen Erkrankungen des Urogenitaltraktes, insbesondere Cystitis, vorzugsweise interstitieller Cystitis, werden zudem besonders gute Ergebnisse erreicht, wenn die Hyaluronsäure ein spezielles Molekulargewicht bzw. eine spezielle Molmasse aufweist.

In diesem Zusammenhang ist es erfindungsgemäß bevorzugt, wenn die Hyaluronsäure bzw. das physiologisch verträgliche Hyaluronsäure-Salz (Komponente (b)) ein zahlenmittleres Molekulargewicht (Molmasse) Mₙ im Bereich von 10 kDa bis 300 kDa, insbesondere im Bereich von 20 kDa bis 275 kDa, vorzugsweise im Bereich von 30 kDa bis 260 kDa, bevorzugt im Bereich von 50 kDa bis 250 kDa, besonders bevorzugt im Bereich von 75 kDa bis 200 kDa, aufweist.

Insbesondere kann die Hyaluronsäure bzw. das physiologisch verträgliche Hyaluronsäure-Salz (Komponente (b)) ein gewichtsmittleres Molekulargewicht (Molmasse) M_{w} im Bereich von 10 kDa bis 500 kDa, insbesondere im Bereich von 20 kDa bis 450 kDa, vorzugsweise im Bereich von 50 kDa bis 425 kDa, bevorzugt im Bereich von 100 kDa bis 400 kDa, besonders bevorzugt im Bereich von 150 kDa bis 395 kDa, aufweisen.

Darüber hinaus kann die Hyaluronsäure bzw. das physiologisch verträgliche Hyaluronsäure-Salz (Komponente (b)) ein zentrifugenmittleres Molekulargewicht (Molmasse) M_{z} im Bereich von 80 kDa bis 1.500 kDa, insbesondere im Bereich von 100 kDa bis 1.250 kDa, vorzugsweise im Bereich von 200 kDa bis 1.000 kDa, bevorzugt im Bereich von 300 kDa bis 750 kDa, aufweisen.

Auf Basis der vorgenannten Molekulargewichte resultiert insbesondere in zweckgerichteter Abstimmung und Kombination mit den entsprechenden Molekulargewichten der Komponente (a) eine besonders gute Stabilisierung und Wirkeffizienz der erfindungsgemäßen Zusammensetzung, insbesondere da - ohne sich auf diese Theorie beschränken bzw. festlegen zu wollen - auf Basis der vorgenannten Molekulargewichte eine optimale Matrix- bzw. Hydrogelausbildung mit entsprechender Stabilisierung und weiterführend optimierter Wechselwirkung insbesondere mit dem Urothel der Harnblase gewährleistet ist.

Diesbezüglich kommt auch dem Polydispersitätsindex der erfindungsgemäß eingesetzten Komponente (b) eine Bedeutung zu:
So kann es erfindungsgemäß vorgesehen sein, dass die Hyaluronsäure bzw. das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, von mindestens 1, insbesondere mindestens 1,1, vorzugsweise mindestens 1,2, bevorzugt mindestens 1,3, besonders bevorzugt mindestens 1,4, aufweist.

Insbesondere kann die Hyaluronsäure bzw. das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, von höchstens 50, insbesondere höchstens 25, vorzugsweise höchstens 10, bevorzugt höchstens 5, besonders bevorzugt höchstens 3, aufweisen.

Weiterhin kann es erfindungsgemäß vorgesehen sein, dass die Hyaluronsäure bzw. das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, im Bereich von 1 bis 50, insbesondere im Bereich von 1,1 bis 25, vorzugsweise im Bereich von 1,2 bis 10, bevorzugt im Bereich von 1,3 bis 5, besonders bevorzugt im Bereich von 1,4 bis 3, aufweist.

Die vorgenannten Molekulargewichte können mit dem Fachmann an sich bekannten Methoden bestimmt werden. Erfindungsgemäß kann das zahlenmittlere Molekulargewicht Mₙ bzw. das gewichtsmittlere Molekulargewicht M_{w} und/oder das zentrifugenmittlere Molekulargewicht M_{z} und/oder der Polydispersitätsindex (PDI) der Hyaluronsäure und/oder des physiologisch verträglichen Hyaluronsäure-Salzes (Komponente (b)) insbesondere mittels Gelpermeationschromatographie (GPC) und/oder gemäß DIN 55672-3:2016-03 bestimmt sein.

Zudem kann das zahlenmittlere Molekulargewicht Mₙ bzw. das gewichtsmittlere Molekulargewicht M_{w} bzw. das zentrifugenmittlere Molekulargewicht M_{z} bzw. der Polydispersitätsindex (PDI) der Hyaluronsäure und/oder des physiologisch verträglichen Hyaluronsäure-Salzes (Komponente (b)) mittels Gelpermeationschromatographie (GPC), insbesondere bei einer Temperatur im Bereich von 20 °C bis 40 °C und/oder mit 0,1 mol/l NaCI-Lösung in deionisiertem Wasser als Elutionsmittel und/oder an einer Lösung von Hyaluronsäure und/oder des physiologisch verträglichen Hyaluronsäure-Salzes mit einer Konzentration von 3 g/l und/oder unter Verwendung eines Dextran/Pullulan-Standards als Kalibrierreagenz und/oder gemäß DIN 55672-3:2016-03, bestimmt sein.

Erfindungsgemäß kann es insbesondere vorgesehen sein, dass das Verhältnis des jeweiligen Molekulargewichts Mₙ, M_{w} oder M_{z} von Chondroitinsulfat bzw. des physiologisch verträglichen Chondroitinsulfat-Salzes (Komponente (a)) zu dem korrespondierenden Molekulargewicht Mₙ, M_{w} oder M_{z} der Hyaluronsäure und/oder des physiologisch verträglichen Hyaluronsäure-Salzes (Komponente (b)) in einem Bereich von 1 : 3 bis 1 : 4, insbesondere 1 : 1 bis 1 : 100, vorzugsweise 1 : 1,5 bis 1 : 50, bevorzugt 1 : 2 bis 1 : 25, besonders bevorzugt 1 : 3 bis 1 : 10, liegt. Infolge der gezielten Abstimmung der jeweiligen Molekulargewichte bzw. Molmassen der Komponente (a) und der Komponente (b) ergeben sich besonders gute Ergebnisse hinsichtlich der Stabilisierung sowie Wirkeffizienz der erfindungsgemäßen Zusammensetzung, insbesondere da - ohne sich auf diese Theorie beschränken bzw. festlegen zu wollen - diesbezüglich eine Wirkergänzung bzw. hohe Kompatibilität der in Rede stehenden Komponenten (a) und (b) untereinander vorliegt.

Erfindungsgemäß kann es zudem insbesondere vorgesehen sein, dass die Hyaluronsäure bzw. das physiologisch verträgliche Hyaluronsäure-Salz (Komponente (b)) nichttierischen Ursprungs ist.

In diesem Zusammenhang kann die Hyaluronsäure bzw. das physiologisch verträgliche Hyaluronsäure-Salz (Komponente (b)) bakteriellen bzw. fermentativen Ursprungs sein. Diesbezüglich kann die Hyaluronsäure bzw. das physiologisch verträgliche Hyaluronsäure-Salz (Komponente (b)) vorzugsweise fermentativ von Bakterien der Gattung *Streptococcus,* insbesondere *Streptococcus lancefields,* bevorzugt *Streptococcus lancefields* Stamm A, gewonnen sein. Die diesbezügliche Gewinnung bzw. Isolierung der Wirksubstanzen ist dem Fachmann als solche geläufig, so dass es diesbezüglich keiner weiterführender Ausführungen bedarf.

In diesem Zusammenhang hat die Anmelderin gleichermaßen in überraschender Weise herausgefunden, dass durch die Verwendung einer nichttierischen Hyaluronsäure der vorgenannten Art auch die pharmazeutische Wirksamkeit der erfindungsgemäßen Zusammensetzung in Bezug auf die Behandlung von vorzugsweise entzündlichen Erkrankungen der Harnblase, wie Cystitis, vorzugsweise interstitieller Cystitis, signifikant verbessert ist. Ohne sich auf eine spezielle Theorie beschränken bzw. festlegen zu wollen, kann dies damit begründet werden, dass es sich bei der nichttierischen, insbesondere bakteriell gewonnenen Hyaluronsäure um ein besonders reines Produkt mit definierten chemischen bzw. physikalischen Eigenschaften handelt, welches hochwirksam ist. Die definierte Ausbildung der Hyaluronsäure mit der hohen Reinheit ist gleichermaßen auch der Stabilität der Zusammensetzung zuträglich, da keine der Stabilität abträglichen Verunreinigungen zugegen sind. Zudem ist die nichttierische Hyaluronsäure gut verträglich, da eine Kontamination bzw. Verunreinigung mit anderen Stoffen, wie es oftmals bei tierisch gewonnenen Produkten der Fall ist, nicht vorliegt. Somit weist Hyaluronsäure nichttierischen Ursprungs eine besonders hohe Reinheit und Homogenität auf, was auch der Stabilität, insbesondere Lagerungsstabilität, der erfindungsgemäßen Zusammensetzung zuträglich ist.

Gemäß einer erfindungsgemäß bevorzugten Ausführungsform kann die Hyaluronsäure bzw. das physiologisch verträgliche Hyaluronsäure-Salz (Komponente (b)) in Form eines Alkalisalzes, vorzugsweise in Form eines Natrium-Salzes, und/oder in Form eines Alkalihyaluronates vorliegen. Dabei werden gleichermaßen besonders gute Ergebnisse erhalten, wenn die Hyaluronsäure bzw. das physiologisch verträgliche Hyaluronsäure-Salz (Komponente (b)) in Form von Natriumhyaluronat vorliegt.

Die Komponente (b) der erfindungsgemäßen Zusammensetzung kann insbesondere auf Basis einer sterilen bzw. hochgereinigten Lösung oder Suspension insbesondere des Natriumsalzes der Hyaluronsäure eingesetzt werden.

Im Rahmen der vorliegenden Erfindung einsetzbare Hyaluronsäure bzw. einsetzbares Hyaluronsäure-Salz ist im Allgemeinen kommerziell erhältlich, beispielsweise von Vivatis Pharma Gmbh, Hamburg (DE), Contipro S.A., Dolni Dobrouc (CZ) oder von GFN Herstellung von Naturextrakten GmbH, Wald-Michelbach (DE).

Für weitergehende Einzelheiten zum Begriff der Hyaluronsäure bzw. deren physiologisch verträglichen Salze kann auf RÖMPP Chemielexikon, 10. Auflage, Band 3, 1997, Georg Thieme Verlag Stuttgart/New York, Seite 1820, Stichwort: "Hyaluronsäure", sowie auf die dort referierte Literatur verwiesen werden, wobei der Gesamtoffenbarungsgehalt der vorgenannten Literatur hiermit durch Bezugnahme vollumfänglich eingeschlossen ist.

Erfindungsgemäß kann es zudem vorgesehen sein, dass das Chondroitinsulfat bzw. das physiologisch verträgliche Chondroitinsulfat-Salz (Komponente (a)) einerseits und die Hyaluronsäure und/oder das physiologisch verträgliche Hyaluronsäure-Salz (Komponente (b)) andererseits in der Zusammensetzung in einem Gewichtsverhältnis (Konzentrationsverhältnis) von Komponente (a) einerseits zu Komponente (b) andererseits [(a) : (b)] in einem Bereich von 1 : 3 bis 1 : 4, insbesondere 1 : 3,2 bis 1 : 3,8, vorzugsweise 1 : 3,4 bis 1 : 3,6, besonders bevorzugt etwa 1 : 3,55, vorliegen.

Denn der Anmelderin ist es insgesamt gelungen, durch die gezielte Anpassung und Abstimmung der jeweiligen Wirksubstanzen auf Basis der Komponenten (a) und (b) in Bezug auf die Glykosaminoglykanschicht des Blasenurothels zu einer besonders guten Wirksamkeit hinsichtlich der Behandlung der zugrundeliegenden Erkrankungen zu gelangen, da - ohne sich auf eine spezielle Theorie beschränken bzw. festlegen zu wollen - insbesondere bei den vorgenannten Mengenverhältnissen eine besonders gute Wechselwirkung bzw. Integration der Wirksubstanzen in die Glykosaminoglykanschicht des Blasenurothels bzw. eine gute Regeneration der Glykosaminoglykanschicht des Blasenurothels vorliegt, insbesondere in Verbindung mit den vorgenannten jeweils speziellen Molekulargewichten bzw. Molmassen der Komponenten (a) bzw. (b). Folglich wird, wie zuvor angeführt, die Permeabilität des Urothels in signifikanter Weise erniedrigt, was zu einer deutlichen Minderung der mit der zugrundeliegenden Erkrankung, insbesondere Cystitis, einhergehenden Symptome verbunden ist, insbesondere da Reizstoffe nicht mehr so tief in das Urothel bzw. in darunterliegende Schichten eindringen können. Die vorgenannten Gewichtsverhältnisse haben auch einen positiven Effekt in Bezug auf die Stabilität der Zusammensetzung gemäß dem chemischen Puffersystem.

Was darüber hinaus die erfindungsgemäß eingesetzte Komponente (c) anbelangt, so kann die Zusammensetzung das Dihydrogenphosphat/Monohydrogenphosphat-Puffersystem (Komponente (c)) in einer Gesamtkonzentration an Dihydrogenphosphat/Monohydrogenphosphat-Puffersystem (Komponente (c)) von (1,75 ± 1,65) mg/ml, insbesondere in einer Gesamtkonzentration von (1,75 ± 1,5) mg/ml, vorzugsweise in einer Gesamtkonzentration von (1,75 ± 1,25) mg/ml, bevorzugt in einer Gesamtkonzentration von (1,75 ± 1) mg/ml, besonders bevorzugt in einer Gesamtkonzentration von (1,75 ± 0,9) mg/ml, ganz besonders bevorzugt in einer Gesamtkonzentration von (1,75 ± 0,8) mg/ml, noch weiter bevorzugt in einer Gesamtkonzentration von etwa 1,75 mg/ml, enthalten.

Im Rahmen der vorliegenden Erfindung hat sich der gezielte Einsatz eines speziellen Puffersystems, insbesondere chemischen Puffersystems, gemäß Komponente (c) auf Basis des Dihydrogenphosphat/Monohydrogenphosphat-Puffersystems als besonders vorteilhaft erwiesen. Bei Verwendung dieses speziellen Puffersystems, insbesondere bei gleichzeitiger Einhaltung der entsprechenden Konzentrationen des Puffers bzw. der Pufferkomponenten, wird einer unerwünschten Veränderung des pH-Wertes bzw. einem unerwünschten Abbau der Wirkstoffe bei Lagerung auch über einen längeren Zeitraum in besonderem Maße entgegengewirkt und wird die Lagerfähigkeit der erfindungsgemäßen Zusammensetzung somit in besonderem Maße verbessert, wie zuvor angeführt.

Insbesondere kann die Zusammensetzung das Dihydrogenphosphat/Monohydrogenphosphat-Puffersystem (Komponente (c)) in einer Gesamtmenge an Dihydrogenphosphat/Monohydrogenphosphat-Puffersystem (Komponente (c)) von (87,5 ± 85) mg, insbesondere in einer Gesamtmenge von (87,5 ± 75) mg, vorzugsweise in einer Gesamtmenge von (87,5 ± 62,5) mg, bevorzugt in einer Gesamtmenge von (87,5 ± 50) mg, besonders bevorzugt in einer Gesamtmenge von (87,5 ± 45) mg, ganz besonders bevorzugt in einer Gesamtmenge von (87,5 ± 40) mg, noch weiter bevorzugt in einer Gesamtmenge von etwa 87,5 mg, enthalten.

Zudem kann die Zusammensetzung das Dihydrogenphosphat des Dihydrogenphosphat/Monohydrogenphosphat-Puffersystems (Komponente (c)), insbesondere bezogen auf die Einwaage bei Herstellung und/oder Bereitstellung der Zusammensetzung, in einer Konzentration von (0,2 ± 0,19) mg/ml, insbesondere in einer Konzentration von (0,2 ± 0,15) mg/ml, vorzugsweise in einer Konzentration von (0,2 ± 0,125) mg/ml, bevorzugt in einer Konzentration von (0,2 ± 0,1) mg/ml, besonders bevorzugt in einer Konzentration von etwa 0,2 mg/ml, enthalten.

Gleichermaßen hat es sich als vorteilhaft erwiesen, wenn die Zusammensetzung das Monohydrogenphosphat des Dihydrogenphosphat/Monohydrogenphosphat-Puffersystems (Komponente (c)), insbesondere bezogen auf die Einwaage bei Herstellung und/oder Bereitstellung der Zusammensetzung, in einer Konzentration von (1,5 ± 1,4) mg/ml, insbesondere in einer Konzentration von (1,5 ± 1,25) mg/ml, vorzugsweise in einer Konzentration von (1,5 ± 1,1) mg/ml, bevorzugt in einer Konzentration von (1,5 ± 1) mg/ml, besonders bevorzugt in einer Konzentration von (1,5 ± 0,75) mg/ml, ganz besonders bevorzugt in einer Konzentration von etwa 1,5 mg/ml, enthält.

Hinsichtlich der Stabilisierung der erfindungsgemäßen Zusammensetzung hat es sich zudem als besonders vorteilhaft erwiesen, wenn die Zusammensetzung das Dihydrogenphosphat/Monohydrogenphosphat-Puffersystem (Komponente (c)) in einem Gewichtsverhältnis (Konzentrationsverhältnis) von Dihydrogenphosphat zu Monohydrogenposphat [Dihydrogenphosphat: Monohydrogenposphat], insbesondere bezogen auf die Einwaage bei Herstellung und/oder Bereitstellung der Zusammensetzung, im Bereich von 2 : 1 bis 1 : 100, vorzugsweise im Bereich von 1 : 1 bis 1 : 75, bevorzugt im Bereich von 1 : 2 bis 1 : 50, besonders bevorzugt im Bereich von 1 : 5 bis 1 : 25, enthält.

Gemäß einer erfindungsgemäßen Ausführungsform kann das Dihydrogenphosphat/Monohydrogenphosphat-Puffersystem (Komponente (c)) als Alkalidihydrogenphosphat/Alkalimonohydrogenposphat-Puffersystem vorliegen bzw. ausgebildet sein. In bevorzugter Weise kann das Alkalimetall ausgewählt sein aus Natrium und/oder Kalium, insbesondere Natrium.

Zudem kann das Dihydrogenphosphat/Monohydrogenphosphat-Puffersystem (Komponente (c)) als Natriumdihydrogenphosphat/Natriummonohydrogenposphat-Puffersystem, vorliegen bzw. ausgebildet sein.

Darüber hinaus kann das Dihydrogenphosphat/Monohydrogenphosphat-Puffersystem (Komponente (c)) als NaH₂(PO₄) / Na₂H(PO₄)-Puffersystem, insbesondere als NaH₂(PO₄) · 2H₂O/Na₂H(PO₄) · 2H₂O-Puflersystem, vorliegen bzw. ausgebildet sein.

Im Allgemeinen kann das Dihydrogenphosphat/Monohydrogenphosphat-Puffersystem (Komponente (c)) zur Einstellung bzw. Konstanthaltung des pH-Wertes der Zusammensetzung dienen bzw. eingesetzt sein. Hierdurch erfolgt eine weiterführende Stabilisierung der erfindungsgemäßen Zusammensetzung, einhergehend mit einer besonders guten Verträglichkeit der Zusammensetzung nach der Erfindung bei deren Anwendung bzw. Applikation.

Im Rahmen der vorliegenden Erfindung einsetzbare Phosphatpuffer sind im Allgemeinen kommerziell erhältlich, beispielsweise von Merck KGaA, Darmstadt (DE).

Zum Begriff des Puffers bzw. des chemischen Puffersystems kann insbesondere verwiesen werden auf RÖMPP Lexikon Chemie, 10. Auflage, Georg-Thieme-Verlag, Stuttgart/New York, Band 5, 1998, Seiten 3618/3619, Stichwort: "Puffer", sowie auf die dort referierte Literatur, wobei der gesamte Offenbarungsgehalt der vorgenannten Literatur hiermit durch Bezugnahme vollumfänglich eingeschlossen ist.

In Ergänzung zu den vorgenannten Wirksubstanzen bzw. Inhaltsstoffen kann die erfindungsgemäße Zusammensetzung gemäß Komponente (d) mindestens einen physiologisch verträglichen Elektrolyten aufweisen: In diesem Zusammenhang kann die Zusammensetzung den Elektrolyten (Komponente (d)) in einer Konzentration von (8 ± 6) mg/ml, insbesondere in einer Konzentration von (8 ± 4) mg/ml, vorzugsweise in einer Konzentration von (8 ± 2) mg/ml, bevorzugt in einer Konzentration von (8 ± 1) mg/ml, besonders bevorzugt in einer Konzentration von etwa 8 mg/ml, enthalten.

Insbesondere kann der Elektrolyt (Komponente (d)) in Form eines Alkalisalzes, insbesondere in Form eines Alkalichlorids, vorzugsweise in Form von Natriumchlorid, vorliegen. Erfindungsgemäß ist es dabei bevorzugt, wenn der Elektrolyt (Komponente (d)) Natriumchlorid ist.

Im Rahmen der vorliegenden Erfindung einsetzbare Elektrolyte sind im Allgemeinen kommerziell erhältlich, beispielsweise von Merck KGaA, Darmstadt (DE).

Infolge der Verwendung eines Elektrolyten können mögliche osmotische Effekte im Rahmen der Applikation bzw. Behandlung mit der erfindungsgemäßen Zusammensetzung so gering wie möglich gehalten werden, was die Verträglichkeit der erfindungsgemäßen Zusammensetzung weiter erhöht.

Gemäß einer erfindungsgemäß bevorzugten Ausführungsform betrifft die vorliegende Erfindung somit gemäß dem vorliegenden Aspekt auch eine Zusammensetzung, insbesondere pharmazeutische Zusammensetzung, vorzugsweise zur prophylaktischen bzw. therapeutischen Behandlung von vorzugsweise entzündlichen Erkrankungen des Urogenitaltraktes, insbesondere von entzündlichen Erkrankungen der Harnblase, bevorzugt von Cystitis, insbesondere eine wie zuvor beschriebene Zusammensetzung,
wobei die Zusammensetzung in Kombination und jeweils in wirksamen, insbesondere pharmazeutisch wirksamen Mengen
(a) Chondroitinsulfat-Natrium (Natrium-Chondroitinsulfat) in einer Konzentration von (4,5 ± 0,5) mg/ml (Komponente (a));
(b) Natriumhyaluronat in einer Konzentration von (16 ± 1,6) mg/ml (Komponente (b));
(c) ein Alkalidihydrogenphosphat/Alkalimonohydrogenposphat-Puffersystem (Komponente (c)) insbesondere in einer Gesamtkonzentration an Alkalidihydrogenphosphat/Alkalimonohydrogenposphat-Puffersystem von (1,75 ± 1,65) mg/ml;
(d) mindestens einen physiologisch verträglichen Elektrolyten (Komponente (d)), vorzugsweise Natriumchlorid, insbesondere in einer Konzentration von (8 ± 6) mg/ml;
enthält,
wobei die Zusammensetzung einen pH-Wert im Bereich von 6,1 bis 7,9 aufweist und/oder wobei die Zusammensetzung auf einen pH-Wert im Bereich von 6,1 bis 7,9 eingestellt ist.

Was den pH-Wert der erfindungsgemäßen Zusammensetzung im Allgemeinen anbelangt, so kann die Zusammensetzung zudem einen pH-Wert im Bereich von 6,6 bis 7,7, insbesondere im Bereich von 6,9 bis 7,6, vorzugsweise im Bereich von 7,1 bis 7,4, aufweisen. Insbesondere kann der pH-Wert der Zusammensetzung im Bereich von 6,6 bis 7,7, insbesondere im Bereich von 6,9 bis 7,6, vorzugsweise im Bereich von 7,1 bis 7,4, konstant gehalten bzw. eingestellt sein. Der pH-Wert kann vorzugsweise mittels des Alkalidihydrogenphosphat/Alkalimonohydrogenposphat-Puffersystems (Komponente (c)) eingestellt bzw. vorgegeben sein.

Bei Einhaltung der vorgenannten pH-Wertebereiche wird einem unerwünschten Abbau der Wirkstoffe bei Lagerung auch über einen längeren Zeitraum in besonderem Maße entgegengewirkt und wird auch die Lagerfähigkeit der erfindungsgemäßen Zusammensetzung in besonderem Maße verbessert.

Aufgrund der vorgenannten Eigenschaften im Hinblick auf den pH-Wert ist die erfindungsgemäße Zusammensetzung zudem besonders gut verträglich.

Im Rahmen der vorliegenden Erfindung kann die Bestimmung des pH-Wertes mit dem Fachmann an sich bekannten Methoden erfolgen. Insbesondere kann die Bestimmung des pH-Wertes auf Basis einer potentiometrischen Analyse erfolgen. Vorzugsweise kann die Bestimmung des pH-Wertes gemäß der Methode nach Ph. Eur. [Pharmacopoea Europaea], 9th Edition (9.0), 2017, 9. Auflage Englisch, Abschnitt 2.2.3. "Potentiometric determination of pH" erfolgen.

Für weitergehende Einzelheiten zum Begriff des pH-Wertes kann insbesondere verwiesen werden auf RÖMPP Lexikon Chemie, 10. Auflage, Georg-Thieme-Verlag, Stuttgart/New York, Band 4, 1998, Seiten 3230 bis 3232, Stichwort: "pH", sowie auf die dort referierte Literatur, wobei der gesamte Offenbarungsgehalt der vorgenannten Literatur hiermit durch Bezugnahme vollumfänglich eingeschlossen ist.

Was die erfindungsgemäße Zusammensetzung weiterhin anbelangt, so kann diese bei einer Temperatur von 20 °C eine dynamische Viskosität von mindestens 2.000 mPas, insbesondere mindestens 4.000 mPas, vorzugsweise mindestens 5.000 mPas, bevorzugt mindestens 5.250 mPas, aufweisen. Zudem kann die Zusammensetzung bei einer Temperatur von 20 °C eine dynamische Viskosität von höchstens 7.900 mPas, insbesondere höchstens 6.900 mPas, vorzugsweise höchstens 6.000 mPas, bevorzugt höchstens 5.750 mPas, aufweisen.

Erfindungsgemäß kann es insbesondere vorgesehen sein, dass die Zusammensetzung bei einer Temperatur von 20 °C eine dynamische Viskosität im Bereich von 2.000 mPas bis 7.900 mPas, insbesondere im Bereich von 4.000 mPas bis 6.900 mPas, vorzugsweise im Bereich von 5.000 mPas bis 6.000 mPas, bevorzugt im Bereich von 5.250 mPas bis 5.750 mPas, aufweist.

Aufgrund der erfindungsgemäß vorgesehenen bzw. eingestellten Viskosität ist eine besonders einfache und weniger schmerzhafte Instillation der Zusammensetzung in die Harnblase gewährleistet. Weiterhin liegt aufgrund der erfindungsgemäß vorgesehenen Viskosität eine besonders gute Interaktion der der Zusammensetzung nach der Erfindung zugrundeliegenden Wirkkomponente mit dem Urothel vor, was die Wirksamkeit positiv beeinflusst. Zudem führt die gezielte Einstellung der Viskosität auch zu einer weiterführenden Stabilisierung der Zusammensetzung, insbesondere im Hinblick auf - ohne sich auf diese Theorie beschränken bzw. festlegen zu wollen - die Ausbildung einer definierten Matrix bzw. eines definierten Hydrogels, was mit einer Verbesserung der Lagerungsstabilität der erfindungsgemäßen Zusammensetzung einhergeht.

Die Bestimmung der dynamischen Viskosität kann mit dem Fachmann an sich bekannten Methoden erfolgen. Insbesondere kann die dynamische Viskosität gemäß der Methode nach Ph. Eur. [Pharmacopoea Europaea], 9th Edition (9.0), 2017, 9. Auflage Englisch, Abschnitt 2.2.8. "Viscosity" und Abschnitt 2.2.9. "Capillary viscometer method" bestimmt sein.

Weiterhin kann die erfindungsgemäße Zusammensetzung eine Osmolalität im Bereich von 150 mosmol/kg bis 600 mosmol/kg, insbesondere im Bereich von 200 mosmol/kg bis 550 mosmol/kg, vorzugsweise im Bereich von 250 mosmol/kg bis 500 mosmol/kg, bevorzugt im Bereich von 275 mosmol/kg bis 450 mosmol/kg, besonders bevorzugt im Bereich von 300 mosmol/kg bis 400 mosmol/kg, aufweisen. Die gezielte Einstellung der Osmolalität dient gleichermaßen der weiterführenden Stabilisierung und insbesondere Verbesserung der Verträglichkeit der erfindungsgemäßen Zusammensetzung.

Die Osmolalität kann dabei gemäß dem Fachmann an sich bekannten Methoden bestimmt werden. Insbesondere kann die Osmolalität gemäß der Methode nach Ph. Eur. [Pharmacopoea Europaea], 9th Edition (9.0), 2017, 9. Auflage Englisch, Abschnitt 2.2.35. "Osmolality", bestimmt sein. Die Einstellung der Osmolalität kann beispielsweise auch auf Basis des zuvor genannten Elektrolyten (Komponente (d)), beispielsweise auf Basis von Alkali-Ionen, wie Natrium-Ionen, und Chlorid-Ionen, insbesondere mittels Natriumchlorid, erfolgen.

Darüber hinaus kann die Zusammensetzung bei einer Temperatur von 20 °C und bei einem Druck von 1.013,25 mbar (Atmosphärendruck) eine Dichte im Bereich von 1,001 g/cm³ bis 1,5 g/cm³, insbesondere im Bereich von 1,005 g/cm³ bis 1,25 g/cm³, vorzugsweise im Bereich von 1,0075 g/cm³ bis 1,1 g/cm³, bevorzugt im Bereich von 1,0075 g/cm³ bis 1,075 g/cm³, besonders bevorzugt im Bereich von 1,0075 g/cm³ bis 1,05 g/cm³, aufweisen.

Insbesondere kann die Zusammensetzung bei einer Temperatur von 20 °C und bei einem Druck von 1.013,25 mbar (Atmosphärendruck) eine relative Dichte, bezogen auf reines Wasser, im Bereich von 1,001 bis 1,5, insbesondere im Bereich von 1,005 bis 1,25, vorzugsweise im Bereich von 1,0075 bis 1,1, bevorzugt im Bereich von 1,0075 bis 1,075, besonders bevorzugt im Bereich von 1,0075 bis 1,05, aufweisen.

Im Rahmen der vorliegenden Erfindung kann die Bestimmung der Dichte mit dem Fachmann an sich bekannten Methoden erfolgen. Insbesondere kann die relative Dichte gemäß der Methode nach Ph. Eur. [Pharmacopoea Europaea], 9th Edition (9.0), 2017, 9. Auflage Englisch, Abschnitt 2.2.5. "Relative density" bestimmt sein. Durch die zweckgerichtete Einstellung der Dichte kann die Handhabung und Verträglichkeit der erfindungsgemäßen Zusammensetzung weiterführend verbessert werden.

Darüber hinaus ist es im Rahmen der vorliegenden Erfindung bevorzugt, wenn die Zusammensetzung als wässrige Zusammensetzung vorliegt. In diesem Zusammenhang kann es insbesondere vorgesehen sein, dass die Zusammensetzung wässrig basiert ist bzw. wässrig formuliert vorliegt.

Insbesondere kann die Zusammensetzung in Form einer wässrigen Lösung bzw. wässrigen Suspension, vorzugsweise in Form einer wässrigen Lösung, vorliegen.

In diesem Zusammenhang kann die Zusammensetzung Wasser, insbesondere gereinigtes Wasser, enthalten. Insbesondere kann die Zusammensetzung Wasser als pharmazeutisch verträglichen Träger (Exzipienten) enthalten. Im Allgemeinen kann die Zusammensetzung folglich wässrig basiert ausgebildet sein, insbesondere unter Verwendung von Wasser für Injektionszwecke.

Was die eingesetzte Menge an Wasser anbelangt, so kann diese in weiten Bereichen variieren. Erfindungsgemäß ist es bevorzugt, wenn die Zusammensetzung nach der Erfindung einen Gehalt an Wasser, insbesondere an gereinigtem Wasser, von mindestens 50 Gew.-%, insbesondere mindestens 75 Gew.-%, vorzugsweise mindestens 80 Gew.-%, bevorzugt mindestens 90 Gew.-%, besonders bevorzugt mindestens 95 Gew.-%, bezogen auf die Zusammensetzung, aufweist.

Im Rahmen der vorliegenden Erfindung einsetzbares Wasser, insbesondere Wasser für Injektionszwecke, ist im Allgemeinen kommerziell erhältlich, beispielsweise von Fresenius Kabi Deutschland GmbH, Bad Homburg (DE).

Gemäß einer erfindungsgemäßen Ausführungsform kann es vorgesehen sein, dass die Zusammensetzung nach der Erfindung aus den vorgenannten Komponenten (a), (b), (c) und gegebenenfalls (d) sowie gegebenenfalls Wasser, insbesondere gereinigtem Wasser, besteht.

Darüber hinaus kann es erfindungsgemäß vorgesehen sein, dass die Zusammensetzung nach der Erfindung zumindest im Wesentlichen frei von organischen Lösemitteln und/oder organischen Dispersionsmitteln, insbesondere alkoholisch basierten Lösemitteln und/oder alkoholisch basierten Dispersionsmitteln, ist. Insbesondere kann es erfindungsgemäß vorgesehen sein, dass die Zusammensetzung zumindest im Wesentlichen frei von Alkoholen ist. Hierdurch wird die Verträglichkeit der erfindungsgemäßen Zusammensetzung weiterführend erhöht.

Nicht zuletzt aufgrund der hohen Stabilität, insbesondere Lagerungsstabilität, der erfindungsgemäßen Zusammensetzung kann es im Rahmen der vorliegenden Erfindung auch vorgesehen sein, dass die Zusammensetzung zumindest im Wesentlichen frei von Konservierungsmitteln bzw. zumindest im Wesentlichen frei von Desinfektionsmitteln ist. Auch hierdurch wird die Verträglichkeit der erfindungsgemäßen Zusammensetzung verbessert, da erfindungsgemäß auf den Einsatz von auf das Urothel bzw. die Schleimhaut der Harnblase gegebenenfalls reizend wirkenden Zusatzstoffen verzichtet werden kann.

Wie zuvor angeführt, ist es im Rahmen der vorliegenden Erfindung gelungen, eine Zusammensetzung bereitzustellen, welche sich durch eine hervorragende Stabilität auszeichnet, so dass die Zusammensetzung über große Lagerungszeiträume aufbewahrt werden kann. Auf dieser Basis wird auch die Anwendungssicherheit weiterführend erhöht.

Insbesondere kann die Zusammensetzung bei Temperaturen im Bereich von 20 °C bis 45 °C, bei einem Druck von 1.013,25 mbar (Atmosphärendruck) und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerungsstabil, sein. In diesem Zusammenhang kann die Zusammensetzung zum jeweiligen Lagerungszeitpunkt einen Gesamtgehalt an Abbauprodukten der Komponenten (a) und (b) von höchstens 5 %, insbesondere höchstens 4 %, vorzugsweise höchstens 3 %, bevorzugt höchstens 2 %, besonders bevorzugt höchstens 1 %, bezogen auf die Gesamtkonzentration der Komponenten (a) und (b) bzw. der Edukte, aufweisen. Insbesondere kann die Zusammensetzung eine Stabilität, insbesondere Lagerungsstabilität, unter beschleunigten Alterungsbedingungen gemäß ASTM F 1980 [ASTM F 1980: Standard Guide for Accelerated Aging of Sterile Barrier Systems for Medical Devices; 2007-04] bei einer Alterungstemperatur von 55 °C von mindestens 6 Monate, insbesondere mindestens 12 Monate, vorzugsweise mindestens 24 Monate, bevorzugt mindestens 36 Monate, aufweisen. Insbesondere kann die Zusammensetzung in diesem Zusammenhang zum jeweiligen Lagerungszeitpunkt einen Gesamtgehalt an Abbauprodukten der Komponenten (a) und (b) von höchstens 5 %, insbesondere höchstens 4 %, vorzugsweise höchstens 3 %, bevorzugt höchstens 2 %, besonders bevorzugt höchstens 1 %, bezogen auf die Gesamtkonzentration der Komponenten (a) und (b) bzw. der Edukte, aufweisen.

Darüber hinaus kann sich die erfindungsgemäße Zusammensetzung - sofern überhaupt vorhanden - durch einen geringen Gehalt an Erdalkali-Bestandteilen auszeichnen:

Gemäß einer erfindungsgemäßen Ausführungsform kann die Zusammensetzung eine Erdalkalisalz-Konzentration, insbesondere Calciumsalz-Konzentration bzw. Magnesiumsalz-Konzentration, von höchstens 1 mg/ml, insbesondere höchstens 0,5 mg/ml, vorzugsweise höchstens 0,1 mg/ml, bevorzugt höchstens 0,05 mg/ml, besonders bevorzugt höchstens 0,01 mg/ml, aufweisen.

Im Rahmen der vorliegenden Erfindung kann die Zusammensetzung darüber hinaus zumindest im Wesentlichen frei von Erdalkalisalzen, insbesondere zumindest im Wesentlichen frei von Calciumsalzen und/oder Magnesiumsalzen, sein.

Im Allgemeinen kann die Zusammensetzung nach der Erfindung eine Erdalkalimetall-Konzentration, insbesondere Calcium-Konzentration bzw. Magnesium-Konzentration, von höchstens 1 mg/ml, insbesondere höchstens 0,5 mg/ml, vorzugsweise höchstens 0,1 mg/ml, bevorzugt höchstens 0,05 mg/ml, besonders bevorzugt höchstens 0,01 mg/ml, aufweisen.

Insbesondere kann die Zusammensetzung nach der Erfindung zumindest im Wesentlichen frei von Erdalkalimetallen, insbesondere zumindest im Wesentlichen frei von Calcium bzw. Magnesiumsalzen, sein.

Zudem kann die Zusammensetzung eine Konzentration an bivalenten Ionen, insbesondere bivalenten Kationen, vorzugsweise Erdalkali-Ionen, bevorzugt Calcium-Ionen bzw. Magnesium-Ionen, von höchstens 1 mg/ml, insbesondere höchstens 0,5 mg/ml, vorzugsweise höchstens 0,1 mg/ml, bevorzugt höchstens 0,05 mg/ml, besonders bevorzugt höchstens 0,01 mg/ml, aufweisen.

Insbesondere kann die Zusammensetzung zumindest im Wesentlichen frei von bivalenten Ionen, insbesondere bivalenten Kationen, vorzugsweise Erdalkali-Ionen, bevorzugt Calcium-Ionen und/oder Magnesium-Ionen, sein.

Die erfindungsgemäße Konzeption, wonach die Zusammensetzung nach der Erfindung zumindest im Wesentlichen frei von Erdalkalisalzen bzw. Erdalkalimetallen, insbesondere in ionischer Form, gemäß den obigen Ausführungen sein kann, führt insbesondere dazu, dass eine diesbezügliche Wechselwirkung mit den Komponenten (a) bzw. (b) bzw. (c), insbesondere im Hinblick auf eine Vermeidung unerwünschter Komplexbildungen oder dergleichen, verringert bzw. verhindert wird. Folglich kann auf dieser Basis einer Ausfällung von Wirkkomponenten bzw. Pufferkomponenten entgegengewirkt werden. Insbesondere kann auf dieser Basis auch eine unerwünschte Veränderung der Viskosität insbesondere infolge einer unkontrollierten Komplexbildung oder dergleichen vermieden werden.

Gemäß einer erfindungsgemäßen Ausführungsform kann die Zusammensetzung, insbesondere anwendungs-, dosier- und/oder applikationsfertig, mit einem Volumen von (50 ± 10) ml, insbesondere (50 ± 5) ml, vorzugsweise (50 ± 2) ml, bevorzugt (50±1)ml, besonders bevorzugt (50 ± 0,5) ml, ganz besonders bevorzugt etwa 50 ml, vorliegen.

In diesem Zusammenhang kann die Zusammensetzung, insbesondere anwendungs-, dosier- und/oder applikationsfertig, zur Verabreichung, insbesondere Instillation in die Harnblase, eines Volumens der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegen bzw. hergerichtet sein. Insbesondere kann die Zusammensetzung, insbesondere anwendungs-, dosier- und/oder applikationsfertig, insbesondere mittels Instillation in die Harnblase, mit einem Volumen der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, verabreicht werden.

Gleichermaßen kann die Zusammensetzung, insbesondere anwendungs-, dosier- bzw. applikationsfertig, mit einer Wirkstoffmenge an Chondroitinsulfat und/oder physiologisch verträglichem Chondroitinsulfat-Salz (Komponente (a)) von (225 ± 25) mg, insbesondere (225 ± 15) mg, vorzugsweise (225 ± 10) mg, bevorzugt etwa 225 mg, vorliegen.

Die Zusammensetzung nach der Erfindung kann zudem, insbesondere anwendungs-, dosier- und/oder applikationsfertig, zur Verabreichung einer Wirkstoffmenge an Chondroitinsulfat bzw. physiologisch verträglichem Chondroitinsulfat-Salz (Komponente (a)) von (225 ± 25) mg, insbesondere (225 ± 15) mg, vorzugsweise (225 ± 10) mg, bevorzugt etwa 225 mg, vorliegen bzw. hergerichtet sein.

In diesem Zusammenhang kann die Zusammensetzung, insbesondere anwendungs-, dosier- und/oder applikationsfertig, mit einer Wirkstoffmenge an Chondroitinsulfat und/oder physiologisch verträglichem Chondroitinsulfat-Salz (Komponente (a)) von (225 ± 25) mg, insbesondere (225 ± 15) mg, vorzugsweise (225 ± 10) mg, bevorzugt etwa 225 mg, verabreicht werden.

Die erfindungsgemäße Zusammensetzung kann in diesem Zusammenhang zudem insbesondere anwendungs-, dosier- bzw. applikationsfertig mit einer Wirkstoffmenge an Hyaluronsäure bzw. physiologisch verträglichem Hyaluronsäure-Salz (Hyaluronat) (Komponente (b)) von (800 ± 80) mg, insbesondere (800 ± 60) mg, vorzugsweise (800 ± 40) mg, bevorzugt etwa 800 mg, vorliegen.

Erfindungsgemäß kann die Zusammensetzung insbesondere anwendungs-, dosier- bzw. applikationsfertig zur Verabreichung einer Wirkstoffmenge an Hyaluronsäure und/oder physiologisch verträglichem Hyaluronsäure-Salz (Hyaluronat) (Komponente (b)) von (800 ± 80) mg, insbesondere (800 ± 60) mg, vorzugsweise (800 ± 40) mg, bevorzugt etwa 800 mg, vorliegen bzw. hergerichtet sein. Insbesondere kann die Zusammensetzung, insbesondere anwendungs-, dosier- und/oder applikationsfertig, mit einer Wirkstoffmenge an Hyaluronsäure bzw. physiologisch verträglichem Hyaluronsäure-Salz (Hyaluronat) (Komponente (b)) von (800 ± 80) mg, insbesondere (800 ± 60) mg, vorzugsweise (800 ± 40) mg, bevorzugt etwa 800 mg, verabreicht werden.

Erfindungsgemäß wird somit in zielgerichteter Weise insbesondere auf eine speziell dosierte Zusammensetzung im Hinblick auf die Wirkkomponenten (a) bzw. (b) abgestellt, wonach Komponente (a) in zweckgerichteter Weise in verhältnismäßig geringen Mengen bzw. Dosen vorliegt, während demgegenüber für Komponente (b) verhältnismäßig hohe Mengen bzw. Dosen vorgesehen sind, wobei die Zusammensetzung zudem vorzugsweise anwendungsfertig mit einem definierten Volumen, wie zuvor definiert, vorliegt. Denn die Anmelderin hat in diesem Zusammenhang in völlig überraschender Weise gefunden, dass die entsprechenden und aufeinander abgestimmten Wirkstoffmengen bei gleichzeitig hoher Stabilität der erfindungsgemäßen Zusammensetzung zu einer besonders guten Wirksamkeit hinsichtlich der zugrundeliegenden Erkrankungen des Urogenitaltraktes, insbesondere Cystitis, vorzugsweise interstitieller Cystitis, führen, so dass diesbezüglich ein Stabilitäts- bzw. Wirkoptimum vorliegt. Diesbezüglich kommt auch dem speziellen Volumen, wie zuvor definiert, insofern eine hohe Bedeutung zu, als auf dieser Basis eine optimale Anpassung der Verabreichungsgrößen der zu applizierenden bzw. zu instillierenden Zusammensetzung an die pathologische Situation insbesondere bei Cystitis, vorzugsweise interstitieller Cystitis, vorliegt, wonach nämlich oftmals eine verringerte Blasenkapazität bzw. eine sogenannte Schrumpfblase vorliegen kann. Durch Verabreichung bzw. Instillation des zugrundeliegenden speziellen Volumens kann die Haltezeit bzw. -dauer und somit das Verbleiben der Zusammensetzung in der Harnblase erhöht werden, was die Wirksamkeit der erfindungsgemäßen Zusammensetzung weiterführend verbessert.

Im Rahmen der vorliegenden Erfindung kann es dabei vorgesehen sein, dass die Zusammensetzung in einer Aufbewahrungs- bzw. Applikationsvorrichtung, insbesondere in ein Aufbewahrungs- bzw. Applikationsbehältnis, eingebracht ist bzw. vorliegt, und zwar insbesondere anwendungs-, dosier- bzw. applikationsfertig.

In diesem Zusammenhang kann die Zusammensetzung somit insbesondere anwendungs-, dosier- bzw. applikationsfertig in einer Aufbewahrungs- bzw. Applikationsvorrichtung, insbesondere in einem Aufbewahrungs- und/oder Applikationsbehältnis, eingebracht vorliegen, bevorzugt in Volumengrößen und/oder mit einem Volumen der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, je Behältnis bzw. Applikationseinheit (Dosiereinheit).

Insbesondere kann in diesem Zusammenhang die erfindungsgemäße Aufbewahrungs- bzw. Applikationsvorrichtung eine vorzugsweise sterile Spritze, insbesondere Applikationsspritze, vorzugsweise Einweg-Applikationsspritze, sein, insbesondere mit einem Aufnahme- bzw. Füllvolumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml. Demgegenüber kann die Aufbewahrungs- bzw. Applikationsvorrichtung auch in Form einer Durchstechflasche, vorzugsweise mit sterilem Verschluss, oder dergleichen vorliegen bzw. ausgebildet sein.

Gemäß dem vorliegenden Aspekt betrifft die vorliegende Erfindung auch die erfindungsgemäße Zusammensetzung zur Instillation bzw. zur vorzugsweise topischen Applikation in den Urogenitalbereich, insbesondere in die Harnblase.

Insbesondere kann die Zusammensetzung nach der Erfindung zur Verabreichung bzw. zur Instillation bzw. zur vorzugsweise topischen Applikation in den Urogenitaltrakt, insbesondere in die Harnblase, hergerichtet sein. Gleichermaßen kann die Zusammensetzung nach der Erfindung in diesem Zusammenhang mittels Instillation bzw. zur vorzugsweise topischen Applikation in den Urogenitaltrakt, insbesondere in die Harnblase, verabreicht werden.

Gleichermaßen betrifft die vorliegende Erfindung auch die erfindungsgemäße Zusammensetzung zur Verwendung bei der prophylaktischen bzw. therapeutischen Behandlung von vorzugsweise entzündlichen Erkrankungen des Urogenitaltraktes, insbesondere von entzündlichen Erkrankungen der Harnblase, bevorzugt von Cystitis, insbesondere akuter oder chronischer Cystitis, bevorzugt interstitieller Cystitis, radiogener Cystitis, chronisch rezidivierender Cystitis, Chemocystitis, chronisch abakterieller Cystitis und chronisch bakterieller Cystitis, besonders bevorzugt interstitieller Cystitis.

Gleichermaßen betrifft die vorliegende Erfindung auch die erfindungsgemäße Zusammensetzung zur prophylaktischen bzw. therapeutischen Behandlung von vorzugsweise entzündlichen Erkrankungen des Urogenitaltraktes, insbesondere von entzündlichen Erkrankungen der Harnblase, bevorzugt von Cystitis, insbesondere akuter oder chronischer Cystitis, bevorzugt interstitieller Cystitis, radiogener Cystitis, chronisch rezidivierender Cystitis, Chemocystitis, chronisch abakterieller Cystitis und chronisch bakterieller Cystitis, besonders bevorzugt interstitieller Cystitis.

Die erfindungsgemäße Zusammensetzung ist somit für die Verwendung bei der prophylaktischen bzw. therapeutischen Behandlung von vorzugsweise entzündlichen Erkrankungen des Urogenitaltraktes geeignet, insbesondere von entzündlichen Erkrankungen der Harnblase, bevorzugt von Cystitis, insbesondere akuter oder chronischer Cystitis, bevorzugt interstitieller Cystitis, radiogener Cystitis, chronisch rezidivierender Cystitis, Chemocystitis, chronisch abakterieller Cystitis und chronisch bakterieller Cystitis, besonders bevorzugt interstitieller Cystitis.

Auch ist die erfindungsgemäße Zusammensetzung, wie zuvor definiert, zur Herstellung eines Arzneimittels bzw. Medikaments zur prophylaktischen bzw. therapeutischen Behandlung von vorzugsweise entzündlichen Erkrankungen des Urogenitaltraktes geeignet, insbesondere von entzündlichen Erkrankungen der Harnblase, bevorzugt von Cystitis, insbesondere akuter oder chronischer Cystitis, bevorzugt interstitieller Cystitis, radiogener Cystitis, chronisch rezidivierender Cystitis, Chemocystitis, chronisch abakterieller Cystitis und chronisch bakterieller Cystitis, besonders bevorzugt interstitieller Cystitis.

Die erfindungsgemäße Zusammensetzung bzw. Kombination, welche Chondroitinsulfat bzw. ein physiologisch verträgliches Chondroitinsulfat-Salz (Komponente (a)) einerseits und Hyaluronsäure bzw. ein physiologisch verträgliches Hyaluronsäure-Salz (Komponente (b)) andererseits in jeweils spezieller Konzentration und darüber hinaus ein spezielles Phosphatpuffersystem (Komponente (c)) enthält, führt über die überraschend gefundene hohe Stabilität, insbesondere Lagerstabilität, hinausgehend auch zu einer besonders guten Wirksamkeit hinsichtlich der vorgenannten Erkrankungen. Eine mögliche Erklärung für die hervorragende Wirkung der erfindungsgemäßen Zusammensetzung kann - ohne sich auf diese Theorie beschränken oder festlegen zu wollen - darin gesehen werden, dass die Wirksubstanzen der erfindungsgemäßen Zusammensetzung in besonders effektiver Weise insbesondere mit dem Urothel der Harnblase wechselwirken, wobei diesbezüglich eine An- bzw. Einlagerung der Wirksubstanzen an diese Schicht vorliegt, was zu einer Reparatur der durch die Erkrankung geschädigten Glykosaminoglykanschicht bzw. zu einer diesbezüglichen Regeneration führt. Die mit dieser Wirkweise verbundene Verringerung der Permeabilität des Urothels - was gewissermaßen einem "Abdichte"-Effekt gegenüber dem in der Blase vorhandenen Harn bzw. Urin gleichkommt - führt zu einer deutlichen Linderung der krankheitsbedingten Symptome, insbesondere auch im Hinblick auf die zugrundeliegende Schmerzsymptomatik. So führt die Verwendung der erfindungsgemäßen Zusammensetzung bereits nach wenigen Behandlungen bei von den vorgenannten Erkrankungen betroffenen Patienten zu einem signifikant verbesserten Gesundheitsstatus. Die erfindungsgemäße Zusammensetzung kann somit gewissermaßen zumindest auch zum vorübergehenden Ersatz einer defekten Glykosaminoglykanschicht des Urothels dienen.

In diesem Zusammenhang kann die Wirkweise der Zusammensetzung nach der Erfindung - ohne sich hierauf festlegen zu wollen - insbesondere in einer maßgeblich physikalischen Wechselwirkung gesehen werden, bei welcher die Wirkstoffe gemäß Komponente (a) bzw. (b) in das Urothel eingebaut bzw. eingelagert werden bzw. sich hieran anlagern, so dass ein durch insbesondere entzündliche Reaktionen hervorgerufener Verlust von Chondroitinsulfat bzw. Hyaluronsäure in der Blasenwand bzw. im Urothel ausgeglichen bzw. kompensiert wird. Es liegt somit gewissermaßen eine Regulation der Permeabilität bzw. Durchlässigkeit der Blasenwand vor, was zu einer Eindämmung der Entzündungsreaktion und somit zu einer Unterstützung der Wundheilung und damit insgesamt zu einer Verbesserung des Gesundheitszustands führt. Die erfindungsgemäße Zusammensetzung bildet quasi einen Schutz des Blasenepithels vor irritierenden Substanzen, wie Bakterien, Mikrokristallen oder dergleichen, wobei die Zusammensetzung nach der Erfindung als Ersatz und Schutz der Glykosaminoglykanschicht in der Harnblase und den ableitenden Harnwegen fungiert.

Im Rahmen ihrer Verwendung kann die erfindungsgemäße Zusammensetzung im Urogenitalbereich, insbesondere in die Harnblase, instilliert bzw. topisch appliziert werden. Dabei sollte die Instillation bzw. die Applikation in die vorzugsweise zuvor entleerte Harnblase erfolgen. Diesbezüglich kann die Zusammensetzung nach der Erfindung in der Harnblase beispielsweise für eine Zeitdauer von mehreren Minuten bis zu einigen Stunden verbleiben, um eine optimale Einwirkung der Wirkstoffe auf das Urothel zu ermöglichen. Durch die erfindungsgemäß speziell konzentrierte Zusammensetzung wird dabei eine besonders effiziente Wechselwirkung der in den jeweilig speziellen Mengen bzw. Dosen vorliegenden Wirkstoffe in Form der Komponenten (a) bzw. (b) mit dem Urothel ermöglicht, einhergehend mit einer entsprechend hohen An- bzw. Einlagerung der in Rede stehenden Wirkstoffe an bzw. in die zugrundeliegende Schicht der Harnblase.

In diesem Zusammenhang kann insbesondere derart vorgegangen werden, dass das zugrundeliegende Volumen der Zusammensetzung, welche sich insbesondere in der nachfolgend noch beschriebenen Aufbewahrungs- bzw. Applikationsvorrichtung befindet, nach vollständiger Entleerung der Harnblase in die Harnblase instilliert wird, wobei das zugrundeliegende Volumen insbesondere etwa 50 ml betragen sollte. Um optimale Ergebnisse zu erreichen, sollte die Zusammensetzung nach der Erfindung möglichst lange in der Harnblase verbleiben, und zwar - wie zuvor beschrieben - für einen Zeitraum von mehreren Minuten bis mehreren Stunden.

Beispielsweise kann im Rahmen der Behandlung von Cystitis derart vorgegangen werden, die Instillation der erfindungsgemäßen Zusammensetzung für einen Zeitraum von vier Wochen jeweils einmal pro Woche durchzuführen, wobei die diesbezügliche Einzeldosis an Chondroitinsulfat bzw. Komponente (a) etwa 225 mg und die Einzeldosis an Hyaluronsäure bzw. Komponente (b) etwa 800 mg in einem Volumen der Zusammensetzung von etwa 50 ml betragen sollte. Im Rahmen einer Erhaltungstherapie kann die erfindungsgemäße Zusammensetzung anschließend beispielsweise einmal pro Monat angewendet werden, insbesondere bis die Symptome vollständig abgeklungen sind. Von dem vorgenannten beispielhaften Therapieschema kann jedoch auch abgewichen werden, sofern einzelfallbezogen erforderlich.

Ein weiterer Vorteil der Verwendungen der Zusammensetzung nach der Erfindung ist zudem darin zu sehen, dass diese hinsichtlich ihrer Anwendung sicher ist und während der Behandlung keine nennenswerten Nebenwirkungen auftreten, insbesondere da es sich bei den eingesetzten Substanzen um biokompatible Stoffe handelt. Die besonders gute Verträglichkeit steht insbesondere mit der Verwendung von Chondroitinsulfat marinen Ursprungs bzw. Hyaluronsäure nichttierischen Ursprungs in Zusammenhang.

Durch die zweckgerichtete Verwendung der vorgenannten speziellen Dosen bzw. Mengen an Wirksubstanzen auf Basis der Wirkkomponenten (a) und (b) wird zudem eine hohe Wirksamkeit der erfindungsgemäßen Zusammensetzung erreicht, welche zudem durch das spezielle Phosphatpuffersystem nicht nachteilig beeinträchtigt wird. Vielmehr führt der gezielte Einsatz des in Rede stehenden Puffersystems gemäß Komponente (c) in Kombination mit den weiteren erfindungsgemäßen Maßnahmen zu einer effektiven Stabilisierung der Zusammensetzung, was zu einer hohen Anwendungssicherheit und Aufrechterhaltung der Wirksamkeit bzw. Wirkeffizienz führt.

Zusammenfassend handelt es sich bei der erfindungsgemäßen Zusammensetzung vorzugsweise um ein Medizinprodukt, das gemäß einer erfindungsgemäß bevorzugten Ausführungsform eine speziell dosierte Lösung bzw. Dispersion der Wirkstoffe auf Basis der Komponenten (a) und (b) enthält.

Im Allgemeinen kann die Zusammensetzung mindestens einmal pro Woche, vorzugsweise über einen Zeitraum von vorzugsweise mindestens einem Monat, mittels Instillation bzw. zur topischer Applikation in die Harnblase verabreicht werden.

Was die erfindungsgemäße Zusammensetzung als solche bzw. deren Verwendungen weiterhin anbelangt, so kann die Zusammensetzung insbesondere mit einem Volumen der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, und/oder einer Wirkstoffmenge an Chondroitinsulfat und/oder physiologisch verträglichem Chondroitinsulfat-Salz (Komponente (a)) von (225 ± 25) mg, insbesondere (225 ± 15) mg, vorzugsweise (225 ± 10) mg, bevorzugt etwa 225 mg, bzw. einer Wirkstoffmenge an Hyaluronsäure bzw. physiologisch verträglichem Hyaluronsäure-Salz (Hyaluronat) (Komponente (b)) von (800 ± 80) mg, insbesondere (800 ± 60) mg, vorzugsweise (800 ± 40) mg, bevorzugt etwa 800 mg, insbesondere mindestens einmal pro Woche, vorzugsweise über einen Zeitraum von vorzugsweise mindestens einem Monat, in die Harnblase verabreicht bzw. instilliert bzw. vorzugsweise topisch appliziert werden bzw. zur mindestens wöchentlichen Verabreichung (mindestens einmal pro Woche), vorzugsweise über einen Zeitraum von vorzugsweise mindestens einem Monat, mittels Instillation bzw. topischer Applikation in die Harnblase hergerichtet sein.

Die erfindungsgemäße Zusammensetzung zeichnet sich auch in diesem Zusammenhang über eine hervorragende Lagerstabilität und über eine hohe Wirkeffizienz mit diesbezüglich optimierten Wirkstoffmengen, wie zuvor definiert, aus.

Gemäß einem wiederum **weiteren** Aspekt der vorliegenden Erfindung betrifft die vorliegende Erfindung gleichermaßen eine Aufbewahrungs- bzw. Applikationsvorrichtung, insbesondere ein Aufbewahrungs- bzw. Applikationsbehältnis, insbesondere in Form einer vorzugsweise sterilen Spritze, insbesondere Applikationsspritze, vorzugsweise Einweg-Applikationsspritze, vorzugsweise zur Instillation bzw. zur vorzugsweise topischen Applikation in den Urogenitalbereich, insbesondere in die Harnblase, enthaltend eine Zusammensetzung nach der Erfindung, wie zuvor definiert.

In diesem Zusammenhang betrifft die vorliegende Erfindung gemäß diesem Aspekt auch eine Aufbewahrungs- bzw. Applikationsvorrichtung, insbesondere ein Aufbewahrungs- bzw. Applikationsbehältnis, insbesondere in Form einer vorzugsweise sterilen Spritze, insbesondere Applikationsspritze, vorzugsweise Einweg-Applikationsspritze, vorzugsweise zur Instillation bzw. zur vorzugsweise topischen Applikation in den Urogenitalbereich, insbesondere in die Harnblase, enthaltend eine Zusammensetzung, wie zuvor definiert,
wobei die Zusammensetzung in Kombination und jeweils in wirksamen, insbesondere pharmazeutisch wirksamen Mengen
(a) Chondroitinsulfat und/oder ein physiologisch verträgliches Chondroitinsulfat-Salz in einer Wirkstoffmenge von (225 ± 25) mg (Komponente (a));
(b) Hyaluronsäure und/oder ein physiologisch verträgliches Hyaluronsäure-Salz (Hyaluronat) in einer Wirkstoffmenge von (800 ± 80) mg (Komponente (b));
(c) ein Dihydrogenphosphat/Monohydrogenphosphat-Puffersystem (Komponente (c));
(d) gegebenenfalls mindestens einen physiologisch verträglichen Elektrolyten (Komponente (d));
enthält,
wobei die Zusammensetzung einen pH-Wert im Bereich von 6,1 bis 7,9 aufweist und/oder wobei die Zusammensetzung auf einen pH-Wert im Bereich von 6,1 bis 7,9 eingestellt ist.

Erfindungsgemäß ist es dabei bevorzugt, wenn die Aufbewahrungs- bzw. Applikationsvorrichtung ein Aufnahme- bzw. Füllvolumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, aufweist.

Insbesondere kann die Zusammensetzung eine Wirkstoffmenge an (a) Chondroitinsulfat bzw. physiologisch verträglichem Chondroitinsulfat-Salz (Komponente (a)) von (225 ± 15) mg, vorzugsweise (225 ± 10) mg, bevorzugt etwa 225 mg, aufweisen.

Zudem kann die Zusammensetzung eine Wirkstoffmenge an (b) Hyaluronsäure bzw. physiologisch verträglichem Hyaluronsäure-Salz (Hyaluronat) (Komponente (b)) von (800 ± 60) mg, vorzugsweise (800 ± 40) mg, bevorzugt etwa 800 mg, aufweisen.

Erfindungsgemäß kann es zudem vorgesehen sein, dass die Zusammensetzung das Dihydrogenphosphat/Monohydrogenphosphat-Puffersystem (Komponente (c)) in einer Gesamtmenge an Dihydrogenphosphat/Monohydrogenphosphat-Puffersystem (Komponente (c)) von (87,5 ± 85) mg, insbesondere in einer Gesamtmenge von (87,5 ± 75) mg, vorzugsweise in einer Gesamtmenge von (87,5 ± 62,5) mg, bevorzugt in einer Gesamtmenge von (87,5 ± 50) mg, besonders bevorzugt in einer Gesamtmenge von (87,5 ± 45) mg, ganz besonders bevorzugt in einer Gesamtmenge von (87,5 ± 40) mg, noch weiter bevorzugt in einer Gesamtmenge von etwa 87,5 mg, aufweist.

Insbesondere kann die Zusammensetzung den Elektrolyten (Komponente (d)) in einer Menge von (400 ± 300) mg, insbesondere in einer Menge von (400 ± 200) mg, vorzugsweise in einer Menge von (400 ± 100) mg, bevorzugt in einer Menge von (400 ± 50) mg, besonders bevorzugt in einer Menge von etwa 400 mg, enthalten.

Erfindungsgemäß kann es zudem vorgesehen sein, dass das Chondroitinsulfat bzw. das physiologisch verträgliche Chondroitinsulfat-Salz (Komponente (a)) in Form eines Alkalisalzes, vorzugsweise in Form eines Natrium-Salzes, vorliegt.

Gleichermaßen kann es erfindungsgemäß vorgesehen sein, dass das Chondroitinsulfat bzw. das physiologisch verträgliche Chondroitinsulfat-Salz (Komponente (a)) in Form von Chondroitinsulfat-Natrium (Natrium-Chondroitinsulfat) vorliegt.

Zudem kann die Hyaluronsäure bzw. das physiologisch verträgliche Hyaluronsäure-Salz (Komponente (b)) in Form eines Alkalisalzes, vorzugsweise in Form eines Natrium-Salzes, und/oder in Form eines Alkalihyaluronates vorliegen.

Insbesondere kann die Hyaluronsäure bzw. das physiologisch verträgliche Hyaluronsäure-Salz (Komponente (b)) in Form von Natriumhyaluronat vorliegen.

Darüber hinaus kann das Dihydrogenphosphat/Monohydrogenphosphat-Puffersystem (Komponente (c)) als Alkalidihydrogenphosphat/Alkalimonohydrogenposphat-Puffersystem vorliegen bzw. ausgebildet sein. In diesem Zusammenhang kann das Alkalimetall ausgewählt sein aus Natrium und/oder Kalium, insbesondere Natrium.

Insbesondere kann das Dihydrogenphosphat/Monohydrogenphosphat-Puffersystem (Komponente (c)) als Natriumdihydrogenphosphat/Natriummonohydrogenposphat-Puffersystem, vorliegen bzw. ausgebildet sein.

Darüber hinaus kann der Elektrolyt (Komponente (d)) in Form eines Alkalisalzes, insbesondere in Form eines Alkalichlorids, vorzugsweise in Form von Natriumchlorid, vorliegen bzw. ausgebildet sein. Insbesondere kann der Elektrolyt (Komponente (d)) Natriumchlorid sein.

Was die Zusammensetzung weiterhin anbelangt, so kann diese einen pH-Wert im Bereich von 6,6 bis 7,7, insbesondere im Bereich von 6,9 bis 7,6, vorzugsweise im Bereich von 7,1 bis 7,4, aufweisen.

Insbesondere kann der pH-Wert der Zusammensetzung im Bereich von 6,6 bis 7,7, insbesondere im Bereich von 6,9 bis 7,6, vorzugsweise im Bereich von 7,1 bis 7,4, konstant gehalten bzw. eingestellt sein. Der pH-Wert kann vorzugsweise mittels des Alkalidihydrogenphosphat/Alkalimonohydrogenposphat-Puffersystems (Komponente (c)) eingestellt bzw. vorgegeben sein.

Insbesondere kann die Zusammensetzung bei einer Temperatur von 20 °C eine dynamische Viskosität von mindestens 2.000 mPas, insbesondere mindestens 4.000 mPas, vorzugsweise mindestens 5.000 mPas, bevorzugt mindestens 5.250 mPas, aufweisen.

Gleichermaßen kann die Zusammensetzung bei einer Temperatur von 20 °C eine dynamische Viskosität von höchstens 7.900 mPas, insbesondere höchstens 6.900 mPas, vorzugsweise höchstens 6.000 mPas, bevorzugt höchstens 5.750 mPas, aufweisen.

Zudem kann die Zusammensetzung bei einer Temperatur von 20 °C eine dynamische Viskosität im Bereich von 2.000 mPas bis 7.900 mPas, insbesondere im Bereich von 4.000 mPas bis 6.900 mPas, vorzugsweise im Bereich von 5.000 mPas bis 6.000 mPas, bevorzugt im Bereich von 5.250 mPas bis 5.750 mPas, aufweisen.

Weiterhin kann die Zusammensetzung eine Osmolalität im Bereich von 150 mosmol/kg bis 600 mosmol/kg, insbesondere im Bereich von 200 mosmol/kg bis 550 mosmol/kg, vorzugsweise im Bereich von 250 mosmol/kg bis 500 mosmol/kg, bevorzugt im Bereich von 275 mosmol/kg bis 450 mosmol/kg, besonders bevorzugt im Bereich von 300 mosmol/kg bis 400 mosmol/kg, aufweisen.

Weiterhin kann die Zusammensetzung bei einer Temperatur von 20 °C und bei einem Druck von 1.013,25 mbar (Atmosphärendruck) eine Dichte im Bereich von 1,001 g/cm³ bis 1,5 g/cm³, insbesondere im Bereich von 1,005 g/cm³ bis 1,25 g/cm³, vorzugsweise im Bereich von 1,0075 g/cm³ bis 1,1 g/cm³, bevorzugt im Bereich von 1,0075 g/cm³ bis 1,075 g/cm³, besonders bevorzugt im Bereich von 1,0075 g/cm³ bis 1,05 g/cm³, aufweisen.

Insbesondere kann die Zusammensetzung bei einer Temperatur von 20 °C und bei einem Druck von 1.013,25 mbar (Atmosphärendruck) eine relative Dichte, bezogen auf reines Wasser, im Bereich von 1,001 bis 1,5, insbesondere im Bereich von 1,005 bis 1,25, vorzugsweise im Bereich von 1,0075 bis 1,1, bevorzugt im Bereich von 1,0075 bis 1,075, besonders bevorzugt im Bereich von 1,0075 bis 1,05, aufweisen.

Erfindungsgemäß ist es bevorzugt, wenn die Aufbewahrungs- bzw. Applikationsvorrichtung die Zusammensetzung, insbesondere anwendungs-, dosier- und/oder applikationsfertig, mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, enthält.

Insbesondere kann die Zusammensetzung mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegen.

Erfindungsgemäß ist es zudem bevorzugt, wenn die Zusammensetzung als wässrige Zusammensetzung vorliegt. Insbesondere kann die Zusammensetzung insbesondere wässrig basiert sein bzw. wässrig formuliert vorliegen, insbesondere in Form einer wässrigen Lösung bzw. wässrigen Suspension, vorzugsweise in Form einer wässrigen Lösung.

Insbesondere kann die Zusammensetzung Wasser, insbesondere gereinigtes Wasser, enthalten. Insbesondere kann die Zusammensetzung Wasser als pharmazeutisch verträglichen Träger (Exzipienten) enthalten. Erfindungsgemäß kann die Zusammensetzung dabei wässrig basiert ausgebildet sein. In diesem Zusammenhang kann die Zusammensetzung einen Gehalt an Wasser von mindestens 50 Gew.-%, insbesondere mindestens 75 Gew.-%, vorzugsweise mindestens 80 Gew.-%, bevorzugt mindestens 90 Gew.-%, besonders bevorzugt mindestens 95 Gew.-%, bezogen auf die Zusammensetzung, aufweisen.

Erfindungsgemäß kann die für die Aufbewahrungs- bzw. Applikationsvorrichtung vorgesehene Zusammensetzung nach der Erfindung aus den vorgenannten Komponenten (a), (b), (c) und gegebenenfalls (d) sowie Wasser bestehen.

Für weitergehende Einzelheiten zu der erfindungsgemäßen Aufbewahrungs- bzw. Applikationsvorrichtung, welche die Zusammensetzung nach der Erfindung enthält, kann zudem auf die Ausführungen zu den übrigen Erfindungsaspekten verwiesen werden, welche in Bezug auf die erfindungsgemäße Aufbewahrungs- bzw. Applikationsvorrichtung entsprechend gelten.

Darüber hinaus betrifft die vorliegende Erfindung - gemäß einem wiederum **weiteren** Aspekt der vorliegenden Erfindung - auch die Verpackungseinheit nach der Erfindung, welche mindestens eine Aufbewahrungs- bzw. Applikationsvorrichtung, wie zuvor definiert, enthält. In diesem Zusammenhang kann die Aufbewahrungs- bzw. Applikationsvorrichtung in einer vor Kontaminationen schützenden Umverpackung eingebracht vorliegen. Für weitergehende Einzelheiten zu der erfindungsgemäßen Verpackungseinheit kann auf die Ausführungen zu den übrigen Erfindungsaspekten verwiesen werden, welche in Bezug auf die erfindungsgemäße Verpackungseinheit entsprechend gelten.

Schließlich betrifft die vorliegende Erfindung - gemäß einem **weiteren** Aspekt der vorliegenden Erfindung - auch das erfindungsgemäße Kit, insbesondere Instillationssystem, umfassend (i) mindestens eine Aufbewahrungs- bzw. Applikationsvorrichtung, wie zuvor definiert, und (ii) mindestens eine Zusammensetzung, wie zuvor definiert, insbesondere wobei die Zusammensetzung vorzugsweise anwendungs-, dosier- bzw. applikationsfertig in der Aufbewahrungs- und/oder Applikationsvorrichtung vorliegt, und (iii) mindestens eine an die Aufbewahrungs- bzw. Applikationsvorrichtung anschließbare Instillationsvorrichtung, insbesondere in Form eines Instillationsschlauches oder dergleichen.

Auf Basis des erfindungsgemäßen Kits wird insbesondere ein entsprechendes Installationssystem mit einfacher Handhabbarkeit, schneller Einsatzbereitschaft und hoher Anwendungssicherheit bereitgestellt.

Für weitergehende Einzelheiten zu dem erfindungsgemäßen Kit kann auf die vorangegangenen Ausführungen zu den übrigen Erfindungsaspekten verwiesen werden, welche in Bezug auf das erfindungsgemäße Kit entsprechend gelten.

Im Rahmen der vorliegenden Erfindung wird somit insgesamt unter Zugrundelegung der erfindungsgemäßen Zusammensetzung mit den speziell dosierten Wirkstoffkomponenten, wonach nämlich Komponente (a) in relativ kleinen Mengen bzw. Dosen vorliegt und Komponente (b) in relativ großen Mengen bzw. Dosen vorliegt, ein leistungsfähiges Gesamtkonzept zur Behandlung von vorzugsweise entzündlichen Erkrankungen des Urogenitaltraktes, wie von Cystitis, bevorzugt interstitieller Cystitis, bereitgestellt, wobei die erfindungsgemäße Zusammensetzung neben einer hohen Wirksamkeit in Bezug auf die zugrundeliegenden Erkrankungen bei gleichzeitig geringen Nebenwirkungen und hervorragender Handhabbarkeit über eine signifikant erhöhte Stabilität, insbesondere Lagerungsstabilität, verfügt.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### AUSFÜHRUNGSBEISPIELE

### 1. Herstellungsbeispiele

Zur Herstellung von jeweils 50 ml einer klaren, gegebenenfalls leicht gelblich gefärbten wässrigen Lösung erfindungsgemäßer Zusammensetzungen wird in dem Fachmann an sich bekannter Weise vorgegangen.

Zunächst wird bei Raumtemperatur und Umgebungsdruck in einem Glasgefäß mit Rühreinrichtung eine definierte Teilmenge (insbesondere etwa 75 % des gewünschten Endvolumens) an gereinigtem Wasser vorgelegt. Unter Rühren werden zunächst ein Elektrolyt in Form von Natriumchlorid und nachfolgend die Komponenten des Puffersystems in Form von Natriumdihydrogenphosphat und Dinatriumhydrogenphosphat hinzugegeben. Nach vollständiger Auflösung der Komponenten wird der pH-Wert bestimmt und auf den gewünschten Wert (z. B. pH-Wert von ca. 7), gegebenenfalls unter Verwendung von Phosphorsäure oder Natronlauge, eingestellt.

Anschließend werden die nachfolgenden in den Rezepturbeispielen spezifizierten Mengen und Typen der weiteren Wirk- und Inhaltsstoffe unter Rühren hinzugegeben, und zwar Komponente (a) in Form von Chondroitinsulfat-Salz (Chondroitinsulfat-Natrium) und Komponente (b) in Form von Natriumhyaluronat. Die Lösung wird mit weiterem Wasser auf das gewünschte Endvolumen von 50 ml aufgefüllt.

Anschließend wird der pH-Wert der erhaltenen Zusammensetzung nochmals bestimmt und gegebenenfalls nachreguliert. Auch die übrigen entsprechenden Spezifikationen der Lösungen werden auf die eingestellten bzw. vorgewählten Wertebereiche hin überprüft (z. B. Osmolalität; relative Dichte; mikrobiologische Reinheit und Sterilität; Ausschluss von Verunreinigungen, insbesondere Abbauprodukten der Inhalts- und Wirkstoffe; Viskosität; Aussehen).

Die so erhaltene Lösung kann anschließend einer Sterilfiltration unterzogen werden, beispielsweise mit Hilfe von Stickstoff über eine Filterkerze mit speziellem Filtersystem, und nachfolgend in ein insbesondere steriles Aufnahme- bzw. Applikationsbehältnis, wie einer Kunststoffspritze oder dergleichen, abgefüllt werden. Die erhaltene Zusammensetzung kann für weiterführende Stabilitätsuntersuchungen oder für entsprechende Anwendungs- bzw. Wirksamkeitsuntersuchungen verwendet werden.

Nach dieser allgemeinen Herstellvorschrift werden die nachfolgend spezifizierten Rezepturen bzw. Zusammensetzungen durch Auflösen in Wasser der nachfolgenden Komponenten mit den diesbezüglichen Mengenangaben (Einwaage bei Herstellung der Zusammensetzung) hergestellt:

**Zusammensetzung A1 (Angabe pro 50 ml Zusammensetzung)**

| **Inhaltsstoff** | **Menge / mg** | **Qualität** |
|---|---|---|
| Chondroitinsulfat-Natrium | 225 | Ph. Eur. |
| Mₙ = 15 kDa; M_{W} = 100 kDa; M_{z} = 430 kDa | | |
| Natriumhyaluronat | 800 | Ph. Eur. |
| Mₙ = 55 kDa; M_{W} = 200 kDa; M_{z} = 700 kDa | | |
| Puffersystem: Natriumdihydrogenphosphat (NaH₂(PO₄) · 2H₂O)/ | 11 | Ph. Eur. |
| Dinatriumhydrogenphosphat (Na₂H(PO₄) · 2H₂O) | 76 | |
| Elektrolyt | 395 | Ph. Eur. |
| (Natriumchlorid) | | |
| Gereinigtes Wasser | ad 50 ml | Ph. Eur. |

Der pH-Wert der Zusammensetzung A1 beträgt zudem etwa 7,2. Weiterhin weist die vorliegende Zusammensetzung eine dynamische Viskosität von etwa 5.600 mPas auf, und die Dichte beträgt etwa 1,022 g/cm³ (20 °C und Atmosphärendruck). Die Osmolalität der vorliegenden Zusammensetzung beträgt zudem etwa 370 mosmol/kg.

### Zusammensetzungen B1 bis B4

Es werden weitere Zusammensetzungen entsprechend der Zusammensetzung A1 hergestellt, jedoch mit der Maßgabe, dass unterschiedliche Mengen an Chondroitinsulfat-Natrium eingesetzt werden:

| **Zusammensetzung** | **Menge Chondroitinsulfat-Natrium / mg** |
|---|---|
| Zusammensetzung B1 | 150 |
| Zusammensetzung B2 | 200 |
| Zusammensetzung B3 | 250 |
| Zusammensetzung B4 | 300 |

### Zusammensetzungen B5 bis B8

Es werden nochmals weitere Zusammensetzungen entsprechend der Zusammensetzung A1 hergestellt, jedoch mit der Maßgabe, dass unterschiedliche Mengen an Natriumhyaluronat eingesetzt werden:

| **Zusammensetzung** | **Menge Natriumhyaluronat / mg** |
|---|---|
| Zusammensetzung B5 | 720 |
| Zusammensetzung B6 | 680 |
| Zusammensetzung B7 | 880 |
| Zusammensetzung B8 | 940 |

### Zusammensetzungen C1 und C2

Es werden nochmals weitere Zusammensetzungen entsprechend der Zusammensetzung A1 hergestellt, jedoch mit der Maßgabe, dass das Chondroitinsulfat-Natrium (CS-Na) bei einer diesbezüglich eingesetzten Menge von jeweils 225 mg mit unterschiedlichen Molmassen eingesetzt wird:

| **Zusammensetzung** | **CS-Na** | **CS-Na** | **CS-Na** |
|---|---|---|---|
| | **Mₙ** | **M_{W}** | **M_{z}** |
| Zusammensetzung C1 | 1 kDa | 5 kDa | 50 kDa |
| Zusammensetzung C2 | 35 kDa | 250 kDa | 1.100 kDa |

### Zusammensetzungen D1 und D2

Wiederum weitere Zusammensetzungen entsprechen der Zusammensetzung A1, jedoch mit der Maßgabe, dass das von Natriumhyluronat (HA-Na) bei einer diesbezüglich eingesetzten Menge von jeweils 800 mg mit unterschiedlichen Molmassen eingesetzt wird:

| **Zusammensetzung** | **HA-Na** | **HA-Na** | **HA-Na** |
|---|---|---|---|
| | **Mₙ** | **M_{W}** | **M_{z}** |
| Zusammensetzung D1 | 2 kDa | 4 kDa | 275 kDa |
| Zusammensetzung D2 | 275 kDa | 325 kDa | 1.750 kDa |

### Zusammensetzungen E1 bis E4

Bei wiederum weiteren Zusammensetzungen auf Basis der Zusammensetzung A1 wird der pH-Wert wie folgt dargestellt variiert:

| **Zusammensetzung** | **pH-Wert** |
|---|---|
| Zusammensetzung E1 | 5,5 |
| Zusammensetzung E2 | 6,1 |
| Zusammensetzung E3 | 7,9 |
| Zusammensetzung E4 | 8,5 |

### Zusammensetzungen F1 bis F4

Bei wiederum weiteren Zusammensetzungen wird das Puffersystem variiert; hierzu werden zu dem in Zusammensetzung A1 eingesetztem Phosphatpuffersystem korrelierende Mengen von Puffersystemen auf Basis von Kohlensäure/Bicarbonat, Essigsäure/Acetat, Kohlensäure/Silikat und Citronensäure/Citrat eingesetzt; die Zusammensetzungen F1 bis F4 entsprechen somit Zusammensetzung A1 mit der Maßgabe, dass diesbezüglich ein andersartiges Puffersystem eingesetzt wird (Vergleich):

| **Zusammensetzung** | **Puffersystem** |
|---|---|
| Zusammensetzung F1 | Kohlensäure/Bicarbonat |
| Zusammensetzung F2 | Essigsäure/Acetat |
| Zusammensetzung F3 | Kohlensäure/Silikat |
| Zusammensetzung F4 | Citronensäure/Citrat |

### 2.Stabilitätsuntersuchungen

An jeweiligen Chargen der Zusammensetzung gemäß den zuvor beschriebenen Rezeptururen werden Stabilitätsuntersuchungen durchgeführt.

Zu diesem Zweck wird zu entsprechenden Lagerungszeiten bzw. -zeitpunkten zum einen der Gesamtgehalt an Abbauprodukten der in den angeführten Zusammensetzungen eingesetzten Komponenten, bestimmt, und zwar jeweils zu Beginn der Untersuchungen und bei entsprechenden Lagerzeiten von 3 Monaten, 6 Monaten, 12 Monaten, 18 Monaten, 24 Monaten, 36 Monaten und 39 Monaten. Zum anderen erfolgt über denselben Lagerzeitraum zu den vorgenannten Lagerzeiten auch eine Bestimmung des pH-Wertes sowie der Viskosität.

Die Lagerung erfolgt dabei für eine erste Charge der Zusammensetzungen bei einer Temperatur von (25 ± 2) °C und bei einer relativen Luftfeuchte der Umgebung von (60 ± 5) % r.H. (nachfolgende Tabellen 1A bis 1F).

Zudem wird für eine weitere Charge der Zusammensetzungen eine Lagerung bei einer Temperatur von (40 ± 2) °C und bei einer relativen Luftfeuchte der Umgebung von (75 ± 5) % r.H. durchgeführt (nachfolgende Tabellen 2A bis 2F).

Die nachfolgenden Tabellen 1A bis 1F und 2A bis 2F zeigen die diesbezüglich ermittelten Ergebnisse. In den nachfolgenden Tabellen bedeutet "++" einen Gesamtgehalt an Abbauprodukten ("Abbau") zum jeweiligen Lagerungszeitpunkt von höchstens 3 %, bezogen auf die Gesamtkonzentration der Komponenten, bzw. eine Veränderung des pH-Wertes ("pH") bzw. der Viskosität ("Visko") zum jeweiligen Lagerungszeitpunkt von höchstens 3 %, bezogen auf den jeweiligen Ausgangswert. Weiterhin bedeutet "+" einen Gesamtgehalt an Abbauprodukten zum jeweiligen Lagerungszeitpunkt von höchstens 5 %, bezogen auf die Gesamtkonzentration der Komponenten, bzw. eine Veränderung des pH-Wertes bzw. der Viskosität zum jeweiligen Lagerungszeitpunkt von höchstens 5 %, bezogen auf den jeweiligen Ausgangswert. Schließlich bedeutet "-" einen Gesamtgehalt an Abbauprodukten zum jeweiligen Lagerungszeitpunkt von mehr als 5 %, bezogen auf die Gesamtkonzentration der Komponenten, bzw. eine Veränderung des pH-Wertes bzw. der Viskosität zum jeweiligen Lagerungszeitpunkt von mehr als 5 %, bezogen auf den jeweiligen Ausgangswert.

**Tabelle 1A [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| A1 | Abbau | ++ | ++ | ++ | ++ | ++ | ++ | ++ |
| | pH | ++ | ++ | ++ | ++ | ++ | ++ | ++ |
| | Visko | ++ | ++ | ++ | ++ | ++ | ++ | ++ |

**Tabelle 1B [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| B1 | Abbau | ++ | ++ | ++ | + | + | + | - |
| | pH | ++ | ++ | ++ | + | + | + | + |
| | Visko | ++ | ++ | ++ | ++ | + | + | + |
| B2 | Abbau | ++ | ++ | ++ | ++ | + | + | + |
| | pH | ++ | ++ | ++ | ++ | ++ | ++ | + |
| | Visko | ++ | ++ | ++ | ++ | ++ | + | + |
| B3 | Abbau | ++ | ++ | ++ | ++ | + | + | + |
| | pH | ++ | ++ | ++ | ++ | ++ | + | + |
| | Visko | ++ | ++ | ++ | + | + | + | + |
| B4 | Abbau | ++ | ++ | ++ | + | + | + | - |
| | pH | ++ | ++ | ++ | + | + | + | + |
| | Visko | ++ | ++ | ++ | + | + | + | - |
| B5 | Abbau | ++ | ++ | ++ | + | + | + | - |
| | pH | ++ | ++ | ++ | + | + | + | + |
| | Visko | ++ | ++ | ++ | ++ | + | + | + |
| B6 | Abbau | ++ | ++ | ++ | ++ | + | + | + |
| | pH | ++ | ++ | ++ | ++ | + | + | + |
| | Visko | ++ | ++ | ++ | ++ | ++ | + | + |
| B7 | Abbau | ++ | ++ | ++ | ++ | + | + | + |
| | pH | ++ | ++ | ++ | ++ | ++ | ++ | + |
| | Visko | ++ | ++ | ++ | + | + | + | + |
| B8 | Abbau | ++ | ++ | ++ | + | + | + | - |
| | pH | ++ | ++ | + | + | + | + | + |
| | Visko | ++ | ++ | ++ | + | + | + | + |

**Tabelle 1C [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| C1 | Abbau | ++ | ++ | + | + | + | + | + |
| | pH | ++ | ++ | ++ | + | + | + | + |
| | Visko | ++ | ++ | + | + | + | + | + |
| C2 | Abbau | ++ | ++ | + | + | + | + | + |
| | pH | ++ | ++ | ++ | + | + | + | + |
| | Visko | ++ | ++ | ++ | ++ | + | + | + |

**Tabelle 1D [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| D1 | Abbau | ++ | ++ | + | + | + | + | + |
| | pH | ++ | ++ | ++ | + | + | + | + |
| | Visko | ++ | ++ | + | + | + | + | - |
| D2 | Abbau | ++ | ++ | + | + | + | + | + |
| | pH | ++ | ++ | ++ | + | + | + | + |
| | Visko | ++ | ++ | ++ | + | + | + | + |

**Tabelle 1E [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| E1 | Abbau | + | + | + | + | - | - | - |
| | pH | ++ | + | + | + | + | + | - |
| | Visko | ++ | ++ | + | + | + | - | - |
| E2 | Abbau | ++ | ++ | ++ | ++ | ++ | + | + |
| | pH | ++ | ++ | ++ | ++ | ++ | + | + |
| | Visko | ++ | ++ | ++ | ++ | ++ | + | + |
| E3 | Abbau | ++ | ++ | ++ | ++ | + | + | + |
| | pH | ++ | ++ | ++ | ++ | ++ | + | + |
| | Visko | ++ | ++ | ++ | + | + | + | + |
| E4 | Abbau | ++ | + | + | + | + | - | - |
| | pH | ++ | + | + | + | + | + | - |
| | Visko | ++ | + | + | + | + | + | - |

**Tabelle 1F [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| F1 | Abbau | + | + | + | - | - | - | - |
| | pH | ++ | + | + | + | + | - | - |
| | Visko | ++ | + | + | - | - | - | - |
| F2 | Abbau | ++ | + | + | - | - | - | - |
| | pH | ++ | ++ | + | + | + | - | - |
| | Visko | ++ | + | + | + | + | - | - |
| F3 | Abbau | ++ | + | + | + | - | - | - |
| | pH | + | + | + | + | - | - | - |
| | Visko | ++ | ++ | + | + | + | - | - |
| F4 | Abbau | ++ | ++ | + | + | + | - | - |
| | pH | ++ | ++ | + | + | + | - | - |
| | Visko | ++ | ++ | + | + | + | - | - |

**Tabelle 2A [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| A1 | Abbau | ++ | ++ | ++ | ++ | ++ | ++ | + |
| | pH | ++ | ++ | ++ | ++ | ++ | + | + |
| | Visko | ++ | ++ | ++ | ++ | + | + | + |

**Tabelle 2B [(40 ± 2 °C und (75 ± 5 % r.H.]:**

| | | **3 M** | **6 M** | **12 M** | **18 M** | **24 M** | **36 M** | **39 M** |
|---|---|---|---|---|---|---|---|---|
| B1 | Abbau | ++ | ++ | + | + | + | - | - |
| | pH | ++ | ++ | + | + | + | + | - |
| | Visko | ++ | ++ | + | + | + | + | - |
| B2 | Abbau | ++ | ++ | + | + | + | + | - |
| | pH | ++ | ++ | ++ | + | + | + | + |
| | Visko | ++ | ++ | ++ | + | + | + | + |
| B3 | Abbau | ++ | ++ | ++ | + | + | + | + |
| | pH | ++ | ++ | + | + | + | + | + |
| | Visko | ++ | ++ | + | + | + | + | + |
| B4 | Abbau | ++ | ++ | + | + | + | - | - |
| | pH | ++ | ++ | + | + | + | - | - |
| | Visko | ++ | + | + | + | + | - | - |
| | | | | | | | | |

| | | **3 M** | **6 M** | **12 M** | **18 M** | **24 M** | **36 M** | **39 M** |
|---|---|---|---|---|---|---|---|---|
| B5 | Abbau | ++ | + | + | + | - | - | - |
| | pH | ++ | ++ | ++ | + | + | - | - |
| | Visko | ++ | ++ | ++ | + | + | - | - |
| B6 | Abbau | ++ | ++ | ++ | + | + | - | - |
| | pH | ++ | ++ | ++ | ++ | ++ | + | - |
| | Visko | ++ | ++ | ++ | ++ | ++ | + | - |
| B7 | Abbau | ++ | ++ | ++ | ++ | + | + | - |
| | pH | ++ | ++ | ++ | ++ | + | + | - |
| | Visko | ++ | ++ | ++ | + | + | - | - |
| B8 | Abbau | ++ | + | + | + | - | - | - |
| | pH | ++ | ++ | + | + | - | - | - |
| | Visko | ++ | + | + | + | + | - | - |

**Tabelle 2C [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| C1 | Abbau | ++ | ++ | ++ | + | + | + | + |
| | pH | ++ | ++ | + | + | + | + | - |
| | Visko | ++ | + | + | + | + | + | - |
| C2 | Abbau | ++ | ++ | + | + | + | - | - |
| | pH | ++ | ++ | ++ | + | + | + | - |
| | Visko | ++ | ++ | + | + | + | - | - |

**Tabelle 2D [(40 ± 2 °C und (75 ± 5 % r.H.]:**

| | | **3 M** | **6 M** | **12 M** | **18 M** | **24 M** | **36 M** | **39 M** |
|---|---|---|---|---|---|---|---|---|
| D1 | Abbau | ++ | + | + | + | + | - | - |
| | pH | ++ | + | + | + | + | + | - |
| | Visko | ++ | + | + | + | + | + | - |
| D2 | Abbau | ++ | + | + | + | + | + | - |
| | pH | ++ | + | + | + | + | - | - |
| | Visko | ++ | + | + | + | - | - | - |

**Tabelle 2E [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| E1 | Abbau | + | + | + | - | - | - | - |
| | pH | + | + | + | + | - | - | - |
| | Visko | + | + | + | - | - | - | - |
| E2 | Abbau | ++ | ++ | + | + | + | - | - |
| | pH | ++ | ++ | + | + | + | + | - |
| | Visko | ++ | ++ | ++ | + | + | + | - |
| E3 | Abbau | ++ | ++ | + | + | + | - | - |
| | pH | ++ | ++ | ++ | + | + | + | + |
| | Visko | ++ | ++ | + | + | + | - | - |
| E4 | Abbau | ++ | + | + | - | - | - | - |
| | pH | ++ | + | + | + | + | - | - |
| | Visko | ++ | + | + | + | - | - | - |

**Tabelle 2F [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| F1 | Abbau | + | + | - | - | - | - | - |
| | pH | + | + | - | - | - | - | - |
| | Visko | + | + | + | - | - | - | - |
| F2 | Abbau | + | + | + | - | - | - | - |
| | pH | ++ | + | + | + | - | - | - |
| | Visko | ++ | + | + | + | - | - | - |
| F3 | Abbau | + | - | - | - | - | - | - |
| | pH | + | + | - | - | - | - | - |
| | Visko | + | + | - | - | - | - | - |
| F4 | Abbau | ++ | + | + | - | - | - | - |
| | pH | ++ | + | + | - | - | - | - |
| | Visko | ++ | + | + | - | - | - | - |

Ein charakteristisches Maß für die Stabilität einer Zusammensetzung ist zum einen die Konstanz des pH-Wertes bzw. der Viskosität sowie die Konstanz des Gehalts an Wirkstoffen (d. h. Komponente (a) sowie Komponente (b)). Weiterhin kann der Gehalt an Abbauprodukten in der Zusammensetzung bestimmt werden, um die Stabilität der Zusammensetzung zu bewerten.

Vor diesem Hintergrund zeigen die durchgeführten Stabilitätsuntersuchungen, dass sowohl die Menge der Wirkstoffkomponenten in der Zusammensetzung als auch deren spezielles Molekulargewicht und darüber hinaus auch der pH-Wert und maßgeblich auch das eingesetzte Puffersystem einen signifikanten Einfluss auf die Stabilität, insbesondere Lagerungsstabilität, der zugrundeliegenden Zusammensetzungen ausüben, wobei die Zusammensetzung A1 mit der speziellen Abstimmung der Komponenten die diesbezüglich besten Eigenschaften aufweist.

Zudem zeigen die Untersuchungen unter Verwendung unterschiedlicher Puffersysteme, dass sich maßgeblich nur mit dem erfindungsgemäß verwendeten Phosphatpuffersystem eine zuverlässige Langzeitstabilität erreichen lässt. Denn, wie die Stabilitätsuntersuchungen der Anmelderin in überraschender Weise zeigen, bewirkt nur ein solches Phosphatpuffersystem - gegenüber anderen möglichen einsetzbaren Puffersystemen - die angestrebte und zuverlässige Langzeitstabilisierung der erfindungsgemäßen Zusammensetzung auch über große Zeiträume. Mit anderen Puffersystemen dagegen, welche sich im vergleichbaren pH-Wertbereich einsetzen lassen, wie z. B. einem Kohlensäure/Bicarbonat-Puffersystem, einem Kohlensäure/Silikat-Puffersystem, einem Essigsäure/Acetat-Puffersystem, einem Citronensäure/Citrat-Puffersystem oder dergleichen, lassen sich derart gute Stabilitätsergebnisse nicht bzw. nicht immer zuverlässig erhalten. Ohne sich auf eine bestimmte Theorie festlegen zu wollen, lässt sich dieser Effekt des erfindungsgemäß eingesetzten Phosphatpuffersystems möglicherweise auch auf sekundäre Effekte beispielsweise im Hinblick auf eine weiterführende Stabilisierung der Matrix bzw. des Hydrogels auf Basis der Komponenten (a) bzw. (b) in der Zusammensetzung zurückführen.

Wie die vorstehend angeführten Ergebnisse der Stabilitätsuntersuchungen der Anmelderin belegen, bedurfte es zur Auffindung einer stabilen Zusammensetzung für die Behandlung vorzugsweise entzündlicher Erkrankungen des Urogenitaltraktes, insbesondere von entzündlichen Erkrankungen der Harnblase, bevorzugt von Cystitis, nach Art der vorliegenden Erfindung eines intensiven Forschungsaufwands seitens der Anmelderin.

Die vorstehenden Stabilitätsuntersuchungen zeigen weiterhin auch die Komplexität des von der Anmelderin aufgefundenen Lösungsansatzes. Hierzu zählen die gemeinsame Anwesenheit der Wirkstoffe bzw. Komponenten (a) und (b) in einem definierten Mengen unter gleichzeitiger Einhaltung bzw. Konstanthaltung des pH-Wertes der Zusammensetzung im zuvor definierten Bereich - ebenso wie weitere Faktoren, wie die Auswahl des geeigneten Puffersystems.

Der erfindungsgemäße Lösungsansatz kommt im Ergebnis in überraschender Weise mit nur wenigen zusätzlichen Inhaltsstoffen aus, welche aber die pharmakologische Wirksamkeit der zugrundeliegenden Wirkstoffe nicht beeinträchtigen; dies ist im Hinblick auf die beabsichtigte pharmazeutische Wirkung von entscheidender Bedeutung.

Die Ergebnisse belegen nicht zuletzt in ihrer Gesamtheit die ausgezeichnete Langzeitstabilität der erfindungsgemäßen Zusammensetzung.

### 3. Anwendungsbeobachtungen und Wirksamkeitsstudien

Jeweiligen Gruppen von Probanden mit einer diagnostizierten interstitiellen (d. h. nichtbakteriellen) Cystitis werden über einen Zeitraum von zwei Monaten die oben angeführten Zusammensetzungen A1 sowie B1, B4 (Menge Chondroitinsulfat-Natrium); B5, B8 (Menge Natriumhyaluronat); C1, C2 (Molmasse Chondroitinsulfat-Natrium); D1, D2 (Molmasse Natriumhyaluronat) sowie E1 und E4 (pH-Wert) verabreicht. Die Verabreichung erfolgt mittels Instillation in die Harnblase. Die Verabreichung wird jeweils über einen Zeitraum von zwei Monaten wöchentlich wiederholt. Das Volumen der instillierten Zusammensetzung beträgt jeweils 50 ml. Pro Ansatz bzw. Zusammensetzung werden 10 Probanden untersucht. Die Untersuchungsabstände betragen jeweils 1 Woche, 2 Wochen, 1 Monat und schließlich 2 Monate, jeweils bezogen auf den Tag der Erstinstillation. Dabei werden folgende Untersuchungskomplexe durchgeführt:
a) Im Rahmen des ersten Untersuchungskomplexes geben die Probanden ihr subjektives Empfinden anhand einer Schulnotenskala von 1 bis 6 (1 = sehr gut bis 6 = ungenügend) an, wobei auch beliebige Zwischenwerte möglich sind. Bei dieser Untersuchung wird insbesondere auf Schmerzen im Bereich der Harnblase sowie des Beckens, übermäßigen Harndrang und auf eine kleine Harnblasenkapazität abgestellt. Es werden die jeweiligen Mittelwerte sowie die dazugehörigen Standardabweichungen ermittelt.
b) In einer zweiten Untersuchung wird einen Monat nach der ersten Applikation der vorgenannten Zusammensetzungen bei den jeweiligen Probandengruppen eine Cytoskopie (Blasenspiegelung) durchgeführt, wobei mittels Endoskop eine visuelle Begutachtung des Blasenurothels erfolgt.
c) Darüber hinaus werden zum Versuchsbeginn sowie nach einem Monat Urinproben genommen und der Gehalt an Glykosaminoglykan im Urin in Anlehnung an das Verfahren nach *Whitley et al.* (vgl. C. B., Ridnour, M. D., Draper, K. A., Dutton, C. M. and Negila, J. P.: Diagnostic test for mucoplysaccharidosis. I. Direct method for quantifying excessive urinary glycosaminoglycan excretion. Clin. Chem., 35: 374, 1989) ermittelt, wobei die im Urin vorhandenen Glykosaminoglykane mittels Dimethylmethylenblau angefärbt und deren Konzentrationen anschließend spektroskopisch bestimmt werden. In diesem Zusammenhang ist bekannt, dass bei Patienten mit diagnostizierter interstitieller Cystitis eine von der Konzentration bei gesunden Patienten, d. h. bei Patienten ohne Befund einer interstitiellen Cystitis, abweichende Konzentration an im Urin vorhandenem Glykosaminoglykan vorliegt. Diesbezüglich ist auch bekannt, dass bei Patienten mit einer weit fortgeschrittenen bzw. chronischen interstitiellen Cystitis der Gehalt an Glykosaminoglykan im Urin im Vergleich zu einer Kontrollgruppe ohne Befund verringert ist, während der Gehalt an Glykosaminoglykan im Urin bei Probanden mit interstitieller Cystitis im Anfangsstadium bzw. im nicht weit fortgeschrittenen Stadium erhöht ist.

Die Untersuchungen und Wirksamkeitsstudien zeigen, dass für die Zusammensetzung A1 im Vergleich zu den weiteren Zusammensetzungen, wie zuvor angeführt, die beste Wirksamkeit in Bezug auf die Behandlung der zugrundeliegenden Erkrankung in Form von interstitieller Cystitis vorliegt. Insbesondere kann eine deutliche Verbesserung des gesundheitlichen Wohlbefindens beobachtet werden, wobei zudem bei den Probanden der mit der Zusammensetzung A1 behandelten Gruppe ein nahezu intakter Blasenmucus und zudem keine Läsionen und keine Einblutungen im Urothel vorliegen. Zudem liegt eine deutliche Verbesserung des Glykosaminoglykangehalts vor. Die Untersuchungen zeigen somit insgesamt auch die hervorragende therapeutische Wirksamkeit der erfindungsgemäßen Konzeption.

## Patentansprüche

1. Zusammensetzung, wobei die Zusammensetzung in Kombination und jeweils in wirksamen Mengen
(a) Chondroitinsulfat und/oder ein physiologisch verträgliches Chondroitinsulfat-Salz in einer Konzentration von (4,5 ± 0,5) mg/ml (Komponente (a)); und
(b) Hyaluronsäure und/oder ein physiologisch verträgliches Hyaluronsäure-Salz (Hyaluronat) in einer Konzentration von (16 ± 1,6) mg/ml (Komponente (b));
enthält,
wobei die Zusammensetzung einen pH-Wert im Bereich von 6,1 bis 7,9 aufweist und/oder wobei die Zusammensetzung auf einen pH-Wert im Bereich von 6,1 bis 7,9 eingestellt ist,
**dadurch gekennzeichnet,**
**dass** die Zusammensetzung
(c) ein Dihydrogenphosphat/Monohydrogenphosphat-Puffersystem (Komponente (c)) enthält.

2. Zusammensetzung nach Anspruch 1,
wobei die Zusammensetzung das Chondroitinsulfat und/oder das physiologisch verträgliche Chondrointisulfat-Salz (Komponente (a)) in einer Konzentration von (4,5 ± 0,4) mg/ml, insbesondere in einer Konzentration von (4,5 ± 0,3) mg/ml, vorzugsweise in einer Konzentration von (4,5 ± 0,15) mg/ml, besonders bevorzugt in einer Konzentration von etwa 4,5 mg/ml, aufweist; und/oder
wobei das Chondroitinsulfat und/oder das physiologisch verträgliche Chondroitinsulfat-Salz (Komponente (a)) ein zahlenmittleres Molekulargewicht (Molmasse) Mₙ im Bereich von 2 kDa bis 200 kDa, insbesondere im Bereich von 5 kDa bis 150 kDa, vorzugsweise im Bereich von 10 kDa bis 100 kDa, bevorzugt im Bereich von 12 kDa bis 75 kDa, aufweist; und/oder
wobei das Chondroitinsulfat und/oder das physiologisch verträgliche Chondroitinsulfat-Salz (Komponente (a)) ein gewichtsmittleres Molekulargewicht (Molmasse) M_{w} im Bereich von 10 kDa bis 200 kDa, insbesondere im Bereich von 15 kDa bis 175 kDa, vorzugsweise im Bereich von 20 kDa bis 150 kDa, bevorzugt im Bereich von 30 kDa bis 120 kDa, aufweist; und/oder
wobei das Chondroitinsulfat und/oder das physiologisch verträgliche Chondroitinsulfat-Salz (Komponente (a)) ein zentrifugenmittleres Molekulargewicht (Molmasse) M_{z} im Bereich von 30 kDa bis 1.000 kDa, insbesondere im Bereich von 40 kDa bis 800 kDa, vorzugsweise im Bereich von 50 kDa bis 600 kDa, bevorzugt im Bereich von 100 kDa bis 450 kDa, aufweist; und/oder
wobei das Chondroitinsulfat und/oder das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ, von mindestens 1, insbesondere mindestens 1,2, vorzugsweise mindestens 1,5, bevorzugt mindestens 2, aufweist; und/oder
wobei das Chondroitinsulfat und/oder das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ, von höchstens 30, insbesondere höchstens 20, vorzugsweise höchstens 10, bevorzugt höchstens 8, aufweist; und/oder
wobei das Chondroitinsulfat und/oder das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ, im Bereich von 1 bis 30, insbesondere im Bereich von 1,2 bis 20, vorzugsweise im Bereich von 1,5 bis 10, bevorzugt im Bereich von 2 bis 8, aufweist.

3. Zusammensetzung nach Anspruch 1 oder 2,
wobei die Zusammensetzung die Hyaluronsäure und/oder das physiologisch verträgliche Hyaluronsäure-Salz (Komponente (b)) in einer Konzentration von (16 ± 1,2) mg/ml, insbesondere in einer Konzentration von (16 ± 0,8) mg/ml, vorzugsweise in einer Konzentration von (16 ± 0,4) mg/ml, besonders bevorzugt in einer Konzentration von etwa 16 mg/ml, enthält; und/oder
wobei die Hyaluronsäure und/oder das physiologisch verträgliche Hyaluronsäure-Salz (Komponente (b)) ein zahlenmittleres Molekulargewicht (Molmasse) Mₙ im Bereich von 10 kDa bis 300 kDa, insbesondere im Bereich von 20 kDa bis 275 kDa, vorzugsweise im Bereich von 30 kDa bis 260 kDa, bevorzugt im Bereich von 50 kDa bis 250 kDa, besonders bevorzugt im Bereich von 75 kDa bis 200 kDa, aufweist; und/oder
wobei die Hyaluronsäure und/oder das physiologisch verträgliche Hyaluronsäure-Salz (Komponente (b)) ein gewichtsmittleres Molekulargewicht (Molmasse) M_{w} im Bereich von 10 kDa bis 500 kDa, insbesondere im Bereich von 20 kDa bis 450 kDa, vorzugsweise im Bereich von 50 kDa bis 425 kDa, bevorzugt im Bereich von 100 kDa bis 400 kDa, besonders bevorzugt im Bereich von 150 kDa bis 395 kDa, aufweist; und/oder
wobei die Hyaluronsäure und/oder das physiologisch verträgliche Hyaluronsäure-Salz (Komponente (b)) ein zentrifugenmittleres Molekulargewicht (Molmasse) M_{z} im Bereich von 80 kDa bis 1.500 kDa, insbesondere im Bereich von 100 kDa bis 1.250 kDa, vorzugsweise im Bereich von 200 kDa bis 1.000 kDa, bevorzugt im Bereich von 300 kDa bis 750 kDa, aufweist; und/oder
wobei die Hyaluronsäure und/oder das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, von mindestens 1, insbesondere mindestens 1,1, vorzugsweise mindestens 1,2, bevorzugt mindestens 1,3, besonders bevorzugt mindestens 1,4, aufweist; und/oder
wobei die Hyaluronsäure und/oder das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, von höchstens 50, insbesondere höchstens 25, vorzugsweise höchstens 10, bevorzugt höchstens 5, besonders bevorzugt höchstens 3, aufweist; und/oder
wobei die Hyaluronsäure und/oder das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, im Bereich von 1 bis 50, insbesondere im Bereich von 1,1 bis 25, vorzugsweise im Bereich von 1,2 bis 10, bevorzugt im Bereich von 1,3 bis 5, besonders bevorzugt im Bereich von 1,4 bis 3, aufweist.

4. Zusammensetzung nach einem der vorangehenden Ansprüche,
wobei die Zusammensetzung das Dihydrogenphosphat/Monohydrogenphosphat-Puffersystem (Komponente (c)) in einer Gesamtkonzentration an Dihydrogenphosphat/Monohydrogenphosphat-Puffersystem (Komponente (c)) von (1,75 ± 1,65) mg/ml, insbesondere in einer Gesamtkonzentration von (1,75 ± 1,5) mg/ml, vorzugsweise in einer Gesamtkonzentration von (1,75 ± 1,25) mg/ml, bevorzugt in einer Gesamtkonzentration von (1,75 ± 1) mg/ml, besonders bevorzugt in einer Gesamtkonzentration von (1,75 ± 0,9) mg/ml, ganz besonders bevorzugt in einer Gesamtkonzentration von (1,75 ± 0,8) mg/ml, noch weiter bevorzugt in einer Gesamtkonzentration von etwa 1,75 mg/ml, enthält; und/oder
wobei die Zusammensetzung das Dihydrogenphosphat/Monohydrogenphosphat-Puffersystem (Komponente (c)) in einer Gesamtmenge an Dihydrogenphosphat/Monohydrogenphosphat-Puffersystem (Komponente (c)) von (87,5 ± 85) mg, insbesondere in einer Gesamtmenge von (87,5 ± 75) mg, vorzugsweise in einer Gesamtmenge von (87,5 ± 62,5) mg, bevorzugt in einer Gesamtmenge von (87,5 ± 50) mg, besonders bevorzugt in einer Gesamtmenge von (87,5 ± 45) mg, ganz besonders bevorzugt in einer Gesamtmenge von (87,5 ± 40) mg, noch weiter bevorzugt in einer Gesamtmenge von etwa 87,5 mg, enthält; und/oder
wobei die Zusammensetzung das Dihydrogenphosphat des Dihydrogenphosphat/Monohydrogenphosphat-Puffersystems (Komponente (c)), insbesondere bezogen auf die Einwaage bei Herstellung und/oder Bereitstellung der Zusammensetzung, in einer Konzentration von (0,2 ± 0,19) mg/ml, insbesondere in einer Konzentration von (0,2 ± 0,15) mg/ml, vorzugsweise in einer Konzentration von (0,2 ± 0,125) mg/ml, bevorzugt in einer Konzentration von (0,2 ± 0,1) mg/ml, besonders bevorzugt in einer Konzentration von etwa 0,2 mg/ml, enthält; und/oder
wobei die Zusammensetzung das Monohydrogenphosphat des Dihydrogenphosphat/Monohydrogenphosphat-Puffersystems (Komponente (c)), insbesondere bezogen auf die Einwaage bei Herstellung und/oder Bereitstellung der Zusammensetzung, in einer Konzentration von (1,5 ± 1,4) mg/ml, insbesondere in einer Konzentration von (1,5 ± 1,25) mg/ml, vorzugsweise in einer Konzentration von (1,5 ± 1,1) mg/ml, bevorzugt in einer Konzentration von (1,5 ± 1) mg/ml, besonders bevorzugt in einer Konzentration von (1,5 ± 0,75) mg/ml, ganz besonders bevorzugt in einer Konzentration von etwa 1,5 mg/ml, enthält; und/oder
wobei die Zusammensetzung das Dihydrogenphosphat/Monohydrogenphosphat-Puffersystem (Komponente (c)) in einem Gewichtsverhältnis (Konzentrationsverhältnis) von Dihydrogenphosphat zu Monohydrogenposphat [Dihydrogenphosphat : Monohydrogenposphat], insbesondere bezogen auf die Einwaage bei Herstellung und/oder Bereitstellung der Zusammensetzung, im Bereich von 2 : 1 bis 1 : 100, vorzugsweise im Bereich von 1 : 1 bis 1 : 75, bevorzugt im Bereich von 1 : 2 bis 1 : 50, besonders bevorzugt im Bereich von 1 : 5 bis 1 : 25, enthält.

5. Zusammensetzung nach einem der vorangehenden Ansprüche,
wobei das Dihydrogenphosphat/Monohydrogenphosphat-Puffersystem (Komponente (c)) als Alkalidihydrogenphosphat/Alkalimonohydrogenposphat-Puffersystem vorliegt und/oder ausgebildet ist; insbesondere wobei das Alkalimetall ausgewählt ist aus Natrium und/oder Kalium, insbesondere Natrium; und/oder
wobei das Dihydrogenphosphat/Monohydrogenphosphat-Puffersystem (Komponente (c)) als Natriumdihydrogenphosphat/Natriummonohydrogenposphat-Puffersystem, vorliegt und/oder ausgebildet ist, und/oder
wobei das Dihydrogenphosphat/Monohydrogenphosphat-Puffersystem (Komponente (c)) als NaH₂(PO₄)/Na₂H(PO₄)-Puffersystem, insbesondere als NaH₂(PO₄) · 2 H₂O/Na₂H(PO₄) · 2 H₂O-Puffersystem, vorliegt und/oder ausgebildet ist; und/oder
wobei das Dihydrogenphosphat/Monohydrogenphosphat-Puffersystem (Komponente (c)) zur Einstellung und/oder Konstanthaltung des pH-Wertes der Zusammensetzung dient und/oder eingesetzt ist.

6. Zusammensetzung nach einem der vorangehenden Ansprüche,
wobei die Zusammensetzung den Elektrolyten (Komponente (d)) in einer Konzentration von (8 ± 6) mg/ml, insbesondere in einer Konzentration von (8 ± 4) mg/ml, vorzugsweise in einer Konzentration von (8 ± 2) mg/ml, bevorzugt in einer Konzentration von (8 ± 1) mg/ml, besonders bevorzugt in einer Konzentration von etwa 8 mg/ml, enthält; und/oder
wobei der Elektrolyt (Komponente (d)) in Form eines Alkalisalzes, insbesondere in Form eines Alkalichlorids, vorzugsweise in Form von Natriumchlorid, vorliegt und/oder wobei der Elektrolyt (Komponente (d)) Natriumchlorid ist.

7. Zusammensetzung nach einem der vorangehenden Ansprüche,
wobei die Zusammensetzung einen pH-Wert im Bereich von 6,6 bis 7,7, insbesondere im Bereich von 6,9 bis 7,6, vorzugsweise im Bereich von 7,1 bis 7,4, aufweist; und/oder
wobei der pH-Wert der Zusammensetzung im Bereich von 6,6 bis 7,7, insbesondere im Bereich von 6,9 bis 7,6, vorzugsweise im Bereich von 7,1 bis 7,4, konstant gehalten und/oder eingestellt ist; und/oder
wobei der pH-Wert mittels des Alkalidihydrogenphosphat/Alkalimonohydrogenposphat-Puffersystems (Komponente (c)) eingestellt und/oder vorgegeben ist; und/oder
wobei die Zusammensetzung als wässrige Zusammensetzung vorliegt und/oder wobei die Zusammensetzung wässrig basiert ist und/oder wässrig formuliert vorliegt, insbesondere in Form einer wässrigen Lösung und/oder wässrigen Suspension, vorzugsweise in Form einer wässrigen Lösung; und/oder
wobei die Zusammensetzung Wasser, insbesondere gereinigtes Wasser, enthält und/oder wobei die Zusammensetzung Wasser als pharmazeutisch verträglichen Träger (Exzipienten) enthält und/oder wobei die Zusammensetzung wässrig basiert ausgebildet ist;
und/oder wobei die Zusammensetzung einen Gehalt an Wasser von mindestens 50 Gew.-%, insbesondere mindestens 75 Gew.-%, vorzugsweise mindestens 80 Gew.-%, bevorzugt mindestens 90 Gew.-%, besonders bevorzugt mindestens 95 Gew.-%, bezogen auf die Zusammensetzung, aufweist; und/oder
wobei die Zusammensetzung aus den vorgenannten Komponenten (a), (b), (c) und gegebenenfalls (d) sowie gegebenenfalls Wasser, insbesondere gereinigtem Wasser, besteht.

8. Zusammensetzung nach einem der vorangehenden Ansprüche,
wobei die Zusammensetzung bei Temperaturen im Bereich von 20 °C bis 45 °C, bei einem Druck von 1.013,25 mbar (Atmosphärendruck) und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerungsstabil, ist; insbesondere wobei die Zusammensetzung zum jeweiligen Lagerungszeitpunkt einen Gesamtgehalt an Abbauprodukten der Komponenten (a) und (b) von höchstens 5 %, insbesondere höchstens 4 %, vorzugsweise höchstens 3 %, bevorzugt höchstens 2 %, besonders bevorzugt höchstens 1 %, bezogen auf die Gesamtkonzentration der Komponenten (a) und (b), aufweist; und/oder
wobei die Zusammensetzung eine Stabilität, insbesondere Lagerungsstabilität, unter beschleunigten Alterungsbedingungen gemäß ASTM F 1980 bei einer Alterungstemperatur von 55 °C von mindestens 6 Monate, insbesondere mindestens 12 Monate, vorzugsweise mindestens 24 Monate, bevorzugt mindestens 36 Monate, aufweist; insbesondere wobei die Zusammensetzung zum jeweiligen Lagerungszeitpunkt einen Gesamtgehalt an Abbauprodukten der Komponenten (a) und (b) von höchstens 5 %, insbesondere höchstens 4 %, vorzugsweise höchstens 3 %, bevorzugt höchstens 2 %, besonders bevorzugt höchstens 1 %, bezogen auf die Gesamtkonzentration der Komponenten (a) und (b), aufweist.

9. Zusammensetzung nach einem der vorangehenden Ansprüche,
wobei die Zusammensetzung, insbesondere anwendungs-, dosier- und/oder applikationsfertig, mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegt; und/oder
wobei die Zusammensetzung, insbesondere anwendungs-, dosier- und/oder applikationsfertig, zur Verabreichung, insbesondere Instillation in die Harnblase, eines Volumens der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegt und/oder hergerichtet ist bzw. wobei die Zusammensetzung, insbesondere anwendungs-, dosier- und/oder applikationsfertig, insbesondere mittels Instillation in die Harnblase, mit einem Volumen der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, verabreicht wird; und/oder
wobei die Zusammensetzung, insbesondere anwendungs-, dosier- und/oder applikationsfertig, mit einer Wirkstoffmenge an Chondroitinsulfat und/oder physiologisch verträglichem Chondroitinsulfat-Salz (Komponente (a)) von (225 ± 25) mg, insbesondere (225 ± 15) mg, vorzugsweise (225 ± 10) mg, vorliegt; und/oder
wobei die Zusammensetzung, insbesondere anwendungs-, dosier- und/oder applikationsfertig, zur Verabreichung einer Wirkstoffmenge an Chondroitinsulfat und/oder physiologisch verträglichem Chondroitinsulfat-Salz (Komponente (a)) von (225 ± 25) mg, insbesondere (225 ± 15) mg, vorzugsweise (225 ± 10) mg, vorliegt und/oder hergerichtet ist bzw. wobei die Zusammensetzung, insbesondere anwendungs-, dosier- und/oder applikationsfertig, mit einer Wirkstoffmenge an Chondroitinsulfat und/oder physiologisch verträglichem Chondroitinsulfat-Salz (Komponente (a)) von (225 ± 25) mg, insbesondere (225 ± 15) mg, vorzugsweise (225 ± 10) mg, verabreicht wird; und/oder
wobei die Zusammensetzung, insbesondere anwendungs-, dosier- und/oder applikationsfertig, mit einer Wirkstoffmenge an Hyaluronsäure und/oder physiologisch verträglichem Hyaluronsäure-Salz (Hyaluronat) (Komponente (b)) von (800 ± 80) mg, insbesondere (800 ± 60) mg, vorzugsweise (800 ± 40) mg, vorliegt; und/oder
wobei die Zusammensetzung, insbesondere anwendungs-, dosier- und/oder applikationsfertig, zur Verabreichung einer Wirkstoffmenge an Hyaluronsäure und/oder physiologisch verträglichem Hyaluronsäure-Salz (Hyaluronat) (Komponente (b)) von (800 ± 80) mg, insbesondere (800 ± 60) mg, vorzugsweise (800 ± 40) mg, vorliegt und/oder hergerichtet ist bzw. wobei die Zusammensetzung, insbesondere anwendungs-, dosier- und/oder applikationsfertig, mit einer Wirkstoffmenge an Hyaluronsäure und/oder physiologisch verträglichem Hyaluronsäure-Salz (Hyaluronat) (Komponente (b)) von (800 ± 80) mg, insbesondere (800 ± 60) mg, vorzugsweise (800 ± 40) mg, verabreicht wird.

10. Zusammensetzung nach einem der vorangehenden Ansprüche
zur Instillation und/oder zur vorzugsweise topischen Applikation in den Urogenitalbereich, insbesondere in die Harnblase; und/oder
wobei die Zusammensetzung zur Verabreichung und/oder zu Instillation und/oder zur vorzugsweise topischen Applikation in den Urogenitaltrakt, insbesondere in die Harnblase, hergerichtet ist bzw. wobei die Zusammensetzung mittels Instillation und/oder zur vorzugsweise topischen Applikation in den Urogenitaltrakt, insbesondere in die Harnblase, verabreicht wird.

11. Zusammensetzung nach einem der vorangehenden Ansprüche zur Verwendung bei der prophylaktischen und/oder therapeutischen Behandlung von vorzugsweise entzündlichen Erkrankungen des Urogenitaltraktes, insbesondere von entzündlichen Erkrankungen der Harnblase, bevorzugt von Cystitis, insbesondere akuter oder chronischer Cystitis, bevorzugt interstitieller Cystitis, radiogener Cystitis, chronisch rezidivierender Cystitis, Chemocystitis, chronisch abakterieller Cystitis und chronisch bakterieller Cystitis, besonders bevorzugt interstitieller Cystitis.

12. Aufbewahrungs- und/oder Applikationsvorrichtung, insbesondere Aufbewahrungs- und/oder Applikationsbehältnis, insbesondere in Form einer vorzugsweise sterilen Spritze, insbesondere Applikationsspritze, vorzugsweise Einweg-Applikationsspritze, vorzugsweise zur Instillation und/oder zur vorzugsweise topischen Applikation in den Urogenitalbereich, insbesondere in die Harnblase, enthaltend eine Zusammensetzung nach einem der vorangehenden Ansprüche.

13. Aufbewahrungs- und/oder Applikationsvorrichtung nach Anspruch 12,
wobei die Zusammensetzung eine Wirkstoffmenge an (a) Chondroitinsulfat und/oder physiologisch verträglichem Chondroitinsulfat-Salz (Komponente (a)) von (225 ± 15) mg, vorzugsweise (225 ± 10) mg, aufweist; und/oder
wobei die Zusammensetzung eine Wirkstoffmenge an (b) Hyaluronsäure und/oder physiologisch verträglichem Hyaluronsäure-Salz (Hyaluronat) (Komponente (b)) von (800 ± 60) mg, vorzugsweise (800 ± 40) mg, aufweist; und/oder
wobei die Zusammensetzung das Dihydrogenphosphat/Monohydrogenphosphat-Puffersystem (Komponente (c)) in einer Gesamtmenge an Dihydrogenphosphat/Monohydrogenphosphat-Puffersystem (Komponente (c)) von (87,5 ± 85) mg, insbesondere in einer Gesamtmenge von (87,5 ± 75) mg, vorzugsweise in einer Gesamtmenge von (87,5 ± 62,5) mg, bevorzugt in einer Gesamtmenge von (87,5 ± 50) mg, besonders bevorzugt in einer Gesamtmenge von (87,5 ± 45) mg, ganz besonders bevorzugt in einer Gesamtmenge von (87,5 ± 40) mg, noch weiter bevorzugt in einer Gesamtmenge von etwa 87,5 mg, aufweist; und/oder
wobei die Zusammensetzung den Elektrolyten (Komponente (d)) in einer Menge von (400 ± 300) mg, insbesondere in einer Menge von (400 ± 200) mg, vorzugsweise in einer Menge von (400 ± 100) mg, bevorzugt in einer Menge von (400 ± 50) mg, besonders bevorzugt in einer Menge von etwa 400 mg, enthält; und/oder
wobei die Aufbewahrungs- und/oder Applikationsvorrichtung die Zusammensetzung, insbesondere anwendungs-, dosier- und/oder applikationsfertig, mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, enthält; und/oder
wobei die Zusammensetzung mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegt.

14. Verpackungseinheit, enthaltend mindestens eine Aufbewahrungs- und/oder Applikationsvorrichtung nach Anspruch 12 oder 13, insbesondere wobei die Aufbewahrungs- und/oder Applikationsvorrichtung in einer vor Kontaminationen schützenden Umverpackung eingebracht vorliegt.

15. Kit, insbesondere Instillationssystem, umfassend (i) mindestens eine Aufbewahrungs- und/oder Applikationsvorrichtung nach Anspruch 12 oder 13, (ii) mindestens eine Zusammensetzung nach einem der vorangehenden Ansprüche, insbesondere wobei die Zusammensetzung vorzugsweise anwendungs-, dosier- und/oder applikationsfertig in der Aufbewahrungs- und/oder Applikationsvorrichtung vorliegt, und (iii) mindestens eine an die Aufbewahrungs- und/oder Applikationsvorrichtung anschließbare Instillationsvorrichtung, insbesondere in Form eines Instillationsschlauches oder dergleichen.

## Claims

1. A composition containing a combination of respectively effective amounts of
(a) Chondroitin sulfate and/or a physiologically acceptable chondroitin sulfate salt in a concentration of (4.5 ± 0.5) mg/ml (component (a)); and
(b) Hyaluronic acid and/or a physiologically acceptable hyaluronic acid salt (hyaluronate) in a concentration of (16 ± 1.6) mg/ml (component (b));
wherein the composition has a pH value in the range of 6.1 to 7.9 and/or wherein the composition is adjusted to a pH value in the range of 6.1 to 7.9,
**characterised in that**
the composition contains
(c) a dihydrogen phosphate/monohydrogen phosphate buffer system (component (c)).

2. The composition according to claim 1,
wherein the composition comprises the chondroitin sulfate and/or the physiologically acceptable chondroitin sulfate salt (component (a)) in a concentration of (4.5 ± 0.4) mg/ml, in particular in a concentration of (4.5 ± 0.3) mg/ml, preferably in a concentration of (4.5 ± 0.15) mg/ml, particularly preferably in a concentration of about 4.5 mg/ml; and/or
wherein the chondroitin sulfate and/or the physiologically acceptable chondroitin sulfate salt (component (a)) has a number-average molecular weight (molar mass) Mₙ in the range of 2 kDa to 200 kDa, in particular in the range of 5 kDa to 150 kDa, preferably in the range of 10 kDa up to 100 kDa, more preferably in the range of 12 kDa to 75 kDa; and/or
wherein the chondroitin sulfate and/or the physiologically acceptable chondroitin sulfate salt (component (a)) has a weight-average molecular weight (molar mass) M_{w} in the range of 10 kDa to 200 kDa, in particular in the range of 15 kDa to 175 kDa, preferably in the range of 20 kDa up to 150 kDa, more preferably in the range of 30 kDa to 120 kDa; and/or
wherein the chondroitin sulfate and/or the physiologically acceptable chondroitin sulfate salt (component (a)) has a centrifuge-average molecular weight (molar mass) M_{z} in the range of 30 kDa to 1,000 kDa, in particular in the range of 40 kDa to 800 kDa, preferably in the range of 50 kDa up to 600 kDa, more preferably in the range of 100 kDa to 450 kDa; and/or
wherein the chondroitin sulfate and/or the physiologically acceptable chondroitin sulfate salt (component (a)) has a polydispersity index (PDI), calculated as the quotient of weight-average molecular weight M_{w} and number-average molecular weight Mₙ, of at least 1, in particular at least 1.2, preferably at least 1.5, more preferably at least 2; and/or
wherein the chondroitin sulfate and/or the physiologically acceptable chondroitin sulfate salt (component (a)) has a polydispersity index (PDI), calculated as the quotient of weight-average molecular weight M_{w} and number-average molecular weight Mₙ, of at most 30, in particular at most 20, preferably at most 10, more preferably at most 8; and/or
wherein the chondroitin sulfate and/or the physiologically acceptable chondroitin sulfate salt (component (a)) has a polydispersity index (PDI), calculated as the quotient of weight-average molecular weight M_{w} and number-average molecular weight Mₙ, in the range from 1 to 30, in particular in the range of 1.2 to 20, preferably in the range of 1.5 to 10, more preferably in the range of 2 to 8.

3. The composition according to claim 1 or 2,
wherein the composition contains the hyaluronic acid and/or the physiologically acceptable hyaluronic acid salt (component (b)) in a concentration of (16 ± 1.2) mg/ml, in particular in a concentration of (16 ± 0.8) mg/ml, preferably in a concentration of (16 ± 0.4) mg/ml, particularly preferably in a concentration of about 16 mg/ml; and/or
wherein the hyaluronic acid and/or the physiologically acceptable hyaluronic acid salt (component (b)) has a number-average molecular weight (molar mass) Mₙ in the range of 10 kDa to 300 kDa, in particular in the range of 20 kDa to 275 kDa, preferably in the range of 30 kDa to 260 kDa, more preferably in the range of 50 kDa to 250 kDa, particularly preferably in the range of 75 kDa to 200 kDa; and/or
wherein the hyaluronic acid and/or the physiologically acceptable hyaluronic acid salt (component (b)) has a weight-average molecular weight (molar mass) M_{w} in the range of 10 kDa to 500 kDa, in particular in the range of 20 kDa to 450 kDa, preferably in the range of 50 kDa to 425 kDa, more preferably in the range of 100 kDa to 400 kDa, particularly preferably in the range of 150 kDa to 395 kDa; and/or
wherein the hyaluronic acid and/or the physiologically acceptable hyaluronic acid salt (component (b)) has a centrifuge-average molecular weight (molar mass) M_{z} in the range of 80 kDa to 1,500 kDa, in particular in the range of 100 kDa to 1,250 kDa, preferably in the range of 200 kDa up to 1,000 kDa, more preferably in the range of 300 kDa to 750 kDa; and/or
wherein the hyaluronic acid and/or the physiologically acceptable hyaluronic acid salt (component (b)) has a polydispersity index (PDI), calculated as the quotient of weight-average molecular weight M_{w} and number-average molecular weight Mₙ, of at least 1, in particular at least 1.1, preferably at least 1.2, more preferably at least 1.3, particularly preferably at least 1.4; and/or
wherein the hyaluronic acid and/or the physiologically acceptable hyaluronic acid salt (component (b)) has a polydispersity index (PDI), calculated as the quotient of weight-average molecular weight M_{w} and number-average molecular weight Mₙ, of at most 50, in particular at most 25, preferably at most 10, more preferably at most 5, particularly preferably at most 3; and/or
wherein the hyaluronic acid and/or the physiologically acceptable hyaluronic acid salt (component (b)) has a polydispersity index (PDI), calculated as the quotient of weight-average molecular weight M_{w} and number-average molecular weight Mₙ, in the range from 1 to 50, in particular in the range from 1.1 to 25, preferably in the range of 1.2 to 10, more preferably in the range of 1.3 to 5, particularly preferably in the range of 1.4 to 3.

4. The composition according to any one of the preceding claims,
wherein the composition contains the dihydrogen phosphate/monohydrogen phosphate buffer system (component (c)) in a total concentration of dihydrogen phosphate/monohydrogen phosphate buffer system (component (c)) of (1.75 ± 1.65) mg/ml, in particular in a total concentration of (1.75 ± 1.5) mg/ml, preferably in a total concentration of (1.75 ± 1.25) mg/ml, more preferably in a total concentration of (1.75 ± 1) mg/ml, particularly preferably in a total concentration of (1.75 ± 0.9) mg/ml, very particularly preferably in a total concentration of (1.75 ± 0.8) mg/ml, even more preferably in a total concentration of about 1.75 mg/ml; and/or
wherein the composition contains the dihydrogen phosphate/monohydrogen phosphate buffer system (component (c)) in a total amount of dihydrogen phosphate/monohydrogen phosphate buffer system (component (c)) of (87.5 ± 85) mg, in particular in a total amount of (87.5 ± 75) mg, preferably in a total amount of (87.5 ± 62.5) mg, more preferably in a total amount of (87.5 ± 50) mg, particularly preferably in a total amount of (87.5 ± 45) mg, very particularly preferably in a total amount of (87.5 ± 40) mg, even more preferably in a total amount of about 87.5 mg; and/or
wherein the composition contains the dihydrogen phosphate of the dihydrogen phosphate/monohydrogen phosphate buffer system (component (c)), in particular based on the initial weight during manufacture and/or preparation of the composition, in a concentration of (0.2 ± 0.19) mg/ml, in particular at a concentration of (0.2 ± 0.15) mg/ml, preferably at a concentration of (0.2 ± 0.125) mg/ml, more preferably in a concentration of (0.2 ± 0.1) mg/ml, particularly preferably in a concentration of about 0.2 mg/ml; and/or
wherein the composition contains the monohydrogen phosphate of the dihydrogen phosphate/monohydrogen phosphate buffer system (component (c)), in particular based on the initial weight during manufacture and/or preparation of the composition, in a concentration of (1.5 ± 1.4) mg/ml, in particular in a concentration of (1.5 ± 1.25) mg/ml, preferably at a concentration of (1.5 ± 1.1) mg/ml, more preferably in a concentration of (1.5 ± 1) mg/ml, particularly preferably at a concentration of (1.5 ± 0.75) mg/ml, very particularly preferably in a concentration of about 1.5 mg/ml; and/or
wherein the composition contains the dihydrogen phosphate/monohydrogen phosphate buffer system (component (c)) in a weight ratio (concentration ratio) of dihydrogen phosphate to monohydrogen phosphate [dihydrogen phosphate: monohydrogen phosphate], in particular based on the initial weight during manufacture and/or preparation of the composition, in the range of 2:1 to 1:100, preferably in the range of 1:1 to 1:75, more preferably in the range of 1:2 to 1:50, particularly preferably in the range of 1:5 to 1:25.

5. The composition according to any one of the preceding claims,
wherein the dihydrogen phosphate/monohydrogen phosphate buffer system (component (c)) is present and/or formed as an alkali dihydrogen phosphate/alkali monohydrogen phosphate buffer system; in particular wherein the alkali metal is selected from sodium and/or potassium, in particular sodium; and/or
wherein the dihydrogen phosphate/monohydrogen phosphate buffer system (component (c)) is present and/or formed as a sodium dihydrogen phosphate/sodium monohydrogen phosphate buffer system, and/or
wherein the dihydrogen phosphate/monohydrogen phosphate buffer system (component (c)) is present and/or formed as an NaH₂(PO₄)/Na₂H(PO₄) buffer system, in particular as an NaH₂(PO₄) • 2 H₂O/Na₂H(PO₄) • 2 H₂O buffer system; and/or
wherein the dihydrogen phosphate/monohydrogen phosphate buffer system (component (c)) serves to and/or is used to adjust and/or maintain the pH value of the composition.

6. The composition according to any one of the preceding claims,
wherein the composition contains the electrolytes (component (d)) in a concentration of (8 ± 6) mg/ml, in particular in a concentration of (8 ± 4) mg/ml, preferably in a concentration of (8 ± 2) mg/ml, more preferably in a concentration of (8 ± 1) mg/ml, particularly preferably in a concentration of about 8 mg/ml; and/or
wherein the electrolyte (component (d)) is present in the form of an alkali salt, in particular in the form of an alkaline chloride, preferably in the form of sodium chloride, and/or wherein the electrolyte (component (d)) is sodium chloride.

7. The composition according to any one of the preceding claims,
wherein the composition has a pH value in the range of 6.6 to 7.7, in particular in the range of 6.9 to 7.6, preferably in the range of 7.1 to 7.4; and/or
wherein the pH value of the composition is kept constant and/or adjusted in the range of 6.6 to 7.7, in particular in the range of 6.9 to 7.6, preferably in the range of 7.1 to 7.4; and/or
wherein the pH value is adjusted and/or predetermined by means of the alkali dihydrogen phosphate/alkali monohydrogen phosphate buffer system (component (c)); and/or
wherein the composition is present as an aqueous composition and/or wherein the composition is aqueous-based and/or has an aqueous formulation, in particular in the form of an aqueous solution and/or aqueous suspension, preferably in the form of an aqueous solution; and/or
wherein the composition contains water, in particular purified water, and/or wherein the composition contains water as a pharmaceutically acceptable carrier (excipient) and/or wherein the composition is aqueous-based;
and/or wherein the composition has a water content of at least 50% by weight, in particular at least 75% by weight, preferably at least 80% by weight, more preferably at least 90% by weight, particularly preferably at least 95% by weight, based on the composition; and/or
wherein the composition consists of the aforementioned components (a), (b), (c) and optionally (d) and optionally water, in particular purified water.

8. The composition according to any one of the preceding claims,
wherein the composition is stable, in particular storage-stable, for at least 6 months, in particular at least 12 months, preferably at least 24 months, more preferably at least 36 months at temperatures in the range of 20°C to 45°C, at a pressure of 1,013.25 mbar (atmospheric pressure) and at a relative humidity in the range of 50% to 90%; in particular wherein the composition at the respective storage time point has a total content of degradation products of components (a) and (b) of at most 5%, in particular at most 4%, preferably at most 3%, more preferably at most 2%, particularly preferably at most 1%, based on the total concentration of the components (a) and (b); and/or
wherein the composition has a stability, in particular storage stability, under accelerated ageing conditions according to ASTM F 1980 at an ageing temperature of 55°C of at least 6 months, in particular at least 12 months, preferably at least 24 months, more preferably at least 36 months; in particular wherein the composition at the respective storage time point has a total content of degradation products of the components (a) and (b) of at most 5%, in particular at most 4%, preferably at most 3%, preferably at most 2%, particularly preferably at most 1%, based on the total concentration of components (a) and (b).

9. The composition according to any one of the preceding claims,
wherein the composition is present, in particular ready for use, dosing and/or application, with a volume of (50 ± 10) ml, in particular (50 ± 5) ml, preferably (50 ± 2) ml, more preferably (50 ± 1) ml, particularly preferably (50 ± 0.5) ml, very particularly preferably about 50 ml; and/or
wherein the composition is present and/or is prepared, in particular ready for use, dosing and/or application, for administration, in particular instillation into the urinary bladder, at a volume of the composition of (50 ± 10) ml, in particular (50 ± 5) ml, preferably (50 ± 2) ml, more preferably (50 ± 1) ml, particularly preferably (50 ± 0.5) ml, very particularly preferably about 50 ml or wherein the composition is administered, in particular ready for use, dosing and/or application, in particular by means of instillation into the urinary bladder, with a volume of the composition of (50 ± 10) ml, in particular (50 ± 5) ml, preferably (50 ± 2) ml, more preferably (50 ± 1) ml, particularly preferably (50 ± 0.5) ml, very particularly preferably about 50 ml; and/or
wherein the composition is present, in particular ready for use, dosing and/or application, with an active ingredient amount of chondroitin sulfate and/or physiologically acceptable chondroitin sulfate salt (component (a)) of (225 ± 25) mg, in particular (225 ± 15) mg, preferably (225 ± 10) mg; and/or
wherein the composition is present and/or is prepared, in particular ready for use, dosing and/or application, for administering of an active ingredient amount of chondroitin sulfate and/or physiologically acceptable chondroitin sulfate salt (component (a)) of (225 ± 25) mg, in particular (225 ± 15) mg, preferably (225 ± 10) mg, or wherein the composition is administered, in particular ready for use, dosing and/or application, with an active ingredient amount of chondroitin sulfate and/or physiologically acceptable chondroitin sulfate salt (component (a)) of (225 ± 25) mg, in particular (225 ± 15) mg, preferably (225 ± 10) mg; and/or
wherein the composition is present, in particular ready for use, dosing and/or application, with an active ingredient amount of hyaluronic acid and/or physiologically acceptable hyaluronic acid salt (hyaluronate) (component (b)) of (800 ± 80) mg, in particular (800 ± 60) mg, preferably (800 ± 40) mg; and/or
wherein the composition is present and/or is prepared, in particular ready for use, dosing and/or application, for administering an active ingredient amount of hyaluronic acid and/or physiologically acceptable hyaluronic acid salt (hyaluronate) (component (b)) of (800 ± 80) mg, in particular (800 ± 60) mg, preferably (800 ± 40) mg, or wherein the composition is administered, in particular ready for use, dosing and/or application, with an active ingredient amount of hyaluronic acid and/or physiologically acceptable hyaluronic acid salt (hyaluronate) (component (b)) of (800 ± 80) mg, in particular (800 ± 60) mg, preferably (800 ± 40) mg.

10. The composition according to any one of the preceding claims
for instillation and/or for preferably topical application in the urogenital area, in particular in the urinary bladder; and/or
wherein the composition is prepared for administration and/or instillation and/or for topical application in the urogenital tract, in particular in the urinary bladder, or wherein the composition is administered by instillation and/or by preferably topical application in the urogenital tract, in particular in the urinary bladder.

11. The composition according to any one of the preceding claims for use in the prophylactic and/or therapeutic treatment of preferably inflammatory diseases of the urogenital tract, in particular of inflammatory diseases of the urinary bladder, more preferably of cystitis, in particular acute or chronic cystitis, more preferably interstitial cystitis, radiogenic cystitis, chronic recurrent cystitis, chemo-cystitis, chronic abacterial cystitis and chronic bacterial cystitis, particularly preferably interstitial cystitis.

12. A storage and/or application device, in particular a storage and/or application container, in particular in the form of a preferably sterile syringe, in particular an administration syringe, preferably a disposable administration syringe, preferably for instillation and/or preferably for topical application in the urogenital area, in particular in the urinary bladder, containing a composition according to any one of the preceding claims.

13. The storage and/or application device according to claim 12,
wherein the composition comprises an active ingredient amount of (a) chondroitin sulfate and/or physiologically acceptable chondroitin sulfate salt (component (a)) of (225 ± 15) mg, preferably (225 ± 10) mg; and/or
wherein the composition comprises an active ingredient amount of (b) hyaluronic acid and/or physiologically acceptable hyaluronic acid salt (hyaluronate) (component (b)) of (800 ± 60) mg, preferably (800 ± 40) mg; and/or
wherein the composition comprises the dihydrogen phosphate/monohydrogen phosphate buffer system (component (c)) in a total amount of dihydrogen phosphate/monohydrogen phosphate buffer system (component (c)) of (87.5 ± 85) mg, in particular in a total amount of (87.5 ± 75) mg, preferably in a total amount of (87.5 ± 62.5) mg, more preferably in a total amount of (87.5 ± 50) mg, particularly preferably in a total amount of (87.5 ± 45) mg, very particularly preferably in a total amount of (87.5 ± 40) mg, even more preferably in a total amount of about 87.5 mg; and/or
wherein the composition contains the electrolytes (component (d)) in an amount of (400 ± 300) mg, in particular in an amount of (400 ± 200) mg, preferably in an amount of (400 ± 100) mg, more preferably in an amount of (400 ± 50) mg, particularly preferably in an amount of about 400 mg; and/or
wherein the storage and/or application device contains the composition, in particular ready for use, dosing and/or application, with a volume of (50 ± 10) ml, in particular (50 ± 5) ml, preferably (50 ± 2) ml, more preferably (50 ± 1) ml, particularly preferably (50 ± 0.5) ml, very particularly preferably about 50 ml; and/or
wherein the composition is present with a volume of (50 ± 10) ml, in particular (50 ± 5) ml, preferably (50 ± 2) ml, more preferably (50 ± 1) ml, particularly preferably (50 ± 0.5) ml, very particularly preferably about 50 ml.

14. A packaging unit containing at least one storage and/or application device according to claim 12 or 13, in particular wherein the storage and/or application device is present in outer packaging that protects against contamination.

15. A kit, in particular an instillation system, comprising (i) at least one storage and/or application device according to claim 12 or 13, (ii) at least one composition according to any one of the preceding claims, in particular wherein the composition is present, preferably ready for use, dosing and/or application, in the storage and/or application device, and (iii) at least one instillation device connectable to the storage and/or application device, in particular in the form of an instillation tube or the like.

## Revendications

1. Composition, la composition contenant, en association et respectivement en des quantités efficaces :
(a) du sulfate de chondroïtine et/ou un sel de sulfate de chondroïtine physiologiquement acceptable à une concentration de (4,5 ± 0,5) mg/ml (composant (a)) ; et
(b) de l'acide hyaluronique et/ou un sel d'acide hyaluronique physiologiquement acceptable (hyaluronate) à une concentration de (16 ± 1,6) mg/ml (composant (b)) ;
la
composition ayant un pH dans la plage de 6,1 à 7,9 et/ou la composition étant ajustée à un pH dans la plage de 6,1 à 7,9,
**caractérisée en ce que**
la composition
(c) contient un système de tampon dihydrogénophosphate/monohydrogénophosphate (composant (c)).

2. Composition selon la revendication 1,
ladite composition présentant le sulfate de chondroïtine et/ou le sel de sulfate de chondroïtine physiologiquement acceptable (composant (a)) à une concentration de (4,5 ± 0,4) mg/ml, en particulier à une concentration de (4,5 ± 0,3) mg / ml, de préférence à une concentration de (4,5 ± 0,15) mg/ml, plus préférablement à une concentration d'environ 4,5 mg/ml ; et/ou
le sulfate de chondroïtine et/ou le sel de sulfate de chondroïtine physiologiquement acceptable (composant (a)) présentant une masse moléculaire moyenne en nombre (masse molaire) Mₙ dans la plage de 2 kDa à 200 kDa, en particulier dans la plage de 5 kDa à 150 kDa, de préférence dans la plage de 10 kDa à 100 kDa, préférablement dans la plage de 12 kDa à 75 kDa ; et/ou
le sulfate de chondroïtine et/ou le sel de sulfate de chondroïtine physiologiquement acceptable (composant (a)) présentant une masse moléculaire moyenne en poids (masse molaire) M_{w} dans la plage de 10 kDa à 200 kDa, en particulier dans la plage de 15 kDa à 175 kDa, de préférence dans la plage de 20 kDa à 150 kDa, préférablement dans la plage de 30 kDa à 120 kDa ; et/ou
le sulfate de chondroïtine et/ou le sel de sulfate de chondroïtine physiologiquement acceptable (composant (a)) présentant une masse moléculaire moyenne de centrifugation (masse molaire) M_{z} dans la plage de 30 kDa à 1000 kDa, en particulier dans la plage de 40 kDa à 800 kDa, de préférence dans la plage de 50 kDa à 600 kDa, préférablement dans la plage de 100 kDa à 450 kDa ; et/ou
le sulfate de chondroïtine et/ou le sel de sulfate de chondroïtine physiologiquement acceptable (composant (a)) présentant un indice de polydispersité (PDI), calculé en tant que rapport de la masse moléculaire moyenne en poids M_{w} et de la masse moléculaire moyenne en nombre Mₙ, d'au moins 1, en particulier d'au moins 1,2, de préférence d'au moins 1,5, préférablement d'au moins 2 ; et/ou
le sulfate de chondroïtine et/ou le sel de sulfate de chondroïtine physiologiquement acceptable (composant (a)) présentant un indice de polydispersité (PDI), calculé en tant que rapport de la masse moléculaire moyenne en poids M_{w} et de la masse moléculaire moyenne en nombre Mₙ d'au plus 30, notamment d'au plus 20, de préférence d'au plus 10, préférablement d'au plus 8 ; et/ou
le sulfate de chondroïtine et/ou le sel de sulfate de chondroïtine physiologiquement acceptable (composant (a)) présentant un indice de polydispersité (PDI), calculé en tant que rapport de la masse moléculaire moyenne en poids M_{w} et de la masse moléculaire moyenne en nombre Mₙ, dans la plage de 1 à 30, en particulier dans la plage de 1,2 à 20, de préférence dans la plage de 1,5 à 10, préférablement dans la plage de 2 à 8.

3. Composition selon la revendication 1 ou 2,
la composition contenant l'acide hyaluronique et/ou le sel d'acide hyaluronique physiologiquement acceptable (composant (b)) à une concentration de (16 ± 1,2) mg/ml, en particulier à une concentration de (16 ± 0,8) mg/ml , de préférence à une concentration de (16 ± 0,4) mg/ml, plus préférablement à une concentration d'environ 16 mg/ml ; et/ou
l'acide hyaluronique et/ou le sel d'acide hyaluronique physiologiquement acceptable (composant (b)) présentant une masse moléculaire moyenne en nombre (masse molaire) Mₙ dans la plage de 10 kDa à 300 kDa, en particulier dans la plage de 20 kDa à 275 kDa, de préférence dans la plage de 30 kDa à 260 kDa, préférablement dans la plage de 50 kDa à 250 kDa, plus préférablement dans la plage de 75 kDa à 200 kDa ; et/ou
l'acide hyaluronique et/ou le sel d'acide hyaluronique physiologiquement acceptable (composant (b)) présentant une masse moléculaire moyenne en poids (masse molaire) M_{w} dans la plage de 10 kDa à 500 kDa, en particulier dans la plage de 20 kDa à 450 kDa, de préférence dans la plage de 50 kDa à 425 kDa, préférablement dans la plage de 100 kDa à 400 kDa, de manière particulièrement préférée dans la plage de 150 kDa à 395 kDa ; et/ou
l'acide hyaluronique et/ou le sel d'acide hyaluronique physiologiquement acceptable (composant (b)) présentant une masse moléculaire moyenne de centrifugation (masse molaire) M_{z} dans la plage de 80 kDa à 1500 kDa, en particulier dans la plage de 100 kDa à 1250 kDa, de préférence dans la plage de 200 kDa à 1000 kDa, préférablement dans la plage de 300 kDa à 750 kDa ; et/ou
l'acide hyaluronique et/ou le sel d'acide hyaluronique physiologiquement acceptable (composant (b)) présentant un indice de polydispersité (PDI), calculé en tant que rapport de la masse moléculaire moyenne en poids M_{w} et de la masse moléculaire moyenne en nombre Mₙ, d'au moins 1, en particulier d'au moins 1,1, de préférence d'au moins 1,2, préférablement d'au moins 1,3, plus préférablement d'au moins 1,4 ; et/ou
l'acide hyaluronique et/ou le sel d'acide hyaluronique physiologiquement acceptable (composant (b)) ayant un indice de polydispersité (PDI), calculé en tant que rapport de la masse moléculaire moyenne en poids M_{w} et de la masse moléculaire moyenne en nombre Mₙ, d'au plus 50, en particulier d'au plus 25, de préférence d'au plus 10, préférablement d'au plus 5, plus préférablement d'au plus 3 ; et/ou
l'acide hyaluronique et/ou le sel d'acide hyaluronique physiologiquement acceptable (composant (b)) ayant un indice de polydispersité (PDI), calculé en tant que rapport de la masse moléculaire moyenne en poids M_{w} et de la masse moléculaire moyenne en nombre Mₙ, dans la plage de 1 à 50, notamment dans la plage de 1,1 à 25, de préférence dans la plage de 1,2 à 10, préférablement dans la plage de 1,3 à 5, de manière particulièrement préférée dans la plage de 1,4 à 3.

4. Composition selon l'une quelconque des revendications précédentes,
la composition contenant le système de tampon dihydrogénophosphate/monohydrogénophosphate (composant (c)) à une concentration totale de système de tampon dihydrogénophosphate/monohydrogénophosphate (composant (c)) de (1,75 ± 1,65) mg/ml, en particulier à une concentration totale de (1,75 ± 1,5) mg/ml, de préférence à une concentration totale de (1,75 ± 1,25) mg / ml, préférablement à une concentration totale de (1,75 ± 1) mg/ml, plus préférablement à une concentration totale de (1,75 ± 0,9) mg/ml, de manière préférée entre toutes à une concentration totale de (1,75 ± 0,8) mg/ml, encore plus préférentiellement à une concentration totale d'environ 1,75 mg/ml ; et/ou
la composition contenant le système de tampon dihydrogénophosphate/monohydrogénophosphate (composant (c)) en une quantité totale de système de tampon dihydrogénophosphate/monohydrogénophosphate (composant (c)) de (87,5 ± 85) mg, en particulier en une quantité totale de (87,5 ± 75) mg, de préférence en une quantité totale de (87,5 ± 62,5) mg, préférablement en une quantité totale de (87,5 ± 50) mg, plus préférablement en une quantité totale de (87,5 ± 45) mg, de manière tout particulièrement préférée en une quantité totale de (87,5 ± 40) mg, plus préférablement encore en une quantité totale d'environ 87,5 mg ; et/ou
la composition contenant le dihydrogénophosphate du système de tampon dihydrogénophosphate/monohydrogénophosphate (composant (c)), en particulier rapporté au poids initial lors de la préparation et/ou de la fourniture de la composition, à une concentration de (0,2 ± 0,19) mg/ml, en particulier à une concentration de (0,2 ± 0,15) mg/ml, de préférence à une concentration de (0,2 ± 0,125) mg/ml, préférablement à une concentration de (0,2 ± 0,1) mg/ml, plus préférablement à une concentration d'environ 0,2 mg/ml ; et/ou
la composition contenant le monohydrogénophosphate du système de tampon dihydrogénophosphate/monohydrogénophosphate (composant (c)), en particulier rapporté au poids initial lors de la préparation et/ou de la fourniture de la composition, à une concentration de (1,5 ± 1,4) mg/ml, en particulier à une concentration de (1,5 ± 1,25) mg/ml, de préférence à une concentration de (1,5 ± 1,1) mg/ml, préférablement à une concentration de (1,5 ± 1) mg/ml, plus préférablement à une concentration de (1,5 ± 0,75) mg/ml, de manière tout particulièrement préférée à une concentration d'environ 1,5 mg/ml ; et/ou
la composition contenant le système de tampon dihydrogénophosphate/monohydrogénophosphate (composant (c)) selon un rapport pondéral (rapport de concentration) du dihydrogénophosphate au monohydrogénophosphate [dihydrogénophosphate/monohydrogénophosphate], en particulier rapporté au poids lors de la préparation et/ou de la fourniture de la composition, dans la plage de 2: 1 à 1: 100, de préférence dans la plage de 1: 1 à 1: 75, préférablement dans la plage de 1: 2 à 1: 50, de manière particulièrement préférée dans la plage de 1: 5 à 1: 25.

5. Composition selon l'une quelconque des revendications précédentes,
le système de tampon dihydrogénophosphate/monohydrogénophosphate (composant (c)) étant présent et/ou conçu sous la forme d'un système de tampon hydrogénophosphate alcalin/monohydrogénophosphate alcalin ; en particulier le métal alcalin étant choisi parmi le sodium et/ou le potassium, en particulier le sodium ; et/ou
le système de tampon dihydrogénophosphate/monohydrogénophosphate (composant (c)) étant présent et/ou conçu sous la forme d'un système de tampon dihydrogénophosphate de sodium/monohydrogénophosphate de sodium et/ou
le système de tampon dihydrogénophosphate/monohydrogénophosphate (composant (c)) étant présent et/ou conçu sous la forme d'un système de tampon NaH₂(PO₄)/Na₂H(PO₄), en particulier sous la forme d'un système de tampon NaH₂(PO₄) • 2H₂ O/Na₂H(PO₄) • 2H₂O ; et/ou
le système de tampon dihydrogénophosphate/monohydrogénophosphate (composant (c)) étant utilisé pour et/ou servant à ajuster et/ou maintenir le pH de la composition.

6. Composition selon l'une quelconque des revendications précédentes,
la composition contenant l'électrolyte (composant (d)) à une concentration de (8 ± 6) mg/ml, en particulier à une concentration de (8 ± 4) mg/ml, de préférence à une concentration de (8 ± 2) mg/ml, préférablement à une concentration de (8 ± 1) mg/m, plus préférablement à une concentration d'environ 8 mg/ml ; et/ou
l'électrolyte (composant (d)) se présentant sous la forme d'un sel alcalin, en particulier sous la forme d'un chlorure de métal alcalin, de préférence sous la forme de chlorure de sodium, et/ou l'électrolyte (composant (d)) étant le chlorure de sodium.

7. Composition selon l'une quelconque des revendications précédentes,
la composition présentant un pH dans la plage de 6,6 à 7,7, en particulier dans la plage de 6,9 à 7,6, de préférence dans la plage de 7,1 à 7,4 ; et/ou
le pH de la composition étant maintenu constant et/ou ajusté dans la plage de 6,6 à 7,7, en particulier dans la plage de 6,9 à 7,6, de préférence dans la plage de 7,1 à 7,4 ; et/ou
le pH étant ajusté et/ou prédéterminé au moyen du système de tampon hydrogénophosphate alcalin/monohydrogénophosphate alcalin (composant (c)) ; et/ou
la composition étant présente sous la forme d'une composition aqueuse et/ou la composition ayant une base aqueuse et/ou se présentant sous la forme d'une formulation aqueuse, en particulier sous la forme d'une solution aqueuse et/ou d'une suspension aqueuse, de préférence sous la forme d'une solution aqueuse ; et/ou
la composition contenant de l'eau, en particulier de l'eau purifiée et/ou la composition contenant de l'eau en tant que véhicule (excipient) pharmaceutiquement acceptable et/ou la composition étant à base aqueuse ;
et/ou la composition présentant une teneur en eau d'au moins 50 % en poids, en particulier d'au moins 75 % en poids, de préférence d'au moins 80 % en poids, préférablement d'au moins 90 % en poids, de manière particulièrement préférée d'au moins 95 % en poids, rapporté à la composition ; et/ou
la composition étant constituée des composants (a), (b), (c) et éventuellement (d) susmentionnés et éventuellement d'eau, en particulier d'eau purifiée.

8. Composition selon l'une quelconque des revendications précédentes,
la composition étant, à des températures dans la plage de 20 °C à 45 °C, à une pression de 1013,25 mbar (pression atmosphérique) et à une humidité relative dans la plage de 50 % à 90 % au moins 6 mois, en particulier au moins 12 mois, de préférence au moins 24 mois, préférablement au moins 36 mois, stable, en particulier stable au stockage ; en particulier, la composition présentant au moment de la conservation une teneur totale en produits de dégradation des composants (a) et (b) d'au plus 5 %, en particulier d'au plus 4 %, de préférence d'au plus 3 %, préférablement d'au plus 2 %, de manière particulièrement préférée d'au plus 1 %, rapporté à la concentration totale les composants (a) et (b) ; et/ou
la composition présentant une stabilité, en particulier une stabilité au stockage, dans des conditions de vieillissement accéléré selon ASTM F 1980 à une température de vieillissement de 55 °C, d'au moins 6 mois, en particulier d'au moins 12 mois, de préférence d'au moins 24 mois, préférablement d'au moins 36 mois ; en particulier, la composition présentant au moment de la conservation une teneur totale en produits de dégradation des composants (a) et (b) d'au plus 5 %, en particulier d'au plus 4 %, de préférence d'au plus 3 %, préférablement d'au plus 2 %, de manière particulièrement préférée d'au plus 1 %, rapporté à la concentration totale des composants (a) et (b).

9. Composition selon l'une quelconque des revendications précédentes,
la composition, en particulier prête à utiliser, à doser et/ou à appliquer, étant présente à un volume de (50 ± 10) ml, en particulier (50 ± 5) ml, de préférence (50 ± 2) ml, préférablement (50 ± 1) ml, plus préférablement (50 ± 0,5) ml, de manière tout particulièrement préférée environ 50 ml ; et/ou
la composition, en particulier prête à utiliser, à doser et/ou à appliquer, étant présente et/ou préparée pour administrer, en particulier instiller dans la vessie, un volume de la composition de (50 ± 10) ml, en particulier (50 ± 5) ml, de préférence (50 ± 2) ml, préférablement (50 ± 1) ml, plus préférablement (50 ± 0,5) ml, de manière tout particulièrement préférée environ 50 ml ou la composition, en particulier prête à utiliser, à doser, et/ou à appliquer, étant administrée en particulier par instillation dans la vessie, d'un volume de la composition de (50 ± 10) ml, en particulier (50 ± 5) ml, de préférence (50 ± 2) ml, préférablement (50 ± 1) ml, très préférablement (50 ± 0,5) ml, de manière tout particulièrement préférée environ 50 ml ; et/ou
la composition, en particulier prête à utiliser, à doser et/ou à appliquer, étant présente en une quantité de principe actif tel que le sulfate de chondroïtine et/ou le sel de sulfate de chondroïtine physiologiquement acceptable (composant (a)) de (225 ± 25) mg, en particulier (225 ± 15) mg, de préférence (225 ± 10) mg ; et/ou
la composition, en particulier prête à utiliser, à doser et/ou à appliquer, étant présente et/ou préparée pour administrer une quantité de principe actif tel que le sulfate de chondroïtine et/ou le sel de sulfate de chondroïtine physiologiquement acceptable (composant (a)) de (225 ± 25) mg, en particulier (225 ± 15) mg, de préférence (225 ± 10) mg ou la composition, en particulier prête à utiliser, à doser et/ou à appliquer, étant administrée à une quantité de principe actif tel que le sulfate de chondroïtine et/ou le sel de sulfate de chondroïtine physiologiquement acceptable (composant (a)) de (225 ± 25) mg, en particulier (225 ± 15) mg, de préférence (225 ± 10) mg ; et/ou
la composition, en particulier prête à utiliser, à doser et/ou à appliquer, étant présente en une quantité de principe actif tel que l'acide hyaluronique et/ou le sel d'acide hyaluronique physiologiquement acceptable (hyaluronate) (composant (b)) de (800 ± 80) mg, en particulier (800 ± 60) mg, de préférence (800 ± 40) mg ; et/ou
la composition, en particulier prête à utiliser, à doser et/ou à appliquer, étant présente et/ou préparée pour administrer une quantité de principe actif tel que l'acide hyaluronique et/ou le sel d'acide hyaluronique physiologiquement acceptable (hyaluronate) (composant (b)) de (800 ± 80) mg, en particulier (800 ± 60) mg, de préférence (800 ± 40) mg ou la composition, en particulier prête à utiliser, à doser et/ou à appliquer, étant administrée à une quantité de principe actif tel que l'acide hyaluronique et/ou le sel d'acide hyaluronique physiologiquement acceptable (hyaluronate) (composant (b)) de (800 ± 80) mg, en particulier (800 ± 60) mg, de préférence (800 ± 40) mg.

10. Composition selon l'une quelconque des revendications précédentes,
pour instillation et/ou pour l'application topique de préférence dans la région urogénitale, en particulier dans la vessie ; et/ou
la composition étant préparée pour l'administration et/ou l'instillation et/ou de préférence l'application topique dans le tractus urogénital, en particulier dans la vessie ou la composition étant administrée par instillation et/ou de préférence par application topique dans le tractus urogénital, en particulier dans la vessie.

11. Composition selon l'une quelconque des revendications précédentes, destinée à être utilisée dans le traitement prophylactique et/ou thérapeutique des maladies de préférence inflammatoires du tractus urogénital, en particulier des maladies inflammatoires de la vessie, de préférence de la cystite, en particulier de la cystite aiguë ou chronique, de préférence de la cystite interstitielle, de la cystite radiogène, de la cystite chronique récidivante, de la cystite associée à une chimiothérapie, de la cystite chronique abactérienne et de la cystite chronique bactérienne, de manière particulièrement préférée de la cystite interstitielle.

12. Dispositif de stockage et/ou d'application, en particulier contenant de stockage et/ou d'application, en particulier sous la forme d'une seringue de préférence stérile, en particulier d'une seringue d'application, de préférence d'une seringue jetable, de préférence destinée à une instillation et/ou de préférence à une application topique dans la région urogénitale, en particulier dans la vessie, contenant une composition selon l'une quelconque des revendications précédentes.

13. Dispositif de stockage et/ou d'application selon la revendication 12,
la composition présentant une quantité de principe actif tel que (a) le sulfate de chondroïtine et/ou le sel de sulfate de chondroïtine physiologiquement acceptable (composant (a)) de (225 ± 15) mg, de préférence (225 ± 10) mg ; et/ou
la composition présentant une quantité de principe actif tel que (b) l'acide hyaluronique et/ou le sel d'acide hyaluronique physiologiquement acceptable (hyaluronate) (composant (b)) de (800 ± 60) mg, de préférence (800 ± 40) mg ; et/ou
la composition présentant le système de tampon dihydrogénophosphate/monohydrogénophosphate (composant (c)) en une quantité totale de système de tampon dihydrogénophosphate/monohydrogénophosphate (composant (c)) de (87,5 ± 85) mg, en particulier en une quantité totale de (87,5 ± 75) mg, de préférence en une quantité totale de (87,5 ± 62,5) mg, préférablement en une quantité totale de (87,5 ± 50) mg, plus préférablement en une quantité totale de (87,5 ± 45) mg, de manière tout particulièrement préférée en une quantité totale de (87,5 ± 40) mg, encore plus préférablement en une quantité totale d'environ 87,5 mg ; et/ou
la composition contenant l'électrolyte (composant (d)) en une quantité de (400 ± 300) mg, en particulier en une quantité de (400 ± 200) mg, de préférence en une quantité de (400 ± 100) mg, préférablement en une quantité de (400 ± 50) mg, plus préférablement en une quantité d'environ 400 mg ; et/ou
le dispositif de stockage et/ou d'application contenant la composition, en particulier prête à utiliser, à doser et/ou à appliquer, à un volume de (50 ± 10) ml, en particulier (50 ± 5) ml, de préférence (50 ± 2) ml, de préférence (50 ± 1) ml, plus préférablement (50 ± 0,5) ml, de manière tout particulièrement préférée environ 50 ml ; et/ou
la composition présentant un volume de (50 ± 10) ml, en particulier (50 ± 5) ml, de préférence (50 ± 2) ml, préférablement (50 ± 1) ml, très préférablement (50 ± 0,5) ml, de manière tout particulièrement préférée environ 50 ml.

14. Unité de conditionnement contenant au moins un dispositif de stockage et/ou d'application selon la revendication 12 ou 13, en particulier le dispositif de stockage et/ou d'application étant inséré dans un emballage extérieur protégeant contre toute contamination.

15. Kit, en particulier système d'instillation, comprenant (i) au moins un dispositif de stockage et/ou d'application selon la revendication 12 ou 13, (ii) au moins une composition selon l'une quelconque des revendications précédentes, en particulier la composition de préférence prête à utiliser, à doser et/ou à appliquer étant présente dans le dispositif de stockage et/ou d'application et (iii) au moins un dispositif d'instillation pouvant être connecté au dispositif de stockage et/ou d'application, en particulier sous la forme d'une tubulure pour instillation ou analogue.
